# FASCICULE DE BREVET EUROPEEN

(11) **EP 2 024 379 B1**
(45) Date de publication et mention de la délivrance du brevet: **22.09.2010**
(21) Numéro de dépôt: 07731356.7
(22) Date de dépôt: 24.04.2007
(51) Int. Cl.: C07H 7/00, A61K 31/70

(54) **NOUVEAUX COMPOSES C-GLYCOSIDES GEM-DIFLUORES DERIVES DE LA PODOPHYLLOTOXINE, LEUR PREPARATION ET LEURS APPLICATIONS**
NEUE, VON PODOPHYLLOTOXIN ABGELEITETE GEM-DIFLUORID-C-GLYCOSIDVERBINDUNGEN, IHRE HERSTELLUNG UND ANWENDUNGEN
NEW GEM-DIFLUORIDE C-GLYCOSIDE COMPOUNDS DERIVED FROM PODOPHYLLOTOXIN, THEIR PREPARATION AND APPLICATIONS

(30) Priorité: 25.04.2006 FR 0603766
(43) Date de publication de la demande: 18.02.2009
(73) Titulaire: Institut National des Sciences Appliquées de Rouen (INSA), 76131 Mont Saint Aignan Cedex (FR)
(72) Inventeur: QUIRION, Jean-Charles, 27130 Bourg-Achard (FR); CASTELOT DELIENCOURT-GODEFROY, Géraldine, 76000 Rouen (FR); AUDOUARD, Christophe, 76120 Grand Quevilly (FR)
(74) Mandataire: de Saint-Palais, Arnaud Marie
(86) Numéro de dépôt international: PCT/FR2007/000697
(87) Numéro de publication internationale: WO 2007/125194

(56) Documents cités:
- EP-A2- 0 445 021
- WO-A-01/49693
- JP-A- 1 197 486
- US-A- 5 300 500
- DUCA, MARIA ET AL: "Novel carbamate derivatives of 4-.beta.-amino-4'-O-demethyl-4- desoxypodophyllotoxin as inhibitors of topoisomerase II: synthesis and biological evaluation" ORGANIC & BIOMOLECULAR CHEMISTRY , 3(6), 1074-1080 CODEN: OBCRAK; ISSN: 1477-0520, 2005, XP002405787
- ZHANG, FU-MIN ET AL: "Synthesis and anti-cancer activity of new derivatives of 4'-demethylepipodophyllotoxin" HUAXUE XUEBAO , 60(4), 720-724 CODEN: HHHPA4; ISSN: 0567-7351, 2002, XP009035826
- WANG, ZHIGUANG ET AL: "Synthesis and antitumor activity of 4-[.gamma.-(alkylamino)propylthio]-4- deoxy-4'-demethylepipodophyllotoxins" ZHONGGUO YIYAO GONGYE ZAZHI , 23(2), 65-8 CODEN: ZYGZEA; ISSN: 1001-8255, 1992, XP009074607

## Description

L'invention concerne un procédé pour la synthèse de composés C-glycosides gemdifluorés dérivés de la podophyllotoxine. Elle s'applique plus particulièrement, mais non exclusivement, à la préparation de composés utilisables notamment en oncologie pour le traitement du cancer.

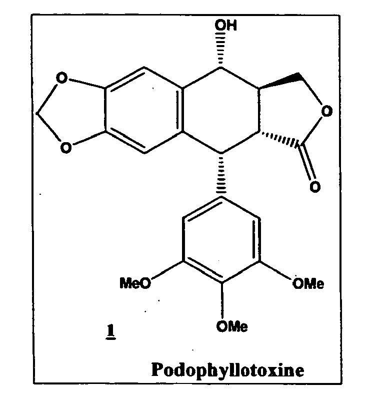

La podophyllotoxine **1** est un lignane isolé des racines de deux plantes *Podophyllum peltatum* (Amérique de Nord) et *Podophyllum emodi* (Asie). Elle possède une forte activité antimitotique en inhibant la polymérisation de la tubuline. Trop toxique pour être utilisée en chimiothérapie, elle a donné naissance après modifications structurales à de nombreux composés antitumoraux. Parmi eux, des dérivés glycosylés, composés habituellement moins toxiques et plus hydrosolubles ont émergé.

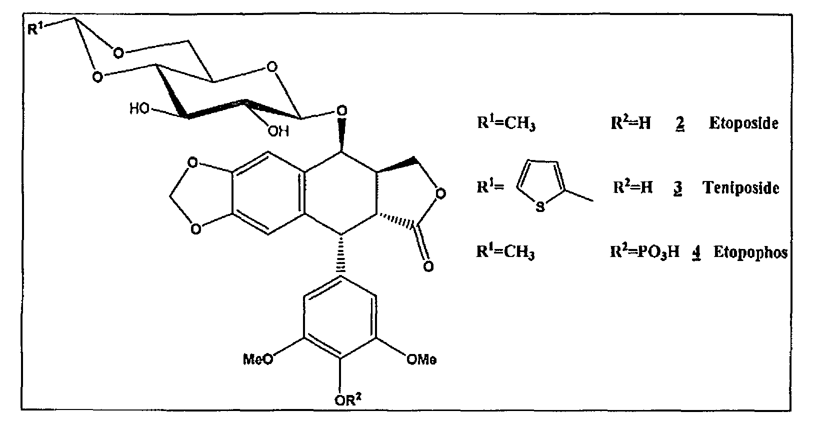

C'est le cas de l'étoposide **2** (ou VP-16) utilisé notamment dans le traitement du cancer du poumon à petites cellules, de la vessie, des testicules, des lymphomes, des leucémies aiguës, des sarcomes de Kaposi.

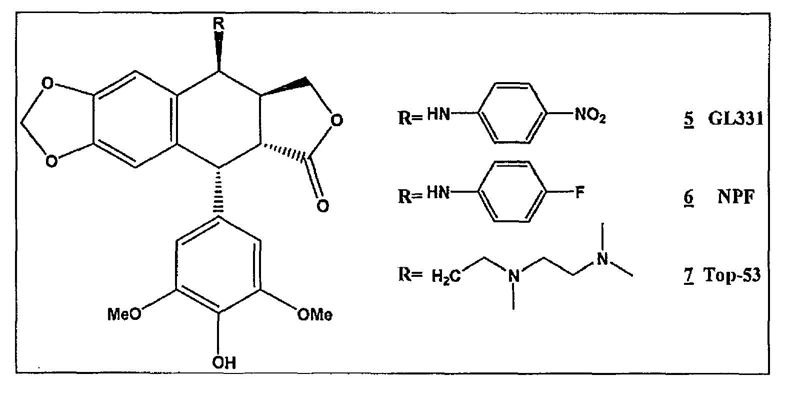

Des dérivés azotés de la podophyllotoxine comme le GL-331 **5** le NPF **6** ou le TOP-53 **7** montrent également des activités très intéressantes.

Toutes ces molécules dérivées de la structure déméthylépipodophyllotoxine sont des inhibiteurs de la topoisomérase II, enzyme qui catalyse la coupure puis la reformation des 2 brins de l'ADN.

De nombreux documents présentent différents dérivés de la podophyllotoxine, en particulier:
- D1 : La demande de brevet international n° WO 01/49693 du 12 juillet 2001
- D2: le brevet européen n° EP 0 445 021 du 4 septembre 1991
- D3: La demande de brevet américain n° US 5 300 500 du 5 avril 1994
- D4: La demande de brevet japonais n° JP 01 197486 du 9 août 1989
- D5: La publication scientifique de DUCA, Maria et al., 'Novel carbamate derivatives cf 4-.beta-amino-4'-O- demethyl-4- desoxypodophyllotoxin as inhibitors cf topoisomerase II: synthesis and biological évaluation' ORGANIC & BIOMOLECULAR CHEMISTRY, 3(6), 1074-1080 (2005), XP002405787
- D6: La publication scientifique de ZHANG, FU-MIN et al., "Synthesis and anti-cancer activity 0f new derivatives cf 4'-demethylepipodophyllotoxin" HUAXUE XUEBAO, 60(4), 720-724 (2002) ; XP009035826
- D7: WANG, ZHIGUANG et al., 'Synthesis and antitumor activity of 4-[.gamma.-(alkylamino)propylthio]-4- deoxy-4'-demethylepipodophyllotoxins" ZHONGGUO YIYAO GONGYE ZAZHI , 23(2), 65-8 (1992), XP009074607

Nous avons développé la synthèse de composés azotés de la podophyllotoxine, avec une fonction amide substitué par un glycoside gem-difluoré. Or, l'art antérieur connu ne divulgue pas de dérivés de la podophyllotoxine modifiés en position 4 par une série de groupements carbohydrate-difluorométhyl-carboxamido diversement substitués, utilisables comme composés anticancéreux.
L'intérêt du groupement CF₂ est outre sa résistance face aux processus de dégradations biochimiques le fait qu'il constitue un excellent mime de l'oxygène. Il permet ainsi la synthèse de structures non hydrolysables.

De tels composés seraient utilisables comme agents de chimiothérapie dans le traitement de différents types de cancer, soit seul ou en association avec d'autres chimiothérapies dans le cadre d'une multithérapie.

Les molécules développées appartiennent à la série des analogues azotés de la podophyllotoxine, famille présentant une importante cytotoxicité.
De plus la présence d'un glycoside est connue pour améliorer la solubilité dans les solvants aqueux, et diminuer la toxicité.

L'utilisation d'analogues difluorés en position anomérique du glycoside renforce de plus leur stabilité face à des hydrolyses acido-basiques et surtout enzymatiques.

A cet effet, l'invention propose un composé glycoconjugué gem-difluoré de formule générale I : 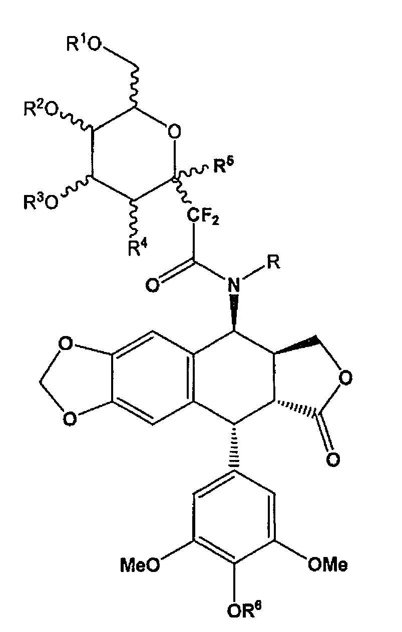 où R représente un atome d'hydrogène ou un groupe alkyle linéaire ou ramifié, benzyle, acétyle, benzoyle,
R¹ et R², identiques ou différents,
représentent un atome d'hydrogène
ou un groupe protecteur alkyle linéaire ou ramifié, benzyle, benzoyle, acétyle, pivaloyle, trialkylsilyle, tertiobutyldiphénylsilyle
ou un groupe acétal, du type CR'R",
avec R'et R", identiques ou différents, représentent un atome d'hydrogène ou un groupe alkyle linéaire ou ramifié, aryle, benzyle, thiophène,
R³ représente un atome d'hydrogène
ou un groupe protecteur alkyle linéaire ou ramifié, benzyle, benzoyle, acétyle, pivaloyle, trialkylsilyle , tertiobutyldiphénylsilyle,
R⁴ représente OR"', NGR'GR", N₃, ou un phtalimide
avec R"' représente un atome d'hydrogène ou un groupe protecteur alkyle linéaire ou ramifié, benzyle, benzoyle, acétyle, pivaloyle, trialkylsilyle, tertiobutyldiphénylsilyle, GR'et GR", identiques ou différents, représentent un atome d'hydrogène ou un groupe alkyle linéaire ou ramifié, benzyle, benzoyle, acétyle, alkyloxycarbonyle, allyloxycarbonyle, benzyloxycarbonyle,
R⁵ représente un groupe hydroxyle libre ou protégé ou un halogène,
R⁶ représente un atome d'hydrogène ou un groupe alkyle linéaire ou ramifié, acétyle, benzyle, PO₃H, PO₃Na,
ainsi que ses dérivés à l'état de base, de sel d'addition à un acide minéral ou organique, d'hydrate ou de solvat éventuellement pharmaceutiquement acceptable.

Plus précisément, un composé selon l'invention pourra avoir une formule générale II : 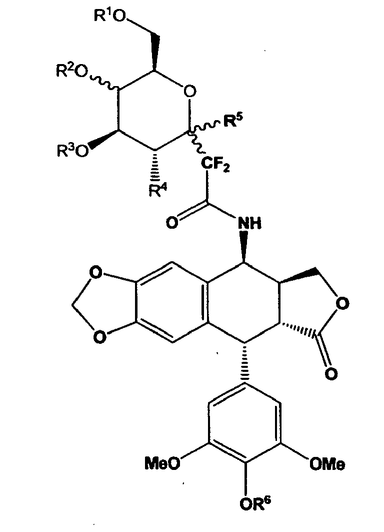 dans laquelle R, R¹, R², R³, R⁴, R⁵ et R⁶ sont tels que définis dans la formule I, ainsi que ses dérivés à l'état de base, de sel d'addition à un acide minéral ou organique, d'hydrate ou de solvat éventuellement pharmaceutiquement acceptable.

Les composés de formule I et II pourront être utilisés, par exemple par une réaction de réduction de la fonction amide, pour la synthèse des composés de formule III : 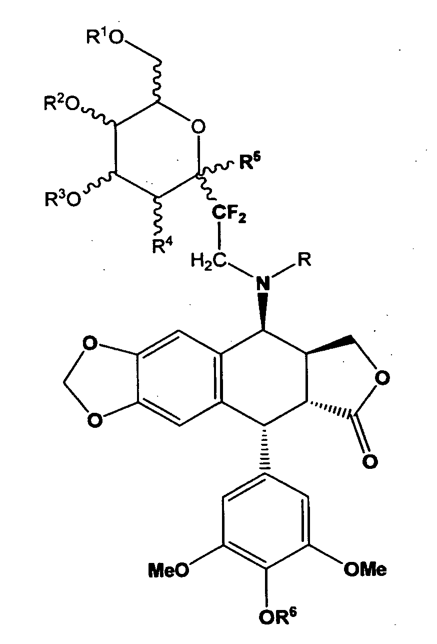 où R représente un atome d'hydrogène ou un groupe alkyle linéaire ou ramifié, benzyle, acétyle, benzoyle,
R¹ et R², identiques ou différents,
représentent un atome d'hydrogène
ou un groupe protecteur alkyle linéaire ou ramifié, benzyle, benzoyle, acétyle, pivaloyle, trialkylsilyle, tertiobutyldiphénylsilyle
ou un groupe acétal, du type CR'R",
avec R' et R", identiques ou différents, représentent un atome d'hydrogène ou un groupe alkyle linéaire ou ramifié, aryle, benzyle, thiophène,
R³ représente un atome d'hydrogène
ou un groupe protecteur alkyle linéaire ou ramifié, benzyle, benzoyle, acétyle, pivaloyle, trialkylsilyle , tertiobutyldiphénylsilyle,
R⁴ représente OR"', NGR'GR", N₃, ou un phtalimide
avec R"' représente un atome d'hydrogène ou un groupe protecteur alkyle linéaire ou ramifié, benzyle, benzoyle, acétyle, pivaloyle, trialkylsilyle, tertiobutyldiphénylsilyle, GR'et GR", identiques ou différents, représentent un atome d'hydrogène ou un groupe alkyle linéaire ou ramifié, benzyle, benzoyle, acétyle, alkyloxycarbonyle, allyloxycarbonyle, benzyloxycarbonyle,
R⁵ représente un groupe hydroxyle libre ou protégé ou un halogène,
R⁶ représente un atome d'hydrogène ou un groupe alkyle linéaire ou ramifié, acétyle, benzyle, PO₃H, PO₃Na,
ainsi que ses dérivés à l'état de base, de sel d'addition à un acide minéral ou organique, d'hydrate ou de solvat éventuellement pharmaceutiquement acceptable.

Dans les formules I à III, les groupes alkyles linéaires ou ramifiés pourront être des groupes possédant de 1 à 10 atomes de carbones.

Les composés de formules générales I à III tels que définis précédemment c'est-à dire y compris leurs dérivés à l'état de base, de sel d'addition à un acide minéral ou organique, d'hydrate ou de solvat éventuellement pharmaceutiquement acceptable pourront être présentés sous différentes formes galéniques adaptées à leur utilisation par exemple des solutions ou suspensions injectables.

Un procédé de préparation de composés de formule I comprend une étape de couplage entre un composé de formule IV : 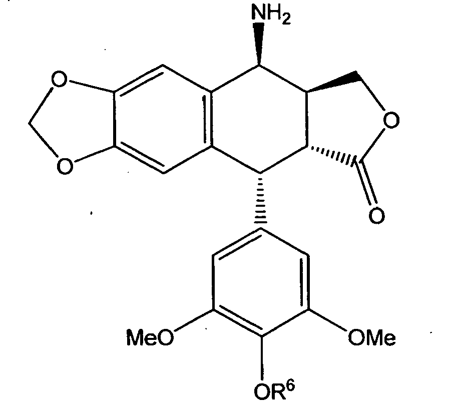 dans laquelle R⁶ est tel que défini dans la formule I
et un composé de formule V : 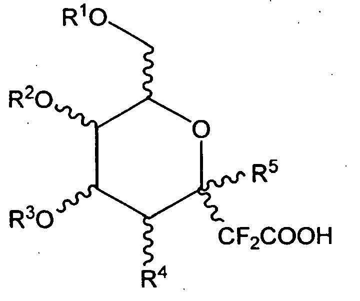 dans laquelle R¹, R², R³, R⁴, R⁵ sont tels que définis dans la formule I.

Ledit composé de formule IV est obtenu par épimérisation puis substitution de la fonction alcool en position 4 par un groupe azido réduit ultérieurement en amine.

Le composé de formule V est obtenu via un composé intermédiaire de formule VI : 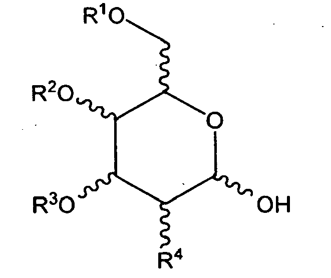 dans laquelle R¹, R², R³, R⁴ sont tels que définis dans la formule I.

Lorsque dans le composé de formule V, R⁵ représente un groupe hydroxyle, la préparation dudit composé de formule V comprend, en outre, une oxydation en lactone du composé de formule VI suivi d'une réaction de Reformatsky.

L'invention a également pour objet un médicament contenant en tant que principe actif au moins un composé de formule I à III tel que défini précédemment.

Selon un autre de ses aspects, la présente invention concerne l'utilisation d'au moins un composé de formule générale I à III tel que défini précédemment pour la préparation de médicaments/compositions pour le traitement de cancers tels que, par exemple, le cancer du poumon à petites cellules, de la vessie, des testicules, des lymphomes, des leucémies aiguës, des sarcomes de Kaposi.

Un autre objet de l'invention se rapporte à une composition comprenant au moins un composé de formule I à III tel que défini précédemment.

Bien entendu, la composition selon l'invention pourra comprendre les composés de formule I à III tel que défini précédemment seuls ou en mélange et en toutes proportions.

La composition selon l'invention pourra être destinée à un usage pharmaceutique.

Dans des compositions pharmaceutiques selon la présente invention pour l'administration par voir orale, sublinguale, inhalée, sous-cutanée, intramusculaire, intraveineuse, transdermique, locale ou rectale, les principes actifs pourront être administrés sous formes unitaires d'administration, en mélange avec des supports/véhicules pharmaceutiquement acceptables classiques.
Les formes unitaires d'administration appropriées comprennent les formes par voie orale telles que les comprimés, les gélules, les poudres, les granules et les solutions ou suspensions orales, les formes d'administration topique, les implants, les formes d'administration sous-cutanée, intramusculaire, intraveineuse, intranasale ou intraoculaire et les formes d'administration rectale.

Outre les excipients inertes, non toxiques et pharmaceutiquement acceptables, tels que l'eau distillée, le glucose, le lactose d'amidon, le talc, les huiles végétales, l'éthylène glycol..., les compositions ainsi obtenues pourront également contenir des agents de préservation.

D'autres principes actifs pourront être ajoutés dans ces compositions.

La quantité de composé selon l'invention et d'autres éventuels principes actifs dans de telles compositions pourra varier selon les applications, l'âge et le poids du malade le cas échéant.

Des exemples de préparation de composés selon l'invention seront décrits ci-après, à titre d'exemples non limitatifs, avec référence aux dessins annexés dans lesquels :
La figure 1 est une équation de réaction pour obtenir les composés 11 et 12 ;
La figure 2 est une équation de réaction pour obtenir le composé 13 ;
La figure 3 est une équation de réaction pour obtenir le composé 14 ;
La figure 4 est une équation de réaction pour obtenir le composé 15 ;
La figure 5 est une équation de réaction pour obtenir le composé 16 ;
La figure 6 est une équation de réaction pour obtenir le composé 17 ;
La figure 7 est une équation de réaction pour obtenir le composé 19a ou 19b ;
La figure 8 est une équation de réaction pour obtenir le composé 20a ou 20b ;
La figure 9 est une équation de réaction pour obtenir le composé 21a ou 21b ;
La figure 10 est une équation de réaction pour obtenir le composé 22a ou 22b;
La figure 11 est une équation de réaction pour obtenir le composé 23a ou 23b ;
La figure 12 est une équation de réaction pour obtenir le composé 24a ou 24b ou 25a ou 25b ;
La figure 13 est une équation de réaction pour obtenir le composé 26a ou 26b ou 27a ou 27b ;
La figure 14 est une équation de réaction pour obtenir le composé 28a ou 28b ou 29a ou 29b ;
La figure 15 est une équation de réaction pour obtenir le composé 30a ;
La figure 16 est une équation de réaction pour obtenir le composé 31a.
La figure 17 est une équation de réaction pour obtenir le composé 32a.
La figure 18 est une équation de réaction pour obtenir le composé 33a.
La figure 19 est une équation de réaction pour obtenir le composé 34a.
La figure20 est une équation de réaction pour obtenir le composé 35a.
La figure 21 est une équation de réaction pour obtenir le composé 36a.
La figure 22 est une équation de réaction pour obtenir le composé 37a.
La figure 23 est une équation de réaction pour obtenir le composé 38a.
La figure 24 est une équation de réaction pour obtenir le composé 39a.
La figure 25 est une équation de réaction pour obtenir le composé 41 a.
La figure 26 est une équation de réaction pour obtenir le composé 42a.
La figure 27 est une équation de réaction pour obtenir le composé 43a.
La figure 28 est une équation de réaction pour obtenir le composé 44a.
La figure 29 est une équation de réaction pour obtenir le composé 45a.
La figure 30 est une équation de réaction pour obtenir le composé 46aa.

Les abréviations rencontrées sont définies ainsi :

| | | |
|---|---|---|
| éq. : équivalent | g : gramme | Hz : Hertz |
| mg : milligramme | MHz : mégaHertz | min.: minute |
| mL: millilitre | mmol: millimole | µmol: micromole |
| nmol : nanomole | app : apparent | |

Les caractéristiques des appareils utilisés pour effectuer les analyses de tous les composés décrits dans la présente demande sont indiquées ci-dessous :

Les spectres RMN ¹H, ¹³C, ¹⁹F ont été enregistrés sur des spectromètres BRUKER DPX 300 et DPX 600. En RMN ¹H et ¹³C, le tétraméthylsilane est utilisé comme référence interne. En RMN ¹⁹F, la référence externe est le fluorotrichlorométhane CFCl₃. Les déplacements chimiques sont exprimés en partie par million (ppm), les constantes de couplage J en Hertz (Hz).
Les abréviations suivantes ont été utilisées :
s pour singulet, bs pour un large singulet, d pour doublet, t pour triplet, q pour quadruplet, m pour multiplet ou massif, dd pour doublet de doublet...

Les spectres de masse ont été obtenus sur un spectrophotomètre de type Micromass TOF-SPEC, E 20 kV, α-cyano. pour l'ionisation Maldi et JEOL AX500, 3 kV, Canon FAB JEOL, Xe, 4 kV, courant limite 10 µA, Gly-NBA 50 :50 pour l'ionisation FAB.

Les séparations par chromatographie sur colonne sont réalisées sous pression légère en suivant les techniques de chromatographie sur silice Kieselgel 60 (230-400 Mesh, Merck).
Le suivi est assuré par chromatographie sur couches minces (CCM) avec des plaques Kieselgel 60F-254-0.25mm. On appelle rapport frontal (Rf) le rapport de la distance de migration d'un composé sur un support donné sur la distance de migration d'un éluant.

Les figures ci-après décrivent la préparation de composés glycoconjugués gem-difluorés de formule **8** et des réactions les impliquant pour obtenir d'autres composés actifs : 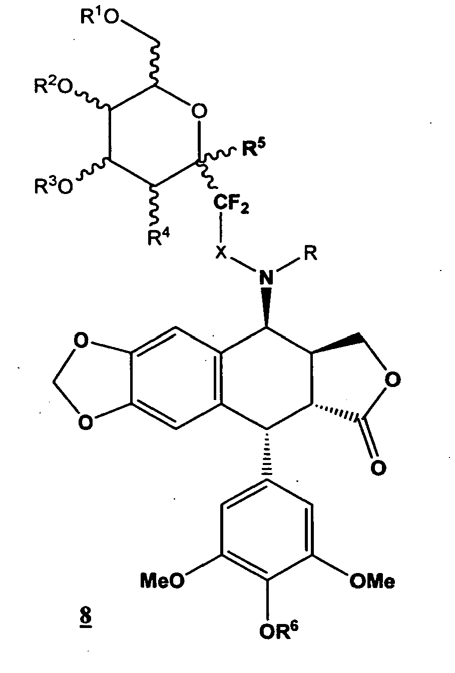 où R représente un atome d'hydrogène ou un groupe méthyle,
X représente un groupe carbonyle -C=O ou un groupe -CH₂,
R¹ et R², identiques ou différents, représentent un atome d'hydrogène ou un groupe benzyle,
R³ représente un atome d'hydrogène ou un groupe benzyle,
R⁴ représente OR"', avec R" représente un atome d'hydrogène ou un groupe benzyle,
R⁵ représente un groupe hydroxyle libre,un hydrogène, ou un halogène tel que l'atome de chlore
R⁶ représente un atome d'hydrogène ou un groupe méthyle.

Les molécules cibles **8** sont obtenues par réaction de couplage entre deux synthons : l'unité glycosidique **10** et le motif aminoépipodophyllotoxine **9** :

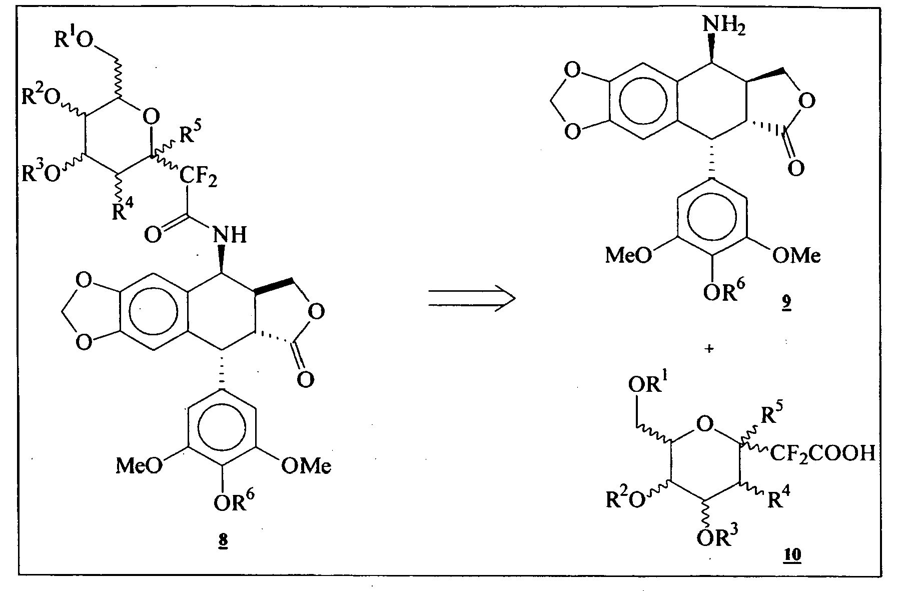

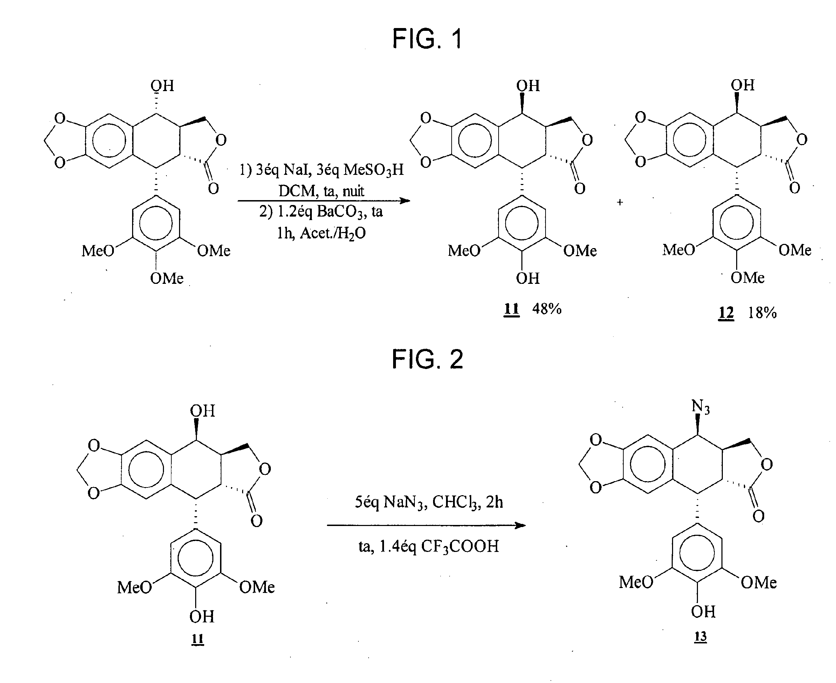

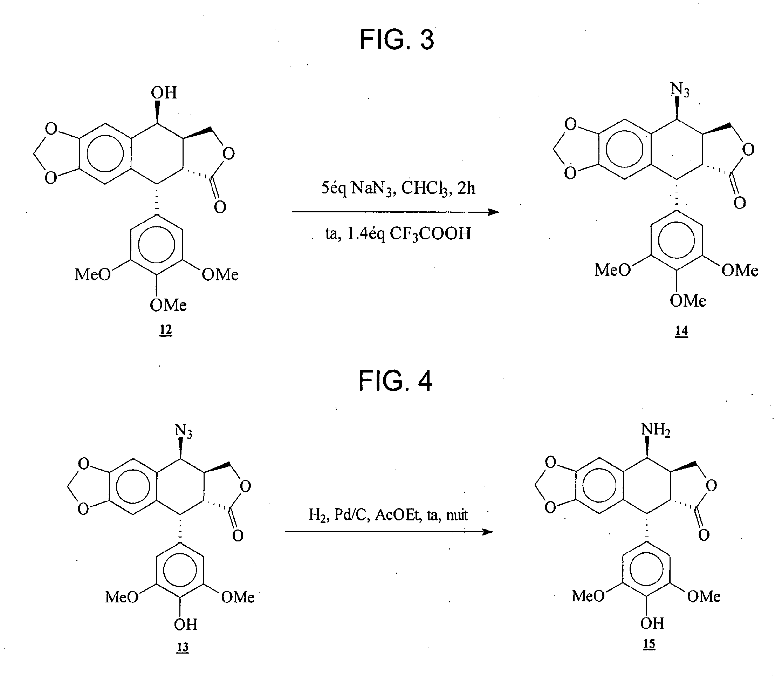

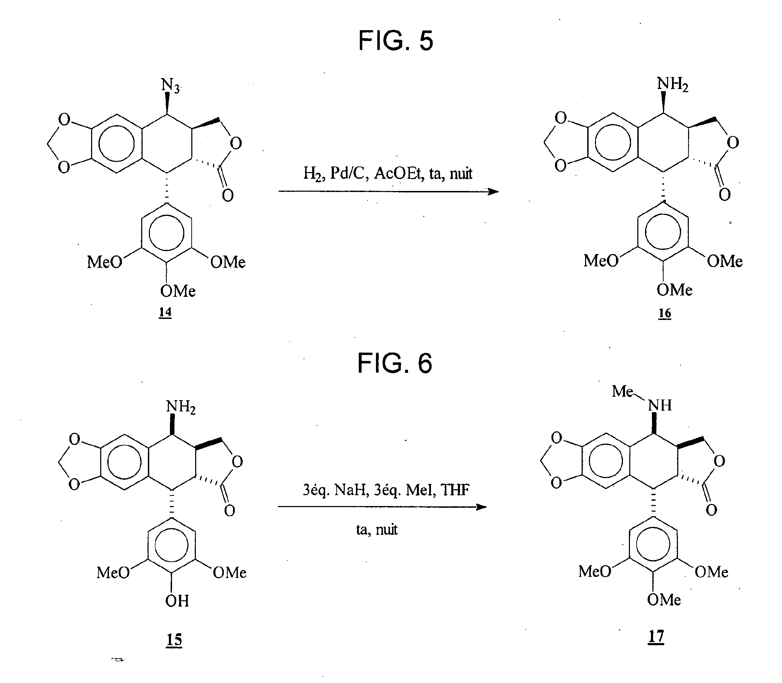

L'aménagement de ces unités fonctionnelles est réalisé comme suit.
En partant de la podophyllotoxine **1**, on réalise les étapes d'épimérisation de l'OH en position 4, et la déméthylation de l'OMe en position 4' pour conduire à la déméthylépipodophyllotoxine **11**, mais on observe également dans le milieu le produit épimérisé mais non déméthylé : l'épipodophyllotoxine **12** (Fig. 1).
Sur ces deux composés le OH en position **4** est ensuite substitué par un groupe azido (Fig. 2 et 3) qui est ensuite réduit en amine (Fig. 4 et 5).

### Synthèse de l'épipodophyllotoxine 12 et du 4'-déméthylépipodophyllotoxine 11 (Fig. 1)

La podophyllotoxine **1** (1,00 g ; 2,29 mmol ; 1 éq.) est dissoute dans du dichlorométhane DCM sec (30 mL). L'iodure de sodium (1,03 g ; 6,88 mmol ; 3 éq.) est additionné et le mélange réactionnel est agité pendant 5 minutes. L'acide méthane sulphonique MeSO₃H (0,66 g ; 0,45 mL ; 6,88 mmol ; 3 éq.) est additionné lentement à 0°C et la mixture est alors réchauffée à température ambiante et agitée pendant une nuit. BaCO₃ (0,54 g ; 2,75 mmol ; 1,2 éq.) et un mélange eau / acétone (25 mL) sont ajoutés dans le milieu à 0°C qui est alors agité pendant une heure à température ambiante. Une solution aqueuse à 10% de thiosulfate de sodium Na₂S₂O₃ (30 mL) est additionnée à la réaction, qui est ensuite extraite avec du dichlorométhane (3 x 30 mL). Les phases organiques sont collectées et lavées avec une solution saturée de chlorure de sodium (50 mL), séchées sur sulfate de magnésium MgSO₄ et concentrées sous pression réduite.

Le solide rouge résultant est purifié par chromatographie sur gel de silice avec comme éluant un mélange dichlorométhane / acétate d'éthyle dans des proportions de huit pour deux.

L'épipodophyllotoxine **12** (0,17 g) et la 4'-déméthylépipodophyllotoxine **11** (0,44 g) sont isolées sous la forme de solides roses pâles avec un rendement pondéral de 66 %.

### Caractérisation de l'épipodophyllotoxine 12:

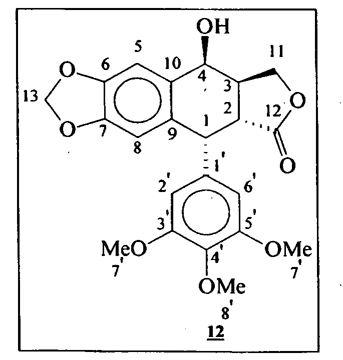

Rf = 0,38, éluant : DCM / acétate d'éthyle (8 : 2).
**RMN ¹H (CDCl₃, 300MHz)**
2,77 (dddd, 1H, ³J_{H3-H2} 14.1, ³J_{H3-H11} 4.1, ³J_{H3-H4} 3.3, ³J_{H3-H11} 1.8, H3) ; 3,21 (dd, 1H, ³J_{H2-H3} 14.1, ³J_{H2-H1} 5.1, H2) ; 3,67 (s, 6H, H7' x 6) ; 3,73 (s, 3H, H8' x 3) ; 4,28 (d, 1H, ³J_{H11-H3} 1.8, H11) ; 4,31 (d, 1H, ³J_{H11-H3} 4.1, H11) ; 4,54 (d, 1H, ³J_{H1-H2} 5.1, H1); 4,79 (d, 1H, ³J_{H4-H3} 3.3, H4) ; 5,91 (dd, 2H, ²J_{H13-H13} 8.1, 1.3, H13) ; 6,21 (s, 2H, H2', H6') ; 6,48 (s, 1H, H8) ; 6,81 (s, 1H, H5).
**RMN ¹³C (CDCl₃, 75MHz)**
38,1 (C3) ; 40,5 (C2) ; 43,8 (C1) ; 56,1 (2C ; C7' x 2) ; 60,6 (C8') ; 66,6 (C4) ; 67,5 (C11) ; 101,5 (C13) ; 107,7 (2C; C2' ; C6'); 108,8 (C5); 110,4 (C8); 131,7; 131,8 ; 134,9 ; 137,0 ; 147,4 (C7) ; 148,4 (C6) ; 152,4 (2C ; C3' ; C5') ; 174,9 (C12).

### Caractérisation de la 4'-déméthylépipodophyllotoxine 11:

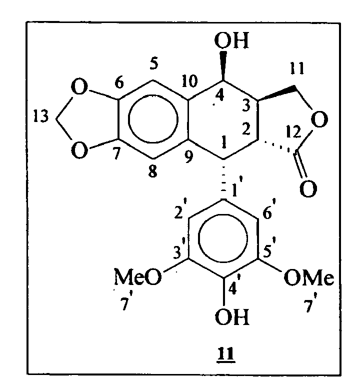

Rf = 0,23, éluant : DCM / acétate d'éthyle (8 : 2).
**RMN ¹H (CDCl₃, 300MHz)**
2,75 (dddd, 1H, ³J_{H3-H2} 14.1, ³J_{H3-H11} 6.3, ³J_{H3-H1} 3.8, ³J_{H3-H4} 3.5, H3) ; 3,19 (dd, 1H, ³J_{H2-H3} 14.1, ³J_{H2-H1} 5.1, H2) ; 3,70 (s, 6H, H7' x 6) ; 4,27 (d, 1H, ³J_{H11-H3} 3.8, H11) ; 4,31 (d, 1H, ³J_{H11-H3} 6.3, H11) ; 4,54 (d, 1H, ³J_{H1-H2} 5.1, H1) ; 4,79 (d, 1H, ³J_{H4-H3} 3.5, H4) ; 5,35 (s, 1H, OH) ; 5,92 (dd, 2H, ³J_{H13-H13} 8.8, 1.3, H13) ; 6,22 (s, 2H, H2', H6') ; 6,48 (s, 1H, H8) ; 6,81 (s, 1H, H5).
**RMN ¹³C (CDCl₃, 75MHz)**
38,6 (C3) ; 41,0 (C2) ; 44,1 (C1) ; 56,8 (2C ; C7' x 2) ; 67,2 (C4) ; 68,0 (C11) ; 101,9 (C13) ; 108,2 (2C; C2' ; C6'); 109,3 (C5); 110,9 (C8); 130,9; 132,2; 132,5; 134,4 ; 146, (2C ; C3' ; C5') ; 147,8 (C7) ; 148,9 (C6) ; 175,5 (C12).

### Synthèse de la 4β-azido-4-déoxy-4'-déméthylépipodophyllotoxine 13 (Fig. 2)

Sur une solution contenant la 4'-déméthylépipodophyllotoxine **11** (1,65 g ; 4,1 mmol ; 1 éq.) et l'azoture de sodium NaN₃ (1,4 g ; 21,0 mmol ; 5 éq.) dans le chloroforme CHCl₃ (15 mL), l'acide trifluoroacétique CF₃COOH (4,5 mL; 5,8 mmol ; 1,4 éq.) est additionné goutte à goutte et le milieu est agité pendant 2h à température ambiante. Une solution saturée d'hydrogénocarbonate de sodium NaHCO₃ (10 mL) est ajoutée, le mélange est ainsi extrait au chloroforme (3 x 20 mL). Les phases organiques sont combinées, lavées avec de l'eau (40 mL), séchées sur sulfate de magnésium MgSO₄ et concentrées pour donner le 4-azido-4-déoxy-4'-déméthylépipodophyllotoxine **13** sous la forme d'un solide jaune avec un rendement quantitatif. Le produit est suffisamment pur pour être utilisé pour la prochaine étape sans plus de purification.

### Caractérisation de 4β-azido-4-déoxy-4'-déméthylépipodophyllotoxine 13

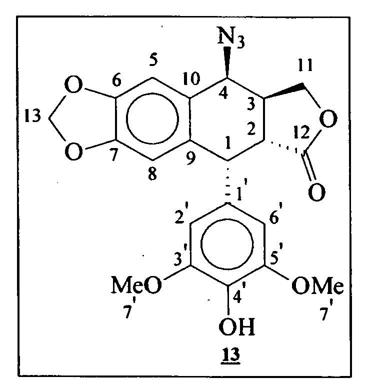

Rf = 0,56, éluant : dichlorométhane / acétate d'éthyle (8 : 2).
**RMN ¹H (CDCl₃, 300MHz)**
2,86 (m, 1H, H3) ; 3,10 (dd, 1H, ³J_{H2-H3} 13.8, ³J_{H2-H1} 5.2, H2) ; 3,70 (s, 6H, H7' x 6) ; 4,23 (dₐₚₚ, 2H, ³J_{H11-H3} 9.4, H11 x 2) ; 4,55 (d, 1H, ³J_{H1-H2} 5.2, H1) ; 4,70 (d, 1H, ³J_{H4-H3} 3.7, H4) ; 5,36 (s, 1H, -OH) ; 5,95 (dd, 2H, ³J_{H13-H13} 6.3, 1.2, H13) ; 6,20 (s, 2H, H2', H6') ; 6,52 (s, 1H, H8) ; 6,73 (s, 1H, H5).
**RMN ¹³C (CDCl₃, 75MHz)**
35,8 (C3) ; 40,3 (C2) ; 42,5 (C1) ; 55,5 (2C, C7' x 2) ; 58,2 (C4) ; 66,8 (C11) ; 100,8 (C13); 106,8 (2C, C2', C6'); 107,6 (C5); 110,1 (C8); 125,8; 129,5; 131,3; 133,2 ; 145,4 (2C, C3', C5') ; 146,4 (C7) ; 147,9 (C6) ; 173,1 (C12).

### Synthèse du 4β-azido-4-déoxyépipodophyllotoxine 14 (Fig. 3)

Dans une solution contenant l'épipodophyllotoxine **12** (670 mg ; 1,6 mmol ; 1 éq.) et l'azoture de sodium NaN₃ (530 mg ; 8,1 mmol ; 5 éq.) dans le chloroforme CHCl₃ (8 mL), de l'acide trifluoroacétique CF₃COOH (0,55 mL ; 2,2 mmol ; 1,4 éq.) est additionné goutte à goutte et le milieu est agité pendant deux heures à température ambiante. Une solution saturée d'hydrogénocarbonate de sodium NaHCO₃ (10 mL) est ajoutée, le mélange est ainsi extrait au chloroforme (3 x 15 mL). Les phases organiques sont combinées, lavées avec de l'eau (30 mL), séchées sur sulfate de magnésium MgSO₄ et concentrées pour donner le 4β-azido-4-déoxyépipodophyllotoxine **14** sous la forme d'un solide jaune avec un rendement quantitatif. Le produit est suffisamment pur pour être utilisé pour la prochaine étape sans plus de purification.

### Caractérisation de 4β-azido-4-déoxyépipodophyllotoxine 14

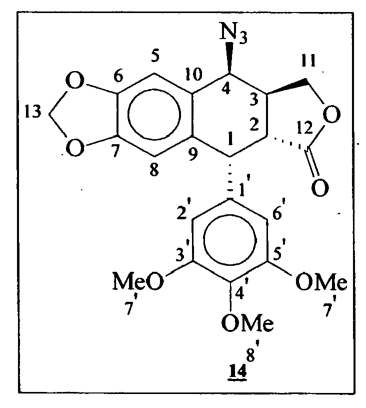

Rf = 0,86 ; éluant : dichlorométhane / acétate d'éthyle (8 : 2).
**RMN ¹H (CDCl₃, 300MHz)**
2,84-2,91 (m, 1H, H3) ; 3,12 (dd, 1H, ³J_{H2-H3} 13.8, ³J_{H2-H1} 5.2, H2) ; 3,67 (s, 6H, H7' x 6) ; 3,73 (s, 3H, H8' x 3) ; 4,24 (d, 1H, ³J_{H11-H3} 2.5, H11) ; 4,26 (d, 1H, ³J_{H11-H11} 0.6, H11) ; 4,56 (d, 1H, ³J_{H1-H2} 5.2, H1) ; 4,71 (d, 1H, ³J_{H4-H3} 3.7, H4) ; 5,95 (dd, 2H, ³J_{H13-H13} 5.5, 1.3, H13) ; 6,19 (s, 2H, H2', H6') ; 6,52 (s, 1H, H8) ; 6,74 (s, 1H, H5).
**RMN ¹³C (CDCl₃, 75MHz)**
38,7 (C3) ; 43,0 (C2) ; 45,4 (C1) ; 58,1 (2C, C7' x 2) ; 61,4 (C4) ; 62,5 (C8') ; 69,4 (C11) ; 103,8 (C13) ; 110,0 (2C, C2', C6') ; 110,6 (C5) ; 112,9 (C8) ; 128,6, 133,9; 136,8 ; 139,1 ; 149,1 (C7) ; 150,8 (C6) ; 154,4 (2C, C3', C5') ; 175,9 (C12).

### Synthèse du 4β-amino-4-déoxy-4'-déméthylépipodophyllotoxine 15 (Fig. 4)

Le 4β-azido-4-déoxy-4'-déméthylépipodophyllotoxine **13** (354 mg ; 0,83 mmol) est dissous dans de l'acétate d'éthyle (25 mL) et du palladium sur charbon est additionné. Le milieu réactionnel est placé sous atmosphère d'hydrogène et agité pendant une nuit à température ambiante. Le mélange est filtré et concentré sous pression réduite pour donner un solide qui est purifié par flash chromatographie sur gel de silice avec comme éluant un mélange dichlorométhane / acétate d'éthyle dans des proportions de huit pour deux pour obtenir ainsi le 4β-amino-4-déoxy-4'-déméthylépipodophyllotoxine **15** sous forme de solide blanc avec un rendement pondéral de 67%.

### Caractérisation de 4β-amino-4-déoxy-4'-déméthylépipodophyllotoxine 15

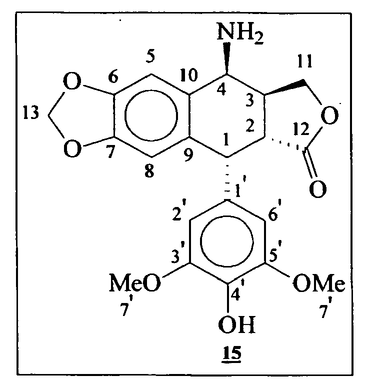

Rf = 0,21 ; éluant : chloroforme/ méthanol (19 : 1).
**RMN ¹H (CDCl₃, 300MHz)**
2,57-2,82 (m, 3H, H3, NH₂) ; 3,21 (dd, 1H, ³J_{H2-H3} 14.1, ³J_{H2-H1} 5.2, H2) ; 3,69 (s, 6H, H7' x 6) ; 4,13 (d, 1H, ³J_{H4-H3} 4.0, H4) ; 4,22 (dₐₚₚ, 2H, ³J_{H11-H3} 9.6, H11 x 2) ; 4,48 (d, 1H, ³J_{H1-H2} 5.2, H1) ; 5,88 (dd, 2H, ³J_{H13-H13} 7.7, 1.2, H13) ; 6,23 (s, 2H, H2', H6') ; 6,41 (s, 1H, H8) ; 6,74 (s, 1H, H5).
**RMN ¹³C (CDCl₃, 75MHz)**
38,3 (C3) ; 40,7 (C2) ; 44,1 (C1) ; 49,3 (C4) ; 56,8 (2C, C7' x 2) ; 68,6 (C11) ; 101,8 (C13) ; 108,4 (2C, C2', C6'); 109,4 (C5); 110,3 (C8); 131,5; 131,6; 134,3 ; 134,4 ; 146,8 (2C, C3', C5') ; 147,7 ; 148,0 ; 175,9 (C12).

### Synthèse du 4β-amino-4-déoxyépipodophyllotoxine 16 (Fig. 5)

Le 4-azido-4-déoxyépipodophyllotoxine **14** (1150 mg ; 2,62 mmol) est dissous dans de l'acétate d'éthyle (25 mL) et du palladium sur charbon est additionné. Le milieu réactionnel est placé sous atmosphère d'hydrogène et agité pendant une nuit à température ambiante. Le mélange est filtré et concentré sous pression réduite pour donner un solide qui est purifié par flash chromatographie sur gel de silice avec comme éluant un mélange dichlorométhane / acétate d'éthyle dans des proportions de huit pour deux pour obtenir ainsi le 4β-amino-4-déoxyépipodophyllotoxine **16** sous forme de solide jaune pâle avec un rendement pondéral de 81%.

### Caractérisation de 4β-amino-4-déoxyépipodophyllotoxine 16

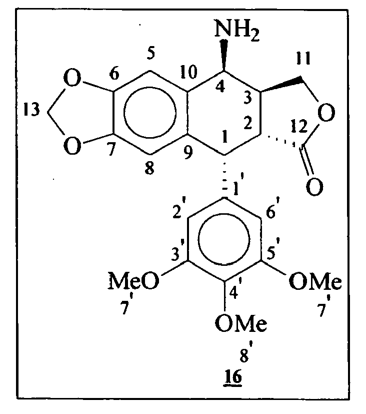

Rf = 0,29, éluant : CHCl₃ / méthanol (19 : 1).
**RMN ¹H (CDCl₃, 300MHz)**
2,21 (ls, 2H, -NH₂) ; 2,75-2,85 (m, 1H, H3) ; 3,25 (dd, 1H, ³J_{H2-H3} 14.1, ³J_{H2-H1} 5.4, H2) ; 3,67 (s, 6H, H7' x 6) ; 3,72 (s, 3H, H8' x 3) ; 4,18 (d, 1H, ³J_{H4-H3} 4.2, H4) ; 4,22 (dd, 1H, ³J_{H11-H3} 8.8, ³J_{H11-H11} 0.9, H11 x 2) ; 4,25 (s, 1H, H11) ; 4,49 (d, 1H, ³J_{H11-H2} 5.4, H1) ; 5,88 (dd, 2H, ³J_{H13-H13} 6.4, 1.3, H13) ; 6,22 (s, 2H, H2', H6') ; 6,42 (s, 1H, H8) ; 6,77 (s, 1H, H5).
**RMN ¹³C (CDCl₃, 75MHz)**
38,2 (C3) ; 40,6 (C2) ; 44,3 (C1) ; 49,4 (C4) ; 56,6 (2C, C7' x 2) ; 61,1 (C8') ; 68,5 (C11) ; 101,8 (C13) ; 108,6 (2C, C2', C6') ; 109,1 (C5) ; 110,7 (C8); 131,6; 133,7; 136,0 ; 137,4, 147,8 (C7) ; 148,2 (C6) ; 152,9 (2C, C3', C5') ; 175,6 (C12).

### Synthèse de la N-méthyl-4β-amino-4-déoxyépipodophyllotoxine 17 (Fig. 6)

La 4β-amino-4-déoxy-4'-déméthylépipodophyllotoxine **15** (115 mg; 0.29 mmol ; 1 éq.) est dissoute dans du tétrahydrofurane anhydre (5 mL) et est ajoutée lentement à une solution d'hydrure de sodium (21 mg ; 0.86 mmol ; 3 éq.) dans le tétrahydrofurane (2 mL). La solution est ainsi agitée pendant 30 minutes à température ambiante. L'iodure de méthyle (123 mg ; 0.86 mmol ; 3 éq.) est additionné à température ambiante et le mélange réactionnel est agité pendant la nuit. De l'eau (10 mL) est additionnée pour stopper la réaction. Le mélange est ensuite extrait avec du dichlorométhane (3 x 10 mL). Les phases organiques sont collectées et lavées avec une solution saturée de chlorure de sodium (20 mL), séchées sur sulfate de magnésium MgSO₄ et concentrées sous pression réduite. L'huile jaune en résultant est purifiée par chromatographie sur gel de silice avec 100% d'acétate d'éthyle. La N-méthyl-épipodophyllotoxine **17** (54 mg) est isolée comme une huile incolore avec un rendement de 44 %.

### Caractérisation de N-méthyl-4β-amino-4-déoxyépipodophyllotoxine 17

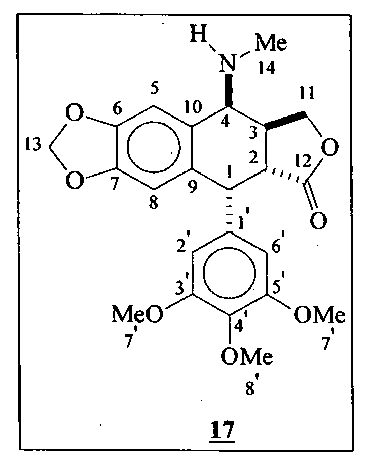

Rf = 0.14, éluant : acétate d'éthyle.
**RMN ¹H (CDCl₃, 300MHz)**
1.95 (ls, 1H, N-H), 2.50 (ls, 1H, H2), 2.60-2.70 (dd, 1H, ²J_{H11-H11} 9.4, 5.6, H3), 2.71 (s, 3H, H14 x 3), 3.59-3.65 (m, 2H, H11 x 2), 3.76 (s, 6H, H7' x 6), 3.81 (s, 3H, H8' x 3), 4.09 (s, 1H, H4), 4.29 (s, 1H, H1), 5.94 (dd, 2H, ²J_{H13-H13} 12.7, 1.3, H13 x 2), 6.30 (s, 2H, H2', H6'), 6.48 (s, 1H, H8), 6.67 (s, 1H, H5).
**RMN ¹³C (CDCl₃, 75MHz)**
27.6 (C14), 42.6 (C3), 45.9 (C1), 51.0 (C2), 55.9 (2C, C7' x 2), 60.5 (C8'), 61.8 (C11), 61.8 (C4), 100.9 (C13), 105.7 (2C, C2', C6'), 107.0 (C5), 111.9 (C8), 128.8, 131.7, 136.4, 139.8, 145.4 (C7), 147.3 (C6), 152.8 (2C, C3', C5'), 175.2 (C12). **Spectrométrie de masse** : 428 (M + H)+, 414 (M + H - Me)+.

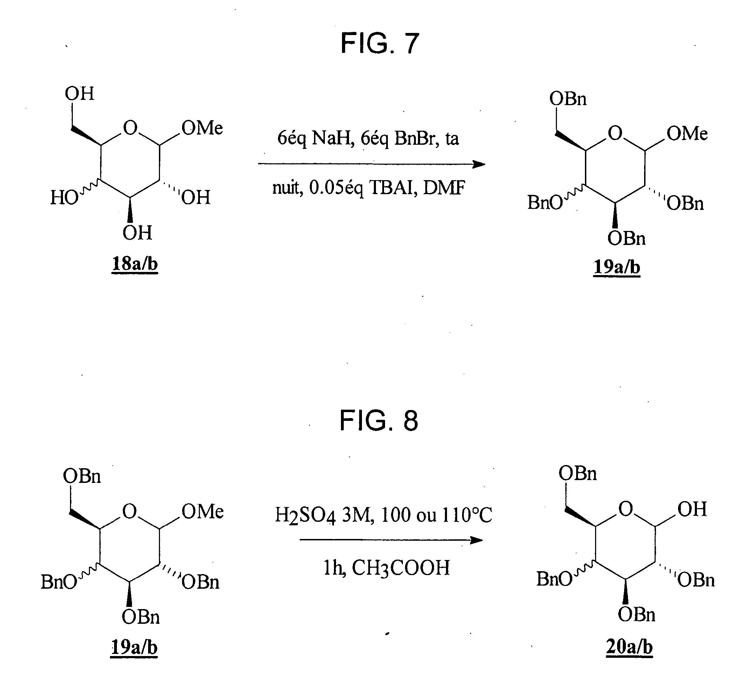

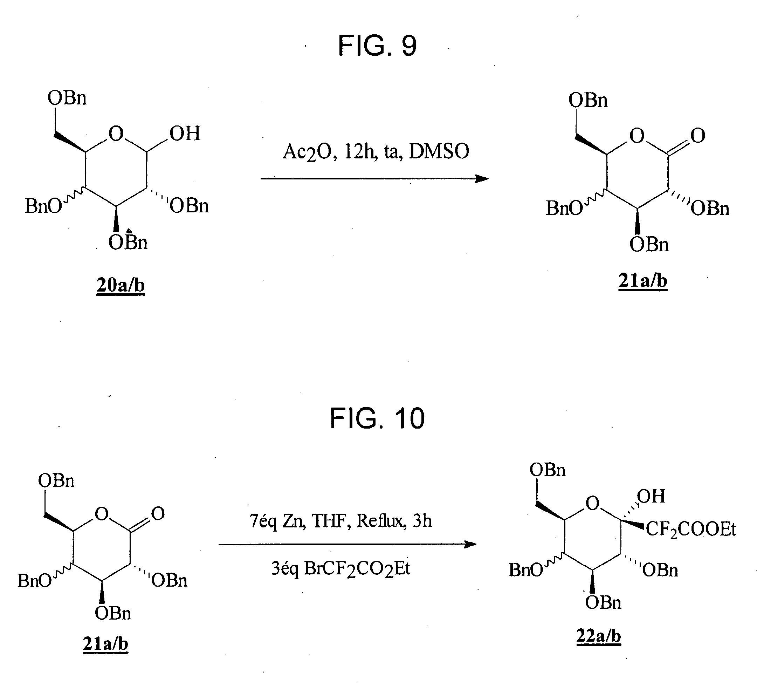

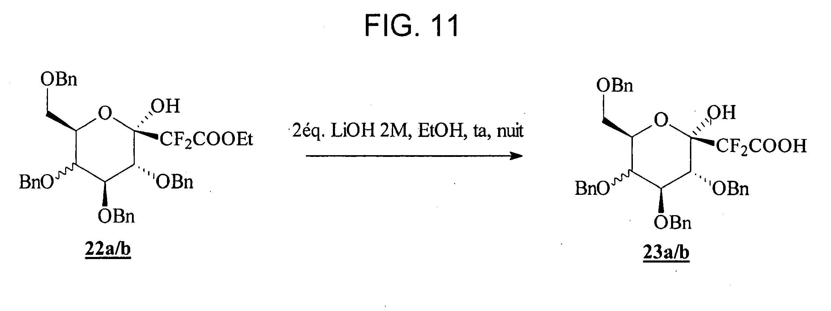

Les étapes de benzylation d'un sucre **18** (Fig. 7), d'hydrolyse acide de la position anomérique d'un composé **19** (Fig. 8), et d'oxydation d'un composé **20** (Fig. 9) conduisent à une lactone **21.** Sur cette lactone **21,** l'introduction du motif difluoroester sur un composé **22** est réalisée via une réaction de Reformatsky (Fig. 10). Cette fonction ester est ensuite saponifiée pour donner un composé **23** (Fig. 11) en vue de l'étape de couplage.

Les résultats expérimentaux sont donnés en série glucose, les références des produits étant suivies de la lettre a, et galactose, les références des produits étant suivies de la lettre b :

### Synthèse du 1-O-methyl-2,3,4,5,6-tetra-O-benzyl-D-glucopyranose 19a (Fig. 7)

Dans un ballon sous atmosphère d'argon, le 1-*O*-méthyl-D-glucopyranose **18a** (5 g ; 26 mmol ; 1 éq.) et de l'iodure de tetrabutylammonium nBu₄NI (0,5 g ; 1,3 mmol ; 0,05 éq.) sont placés en solution dans du diméthyformamide DMF (250 mL).
L'hydrure de sodium NaH (3,7 g ; 150 mmol ; 6 éq.) est introduit lentement. Le bromure de benzyle BnBr (18 mL ; 150 mmol ; 6 éq.) est ensuite additionné et la réaction est agitée pendant 24h à température ambiante. L'eau (200 mL) est additionnée lentement et la phase aqueuse est extraite avec de l'éther (3 x 150 mL). Les phases organiques sont collectées, séchées sur sulfate de magnésium MgSO₄ et concentrées sous pression réduite.
Le produit **19a** est purifié par chromatographie sur colonne de silice avec comme éluant un mélange cyclohexane / acétate d'éthyle (9/1).
Le produit **19a** est isolé sous forme d'huile incolore avec un rendement pondéral de 83%.

### Caractérisation de 19a

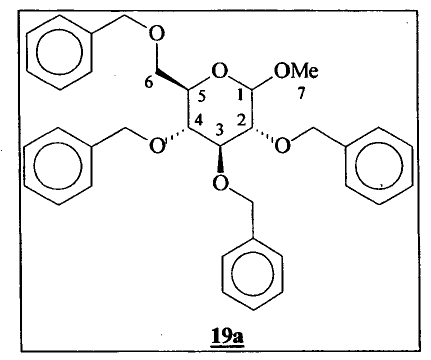

Rf= 0,38, éluant : cyclohexane / acétate d'éthyle (9 : 1).
**RMN ¹H (CDCl₃, 300MHz)**
3,29 (s, 3H, H7) ; 3,45-3,67 (m, 5H, H2, H3, H4, 2 x H6) ; 3,91 (t, 1H, ³J_{H5-H6} 8.9, H5) ; 4,36-4,92 (m, 9H, 4 x CH₂Ph, H1) ; 7,04-7,31 (m, 20H, H_{Ar}).
**RMN ¹³C (CDCl₃, 75MHz)**
53,8 (C7) ; 67,1 ; 68,7 ; 72,1 ; 72,1 ; 73,7 ; 74,4 ; 76,3 ; 78,5 ; 80,8 ; 96,9 (C1) ; 126,3 ; 126,3 ; 126,4 ; 126,5 ; 126,6 (2C) ; 126,6 ; 126,8 ; 127,0 (2C) ; 127,1 ; 127,1 ; 136,6 ; 136,8 ; 136,9 ; 137,5.

### Synthèse du 1-O-methyl-2,3,4,5,6-tetra-O-benzyl-D-galactopyranose 19b (Fig. 7)

Dans un ballon sous atmosphère d'argon, le 1-*O*-méthyl-D-galactopyranose **18b** (5 g ; 26 mmol ; 1 éq.) et de l'iodure de tetrabutylammonium (0,5 g ; 1,3 mmol ; 0,05 éq.) sont placés en solution dans le DMF (250 mL). L'hydrure de sodium (3,7 g ; 150 mmol ; 6 éq.) est introduit lentement. Le bromure de benzyle (18 mL ; 150 mmol ; 6 éq.) est ensuite additionné et la réaction est agitée pendant 24h à température ambiante. L'eau (200 mL) est additionnée lentement et la phase aqueuse est extraite avec de l'éther (3 x 150 mL). Les phases organiques sont collectées, séchées sur sulfate de magnésium MgSO₄ et concentrées sous pression réduite.
Le produit **19b** est purifié par chromatographie sur colonne de silice avec comme éluant un mélange cyclohexane / acétate d'éthyle (9/1).
Le produit **19b** est isolé sous forme d'huile incolore avec un rendement pondéral de 95%.

### Caractérisation de 19b

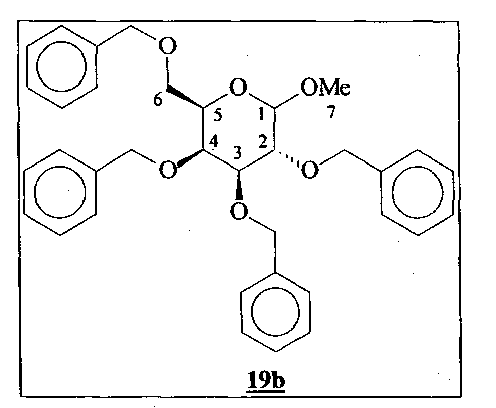

Rf = 0,48, éluant : cyclohexane / acétate d'éthyle (8 : 2).
**RMN ¹H (CDCl₃, 300MHz)**
3,3 et 3,4 (2s, 3H, CH₃) 3,4-3,5 (m , 2H6, H5, 0,5H3_{β}) ; 3,7 (dd , 7,7-9,6, 0,5H, H2_{β}) ;3,8-3,9 (m, 2H, 0,5H3_{α}, H4); 4 (dd, 3,5_10,8, 0,5H, H2_{α}) ;4,2 (d, 7,7, 0,5H, H1_{β}) ; 4,6 (d, 3,5, 0,5H, H1_{α}) ; 4,3-4,9 (m, 8H, H**2**, 4OCH₂Ph) ; 7,2 (m, 20H, H ar.)
**RMN ¹³C (CDCl₃, 75,5MHz)**
55,8 et 57,5 (CH₃) ; 69,3 et 69,5 (C6) ; 69,6 (C5) ; 73,4 ; 73,7 ; 73,8 (C4); 73,9 ; 74,0 ; 74,8 ; 75,2 ; 75,6 ; 76,9 ; 79,5 et 80,1 (C2) ; 82,6 (C3) ; 99,2 et 105,4 (C1) ; 127,9-128 (Car.) ; 138- 139 (Car. quat.).

### Synthèse du 2,3,4,5,6-tetra-O-benzyl-D-glucopyranose 20a (Fig. 8)

Dans un ballon contenant le 1-*O*-méthyl-2,3,4,5,6-tetra-*O*-benzyl-D-glucopyranose **19a** (6,4 g ; 11,54 mmol) en solution dans de l'acide acétique (93 mL), une solution d'acide sulfurique 3M (13 mL) est additionnée et le mélange réactionnel est chauffé à 110°C pendant une heure. La réaction est refroidie à température ambiante. Un précipité blanc apparaît et celui-ci est filtré et séché à l'air ambiant.
Le composé **20a** est obtenu très pur sous la forme d'un solide blanc avec un rendement pondéral de 59% et est engagé directement dans la prochaine étape sans purification.

### Caractérisation de 20a

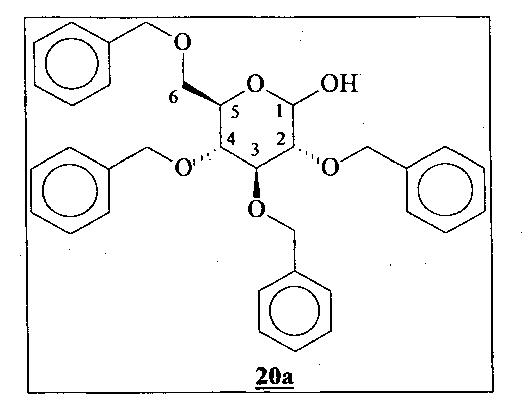

Rf = 0,35 ; éluant : cyclohexane / acétate d'éthyle (7 : 3).
La RMN montre clairement deux anomères (α et β) en RMN du carbone.
**RMN ¹H (CDCl₃, 300MHz)**
3,58-3,66 (m, 4H, H2, H3, 2 x H6) ; 3,94-4,06 (m, 2H, H4, H5) ; 4,44-4,96 (m, 8H, 4 x CH₂Ph) ; 5,21 (d, 1H, ³J_{H1-H2} 3,5, H1) ; 7,04-7,32 (m, 20H, H_{Ar}).
**RMN ¹³C (CDCl₃, 75MHz)**
Anomère majoritaire : 69,0 (C6) ; 70,6 ; 73,6 ; 73,9 ; 75,4 ; 76,1 ; 78,1 ; 80,4 ; 82,1 ; 91,7 (C1) ; 128,0 ; 128,1 ; 128,1 ; 128,3 ; 128,4 ; 128,4 (2C) ; 128,5 ; 128,6 ; 128,8 (2C) ; 128,8 (2C) ; 128,9 ; 138,2 ; 138,3 ; 138,6 ; 139,1.
Anomère minoritaire : 69,3 (C6) ; 75,0 ; 75,2 ; 78,2 ; 83,5 ; 85,0 ; 97,9 (C1) ; 138,1 ; 138,4 ; 138,7; 138,9.
**Point de fusion :**
Pt_{f} = 151°C

### Synthèse du 2,3,4,5,6-tetra-O-benzyl-D-galactopyranose 20b (Fig. 8)

Dans un ballon contenant le 1-O-méthyl-2,3,4,5,6-tetra-O-benzyl-D-galactopyranose **19b** (6,4 g ; 11,54 mmol) en solution dans l'acide acétique (93 mL), une solution d'acide sulfurique 3M (13 mL) est additionnée et le mélange réactionnel est chauffé à 100°C pendant une heure. La réaction est refroidie à température ambiante. Le mélange est extrait quatre fois avec 100 mL de toluène. Les phases organiques sont rassemblées, puis lavées avec 100 mL d'une solution saturée d'hydrogénocarbonate de sodium NaHCO₃ et enfin avec 100 mL d'eau. La phase organique est ensuite concentrée.
Le produit brut ainsi obtenu est purifié par chromatographie sur une colonne de silice avec comme éluant un mélange cyclohexane / acétate d'éthyle dans des proportions de 8,5 pour 1,5.
Après concentration des fractions recueillies, le produit **20b** se présente sous la forme de cristaux blancs avec un rendement pondéral de 75%.

### Caractérisation de 20b

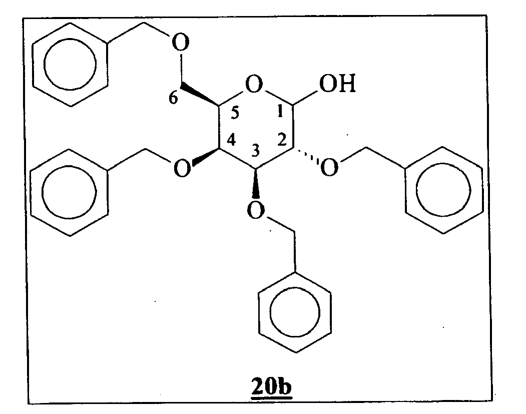

Rf = 0,67, éluant : cyclohexane / acétate d'éthyle (6 : 4).
La RMN montre clairement deux anomères (α et β) en RMN carbone.

### Synthèse de la lactone dérivée du glucose 21a (Fig. 9)

Dans un ballon contenant le 2,3,4,5,6-tetra-*O*-benzyl-D-glucopyranose **20a** (2,9 g ; 5,35 mmol) sous atmosphère inerte, du diméthylsulphoxyde DMSO (19 mL) est additionné avec de l'anhydride acétique (13 mL). Le mélange réactionnel est agité pendant une nuit à température ambiante. Une solution saturée de NaHCO₃ (20 mL) est additionnée et la mixture est extraite deux fois avec de l'éther (20 mL). Les phases organiques sont rassemblées et lavées avec de l'eau (10 mL) dix fois, séchées sur sulfate de magnésium et concentrées.

Le brut réactionnel est purifié par chromatographie sur gel de silice avec comme éluant un mélange cyclohexane / acétate d'éthyle dans des proportions de huit pour deux.

Le produit désiré **21a** est ainsi isolé sous forme d'une huile incolore avec un rendement pondéral de 82%.

### Caractérisation de 21a

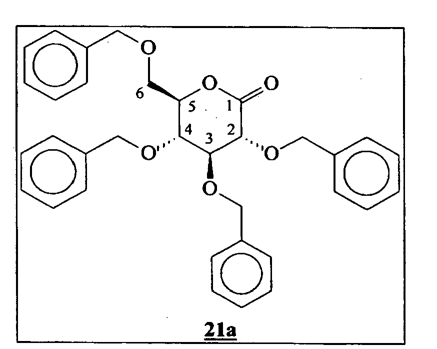

**CCM**
Rf = 0,61, éluant : cyclohexane / acétate d'éthyle (8 : 2).
**RMN ¹H (CDCl₃, 300MHz)**
3,65 (dd, 1H, ³J_{H5-H6} 3.3, ³J_{H6-H6} 11.0, H6) ; 3,72 (dd, 1H, ³J_{H6-H5} 2.4, ³J_{H6-H6} 11.0, H6) ; 3,89-3,95 (m, 2H, H3, H4) ; 4,13 (d, 1H, ³J_{H2-H3} 6.8, H2) ; 4,44-4,74 (m, 8H, CH₂, H5) ; 4,96 (dapp, 1H, ³J 11.4) ; 7,15-7,34 (m, 20H, H_{Ar}).
**RMN ¹³C (CDCl₃, 75MHz)**
68,6 (C6) ; 73,8 ; 74,0 ; 74,1 ; 74,1 ; 76,4 ; 77,8 ; 78,5 ; 81,2 ; 128,2 ; 128,3 ; 128,3 ; 128,4 (2C) ; 128,5 ; 128,7 ; 128,2 (2C) ; 128,8 ; 137,4 ; 137,9 ; 137,9 ; 138,0 ; 166,9 (C1).

### Synthèse de la lactone dérivée du galactose 21b (Fig. 9)

Dans un ballon contenant le 2,3,4,5,6-tetra-*O*-benzyl-D-galactopyranose **20b** (2,9 g ; 5,35 mmol) sous atmosphère inerte, du DMSO (19 mL) est additionné avec l'anhydride acétique (13 mL). Le mélange réactionnel est agité pendant une nuit à température ambiante. Une solution saturée de NaHCO₃ (20 mL) est additionnée et la mixture est extraite deux fois avec de l'éther (20 mL). Les phases organiques sont rassemblées et lavées avec de l'eau (10 mL) dix fois, séchées sur sulfate de magnésium et concentrées.
Le brut réactionnel est purifié par chromatographie sur gel de silice avec comme éluant un mélange cyclohexane / acétate d'éthyle dans des proportions de huit pour deux. Le produit désiré **21b** est ainsi isolé sous forme d'un solide blanc avec un rendement pondéral de 82%.

### Caractérisation de 21b

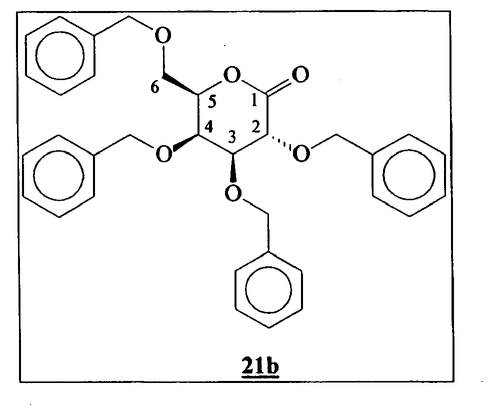

Rf = 0,61, éluant : cyclohexane / acétate d'éthyle (8 : 2).
**RMN ¹H (CDCl₃, 300MHz)**
3,6 (m, 2H, H6) ; 3,8 (dd, 2,1-9,6, 1H, H3) ; 4,1 (s, 1H, H4) ; 4,2 (m, 1H, H5) ; 4,4-5,1 (m ,9H, H2 ; 40CH₂ Bn H6) ; 7,2 (m, 20H, H ar.)
**RMN ¹³C (CDCl₃, 75MHz)**
67,4 (C6) ; 72,4 (C4) ; 72,6 (OCH₂Bn) ; 73,5 (OCH₂Bn) ; 74,6 (OCH₂Bn) ; 75,1 (OCH₂Bn) ; ; 77,1 (C5); 77,2 (C2) ; 79,9 (C3) ; 127,4 -128,3 (Car.) ; 137,2 ; 137,3 ; 137,6 (Car. quat.) ; 169,8 (CO).

### Synthèse du gem-difluoroester dérivé du glucose 22a (Fig. 10)

Dans un ballon sous atmosphère inerte contenant du zinc (3,34 g ; 51 mmol ; 7 éq.) préalablement activé, du tétrahydrofurane THF (60 mL) est ajouté et le mélange est ainsi chauffé à reflux. Une solution constituée de la lactone dérivée du glucose **21a** (3,94 g ; 7,3 mmol ; 1 éq.) et du bromodifluoroacétate d'éthyle (2,83 mL ; 22 mmol ; 3 éq.) dans du THF (60 mL) est lentement additionnée au mélange précédent. La réaction est agitée à reflux pendant trois heures. Le mélange est refroidi à température ambiante et une solution d'acide chlorhydrique HCl 1M (120 mL) est ajoutée, suivie de l'addition de dichlorométhane (120 mL). La phase aqueuse est extraite avec du dichlorométhane (100 mL) deux fois. Les phases organiques sont séchées sur sulfate de magnésium MgSO₄, filtrées et concentrées sous pression réduite. Le résidu est purifié sur colonne chromatographique sur gel de silice avec comme éluant un mélange cyclohexane / acétate d'éthyle dans des proportions de huit pour deux.
Le produit pur **22a** est isolé sous forme d'une huile incolore avec un rendement pondéral de 75%.

### Caractérisation de 22a

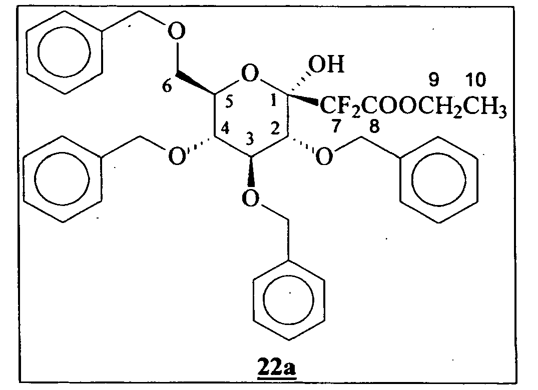

Rf = 0,35, éluant : cyclohexane / acétate d'éthyle (8 : 2).
**RMN ¹H (CDCl₃, 300MHz)**
1,30 (t, 3H, ³J_{H9-H10} 7.1, H10) ; 3,63-3,81 (m, 3H, H3, 2 x H6) ; 4,01-4,10 (m, 2H, H4, H5) ; 4,16 (s, 1H, H2) ; 4,29 (q, 2H, ³J_{H9-H10} 7.1, 2 x H9) ; 4,50-4,81 (m, 4H) ; 4,85-4,92 (m, 4H) ; 7,21-7,38 (m, 20H, H_{Ar}).
**RMN ¹³C (CDCl₃, 75MHz)**
14,3 (C10) ; 63,7 (C9) ; 68,6 (C6) ; 73,0 (C2) ; 73,8 ; 75,5 ; 75,7 ; 76,4 ; 77,8 (C3) ; 78,6 (C4) ; 83,7 (C5) ; 96,5 (t ; ²J_{C-F} 25,5 ; C1) ; 128,0 (2C) ; 128,1 (2C) ; 128,1 ; 128,2 ; 128,3 (2C) ; 128,6 ; 128,7 (2C) ; 128,8 (2C) ; 128,8 (2C) ; 128,9 (2C) ; 137,9; 138,3 ; 138,7; 138,7 ; 163,3 (t ; ²J_{C-F} 30,3 ; C8).
**RMN ¹⁹F (CDCl₃, 282MHz)**
-117,7 (d, 1F, ²J_{F-F} 256.4) ; -120,1 (d, 1F, ²J_{F-F} 256.4).

### Synthèse du gem-difluoroester dérivé du galactose 22b (Fig. 10)

Dans un ballon sous atmosphère inerte contenant du zinc (3,34 g ; 51 mmol ; 7 éq.) préalablement activé, du THF (60 mL) est ajouté et le mélange est ainsi chauffé à reflux. Une solution constituée de la lactone dérivée du galactose **21b** (3,94 g ; 7,3 mmol ; 1 éq.) et du bromodifluoroacétate d'éthyle (2,83 mL ; 22 mmol ; 3 éq.) dans du THF (60 mL) est lentement additionnée au mélange précédent. La réaction est agitée à reflux pendant trois heures. Le mélange est refroidi à température ambiante et une solution d'acide chlorhydrique HCl 1M (120 mL) est ajoutée, suivie de l'addition de dichlorométhane (120 mL). La phase aqueuse est extraite avec du dichlorométhane (100 mL) deux fois. Les phases organiques sont séchées sur sulfate de magnésium MgSO₄, filtrées et concentrées sous pression réduite.
Le résidu est purifié sur colonne chromatographique sur gel de silice avec comme éluant un mélange cyclohexane / acétate d'éthyle dans des proportions de huit pour deux.
Le produit pur **22b** est isolé sous forme d'un solide blanc avec un rendement pondéral de 82%.

### Caractérisation de 22b

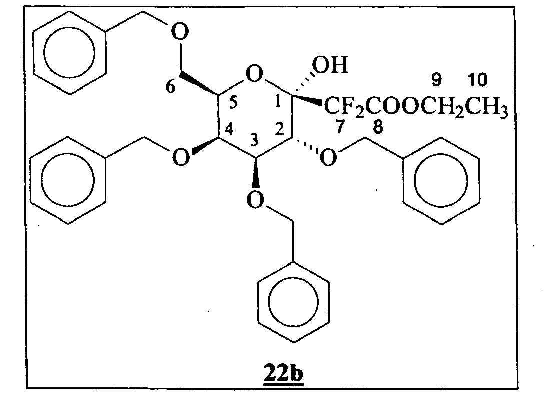

Rf= 0,35, éluant : cyclohexane / acétate d'éthyle (8 : 2).
**RMN ¹H (CDCl₃, 300MHz)**
1,1 (t, 7,2, 3H, CH₃); 3,4-3,5 (m, 2H, H6) ; 3,7-3,8 (dd, 2,5-9,5, 1H, H3); 3,8 (d, 2, 1H, H4) ; 4-4,1 (m, 3H, H5 ; CH₂) ; 4,25-4,85 (m, 9H, H2 ; 4OCH₂Bn) ; 7,2 (m, 20H, *H*ar).
**RMN ¹³C (CDCl₃, 75MHz)**
14,2 (CH₃) ; 63,6 (CH₂) ; 68,6 (C6) ; 71,7 (C5) ; 73,2 (OCH₂Bn) ; 73,9 (OCH₂Bn) ; 74,1 (C4) ; 74,9 (OCH₂Bn) ; 75,1 (C2) ;75,8 (OCH₂Bn) ; 81,2 (C3) ; 96,9 (t, 27Hz, C1) ; 113 (t , 264Hz, CF₂) ; 128,0-128,9 (Car.) ; 138,2;138,3 ; 138,6 ; 139,1 (Car. quat.) ; 163,3 (t, 31Hz, CO₂Et).
**RMN ¹⁹F (CDCl₃, 282MHz)**
-118,4 (d, J_{F-F}=256Hz) ; -120,2 (d, J_{F-F}=256Hz).

### Synthèse du gem-difluoroacide dérivé du glucose 23a (Fig. 11)

Dans un ballon sous atmosphère inerte contenant le *gem*-difluoroester dérivé du glucose **22a** (615 mg ; 0,93 mmol ; 1 éq.) en solution dans du THF (5 mL), une solution aqueuse de lithine (2M ; 2 éq.) est ajoutée et le mélange est agité pendant la nuit à température ambiante. Le milieu est concentré et dissous dans le dichlorométhane (5 mL), il est ensuite acidifié avec une solution d'acide chlorhydrique HCl 1M (50 mL). Le mélange est extrait avec du dichlorométhane (3 x 25 mL), et les phases organiques sont combinées, lavées avec une solution saturée de chlorure de sodium NaCl et concentrées directement.
L'acide **23a** est isolé avec un rendement pondéral de 90% sous la forme d'une huile incolore qui peut être utilisée directement pour la prochaine étape sans purification supplémentaire.

### Caractérisation de 23a

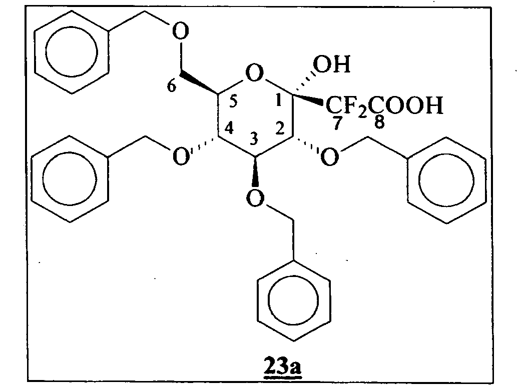

Rf = 0,50, éluant : dichlorométhane / méthanol (9 : 1).
**RMN ¹H (CDCl₃, 300MHz)**
3,36-3,45 (m ; 2H ; H3 ; H6) ; 3,58-3,63 (m ; 1H ; H6) ; 3,88 (m ; 2H ; H4 ; H5) ; 4,00 (m ; 1H ; H2) ; 4,38 (m ; 8H) ; 6,06 (1s ; 2H ; OH ; COOH) ; 7,03-7,28 (m ; 20H;H_{Ar}).
**RMN ¹³C (CDCl₃, 75MHz)**
68,0 (C6) ; 71,4 (C2) ; 73,0 ; 74,9 ; 75,1 ; 75,9 ; 77,2 (C3) ; 77,9 (C4) ; 82,1 (C5) ; 94,9 (t ; ²J_{C-F} 26,8 ; C1) ; 126,6 ; 126,7 ; 126,9 ; 127,0 ; 127,0 ; 127,3 ; 127,3 ; 127,4 ; 127,5 ; 135,6 ; 136,2 ; 136,4 ; 137,1,
**RMN ¹⁹F (CDCl₃, 282MHz)**
-117,2 (d, 1F, ²J_{F}-_{F} 258.6) ; -119,0 (d, 1F, ²J_{F-F} 258.6).

### Synthèse du gem-difluoroacide dérivé du galactose 23b (Fig. 11)

Dans un ballon sous atmosphère inerte contenant le *gem*-difluoroester dérivé du galactose **22b** (615 mg ; 0,93 mmol ; 1 éq.) en solution dans du THF (5 mL), une solution aqueuse de lithine LiOH (2M ; 2 éq.) est ajoutée et le mélange est agité pendant une nuit à température ambiante. Le milieu est concentré et dissous dans du dichlorométhane (5 mL), il est ensuite acidifié avec une solution d'acide chlorhydrique HCl 1M (50 mL). Le mélange est extrait avec du dichlorométhane (3 x 25 mL), et les phases organiques sont combinées, lavées avec une solution saturée de chlorure de sodium NaCl et concentrées directement.
L'acide **23b** est isolé avec un rendement quantitatif sous la forme d'une huile incolore qui peut être utilisée directement pour la prochaine étape sans purification supplémentaire.

### Caractérisation de 23b

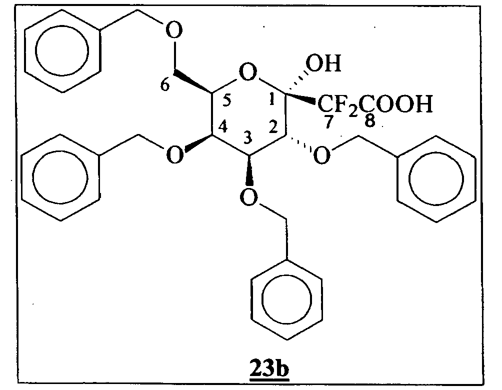

Rf = 0,50, éluant : dichlorométhane / méthanol (9 : 1).
**RMN ¹H (CDCl₃, 300MHz)**
3,2 (dd, 4,5Hz et 9,8Hz, 1H, H6) ; 3,5 (dd, 7,7Hz et 9,8Hz, 1H, H6) ; 3,7 (d, 2Hz, 1H, H4) ; 3,8 (dd, 2,6Hz et 9,5Hz, 1H, H3); 4 (dd, 4,5Hz et 7,7Hz ;1H, H5) ; 4,3-4,9 (m, 9H, H2 ; 40C**H**₂Bn) ; 7,2 (m, 20H, Har).
**RMN ¹³C (CDCl₃, 75MHz)**
69,4 (C6) ; 71,7 (C5 ; 73,5 (OCH₂Bn) ; 74,0 (OCH₂Bn) ; 74,1 (C4) ; 75,0 (OCH₂Bn) ; 75,1 (C2) ; 75,9 (OCH₂Bn) ; 80,8 (C3) ; 95,4 (t, 27Hz, C1) ; 112,5 (t , 260Hz, CF₂) ; 127,8 -129,0 (Car.) ; 137,6 ;138,0 ; 138,1 (Car. quat.) ; 163,1 (t, 30Hz, CO₂H).
**RMN ¹⁹F (CDCl₃, 282MHz)**
-117,3 (d, J_{F-F}=259Hz) ; -119,0 (d, J_{F-F}=259Hz)

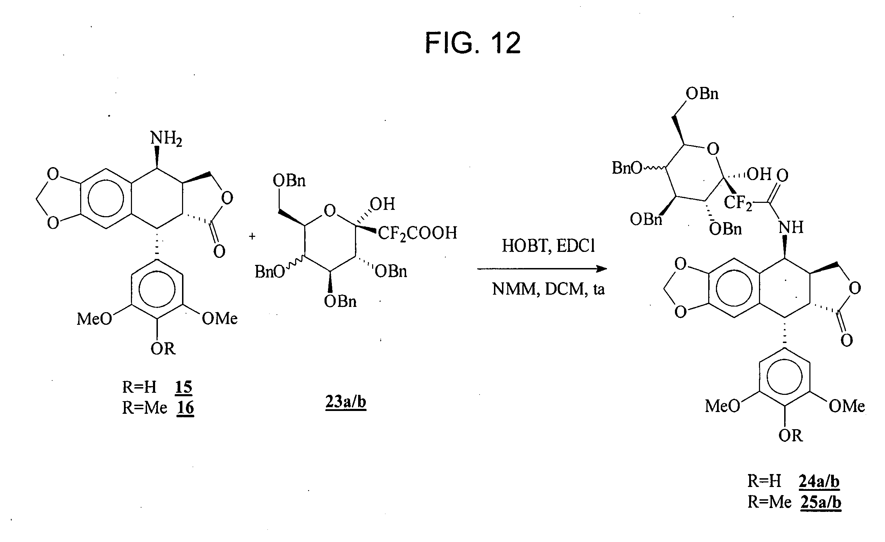

### Synthèse du composé 24a (Fig. 12)

Sur une suspension du difluoroacide dérivé du glucose **23a** (195 mg ; 0,307 mmol ; 1 éq.), de l'amine de la déméthylepipodophyllotoxine **15** (135 mg ; 0,338 mmol ; 1,1 éq.), de 1-hydroxybenzotriazol HOBT (45 mg ; 0,322 mmol ; 1,05 éq.), et de N-méthylmorpholine NMM (65 mg ; 0,629 mmol ; 2,05 éq.) dans du dichlorométhane (10 mL) sous atmosphère d'argon, du 1-(3-Diméthylamino-propyl)-3-éthylcarbodiimide hydrochloride EDCI (62 mg ; 0,322 mmol ; 1,05 éq.) est additionné. La réaction est agitée à température ambiante pendant trois jours. L'eau (10 mL) est ajoutée, et la phase aqueuse est extraite avec du dichlorométhane (3 x 15 mL). Les phases organiques sont lavées avec une solution saturée de chlorure de sodium NaCl (15 mL), séchées sur sulfate de magnésium MgSO₄ et concentrées sous vide pour donner un solide blanc. Le résidu est purifié par chromatographie sur gel de silice avec comme éluant un mélange dichlorométhane / acétate d'éthyle dans des proportions de huit pour deux.

Le produit désiré pur **24a** est obtenu sous forme de solide blanc avec un rendement pondéral de 62 %.

### Caractérisation de 24a

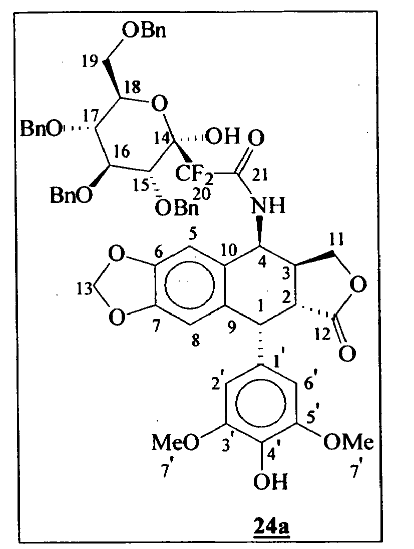

Rf = 0,68 ; éluant : dichlorométhane / acétate d'éthyle (80 : 20).
**RMN ¹H (CDCl₃, 300MHz)**
2,77-2,84 (m, 1H, H2) ; 2,85-2,94 (m, 1H, H3) ; 3,43-3,46 (dₐₚₚ, 1H, J 10.7, H19) ; 3,55 (t, 1H, ³J 9.4, H16) ; 3,61-3,62 (m, 1H, H19) ; 3,66 (s, 6H, H7' x 6) ; 3,81-3,98 (m, 4H, H11, H15, H17, H18) ; 4,24-4,51 (m, 4H, H11, H1) ; 4,66-4,84 (m, 6H) ; 5,16 (dd, 1H, ³J_{H4-H3} 7.5, ³J_{H4-NH} 4.2, H4) ; 5,49 (s, 1H, Ph-OH) ; 5,86 (dd, 2H, ²J_{H13-H13} 7.3, ³J 1.1, H13 x 2) ; 6,19 (s, 2H, H2', H6') ; 6,40 (s, 1H, H8) ; 6,68 (s, 1H, H5) ; 6,98 (d, 1H, ³J_{NH-H4} 4.2, -NH) ; 7,00-7 ;28 (m, 20H).
**RMN¹³C (CDCl₃, 75MHz)**
35,7 (C3) ; 40,3 (C2) ; 42,2 (C1) ; 47,4 (C4) ; 55,1 (2C ; C7' x 2) ; 66,4 (C19) ; 67,2 (C11) ; 70,7 (C15) ; 71,7 ; 73,7 ; 74,1 ; 74,7 ; 75,9 (C16) ; 76,6 (C17) ; 81,7 (C18) ; 94,9 (t ; ²J_{C-F} 27,5 ; C14) ; 100,4 (C 13) ; 106,4 (2C ; C2' ; C6') ; 107,7 (C5) ; 108,8 (C8); 126,0; 126,3; 126,4; 126,5; 126,6; 126,7; 127,1 ; 127,2; 128,7; 131,3; 131,5 ; 132,8 ; 135,9 ; 136,4 ; 136,4 ; 136,9 ; 145,2 (2C ; C3' ; C5') ; 146,4 (C7) ; 147,3 (C6) ; 162,0 (t ; ²J_{C-F} 26,8 ; C21) ; 172,7 (C 12),
**RMN ¹⁹F (CDCl₃, 282MHz)**
-116,3 (d, 1F, ²J_{F-F}258.6); -120,6(d, 1F ²J_{F-F}258.6).

### Synthèse du composé 25a (Fig. 12)

Sur une suspension du difluoroacide dérivé du glucose **23a** (130 mg ; 0,205 mmol ; 1 éq.), de l'amine de l'épipodophyllotoxine **16** (93 mg ; 0,225 mmol ; 1,1 éq.), d'HOBT (30 mg ; 0,215 mmol ; 1,05 éq.), de NMM (43 mg ; 0,420 mmol ; 2,05 éq.) dans le dichlorométhane (10 mL) sous atmosphère d'argon, de l'EDCI (42 mg ; 0,215 mmol ; 1,05 éq.) est additionné. La réaction est agitée à température ambiante pendant cinq jours. L'eau (15 mL) est ajoutée, et la phase aqueuse est extraite avec du dichlorométhane (3 x. 15 mL). Les phases organiques sont lavées avec une solution saturée de NaCl (20 mL), séchées sur sulfate de magnésium MgSO₄ et concentrées sous vide pour laisser un solide blanc.
Le résidu est purifié par chromatographie sur gel de silice avec comme éluant un mélange dichlorométhane / acétate d'éthyle dans des proportions de huit pour deux. Le produit désiré pur **25a** est obtenu sous forme de solide blanc avec un rendement pondéral de 50 %.

### Caractérisation de 25a

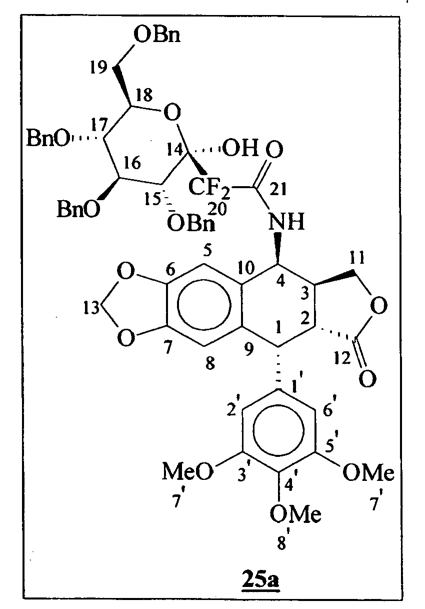

Rf = 0,88 ; éluant : DCM / AcOEt (80 : 20).
**RMN ¹H (CDCl₃, 300MHz)**
2,84-2,90 (m ; 1H ; H2) ; 2,94-3,00 (m ; 1H ; H3) ; 3,51-3,72 (m ; 3H ; H19 x 2 ; H16) ; 3,75 (s ; 6H ; H7' x 6) ; 3,80 (s ; 3H ; H8' x 3) ; 3,95-4,04 (m ; 4H ; H11 ; H15 ; H18 ; H17) ; 4,35-4,53 (m ; 4H ; H11 ; H1) ; 4,77-4,88 (m ; 6H) ; 5,23 (dd, 1H, ³J_{H4-NH} 7.3, ³J_{H4-H3} 4.5, H4) ; 5,96 (dd, 2H, ²J_{H13-H13} 10.3, ³J 1.3, H13 x 2) ; 6,26 (s, 2H, H2', H6') ; 6,48 (s, 1H, H8) ; 6,76 (s, 1H, H5) ; 6,98 (d, 1H, ³J_{NH-H4} 7.2, -NH) ; 7,01-7,26 (m, 20H).
RMN ¹³C (CDCl₃, 75MHz)
37,5 (C3) ; 42,0 (C2) ; 44,1 (C1) ; 48,9 (C4) ; 56,6 (2C ; C7' x 2) ; 61,2 (C8') ; 68,2 (C19) ; 69,0 (C11) ; 72,5 (C15) ; 73,4 ; 75,4 ; 75,9 ; 76,4 ; 77,6 (C16) ; 78,3 (C17) ; 83,5 (C18) ; 96,6 (t ; ²J_{C-F} 27,4 ; C14) ; 102,1 (C13) ; 108,5 (2C ; C2' ; C6') ; 109,5 (C5); 110,5 (C8); 127,7; 128,1 ; 128,1 ; 128,2; 128,2; 128,3; 128,4; 128,7; 128,8; 128,8; 128,9; 132,9; 135,0; 137,6; 137,7; 138,2 ; 138,6; 148,1 (C7); 149,1 (C6) ; 153,0 (2C ; C3' ; C5') ; 163,7 (t ; ²J_{C-F} 28,0 ; C21) ; 172,4 (C12),
**RMN ¹⁹F (CDCl₃, 282MHz)**
-116,6 (d, 1F, ²J_{F-F} 258.6); -120,3 (d, 1F, ²J_{F-F} 258.6).
**Spectrométrie de masse** : ESI+ : 1068 (M+K)+, 1052 (M+Na)+, 1030 (M+H)+.

### Synthèse de 24b (Fig. 12)

Sur une suspension du difluoroacide dérivé du galactose **23b** (185 mg ; 0,291 mmol ; 1 éq.), de l'amine dérivé de la déméthylépipodophyllotoxine **15** (130 mg; 0,321 mmol ; 1,1 éq.), d'HOBT (42 mg ; 0,316 mmol ; 1,05 éq.), d'NMM (61 mg ; 0,597 mmol ; 2,05 éq.) dans le DCM (8 mL) sous atmosphère d'argon, de l'EDCI (60 mg ; 0,306 mmol ; 1,05 éq.) est additionné. La réaction est agitée à température ambiante pendant quatre jours. L'eau (10 mL) est ajoutée, et la phase aqueuse est extraite avec du dichlorométhane (3 x 15 mL). Les phases organiques sont lavées avec une solution saturée de NaCl (25 mL), séchées sur sulfate de magnésium MgSO₄ et concentrées sous vide pour laisser un solide blanc.
Le résidu est purifié par chromatographie sur gel de silice avec comme éluant un mélange dichlorométhane / acétate d'éthyle dans des proportions de neuf pour un. Le produit désiré pur **24b** est obtenu sous forme de solide blanc avec un rendement pondéral de 55 %.

### Caractérisation de 24b

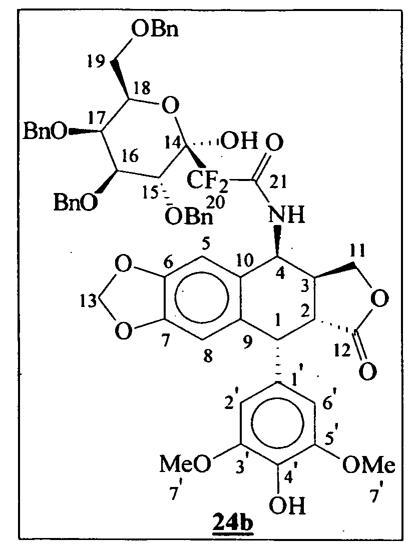

Rf = 0,65 ; éluant : dichlorométhane / AcOEt (80 : 20).
**RMN ¹H (CDCl₃, 300MHz)**
2,78-2,91 (m ; 2H ; H2 ; H3) ; 3,39-3,41 (m ; 2H ; H19 x 2) ; 3,67 (s ; 6H ; H7' x 6) ; 3,70-3,86 (m; 4H ; H11 ; H16 ; H17 ; H18) ; 4,02-4,08 (m ; 1H ; H15) ; 4,26-4,88 (m ; 10H ; H11 ; H1) ; 5,06 (dd, 1H, ³J_{H4-H3} 4.2, ³J_{H4-NH} 7.1, H4) ; 5,86 (d, 2H, ²J_{H13-H13} 7.2, H13 x 2) ; 6,17 (s, 2H, H2', H6') ; 6,38 (s, 1H, H8) ; 6,63 (s, 1H, H5) ; 6,79 (d, 1H, ³J_{NH-H4} 7.1, -NH) ; 7,00-7,28 (m, 20H).
**RMN ¹³C (CDCl₃, 75MHz)**
37,1 (C3) ; 41,9 (C2) ; 43,6 (C1) ; 48,6 (C4) ; 56,5 (2C ; C7' x 2) ; 68,2 (C19) ; 68,8 (C11) ; 71,4 (C15) ; 73,0 ; 73,3 ; 73,4 (C16) ; 74,4 (C17) ; 74,6 ; 75,6 ; 80,7 (C18) ; 96,5 (t ; ²J_{C-F} 26,3 ; C14) ; 101,7 (C13) ; 107,9 (2C ; C2' ; C6') ; 109,4 (C5) ; 110,2 (C8); 127,6; 127,6; 127,8; 127,9; 128,0; 128,0; 128,3 ; 128,4; 128,4; 128,5; 128,5; 128,6; 128,7; 130,2; 132,8; 134,2; 137,7; 137,8; 138,1 ; 138,4; 146,6 (2C ; C3' ; C5') ; 147,6 (C7) ; 148,6 (C6) ; 163,4 (t ; ²J_{C-F} 28,0 ; C21) ; 174,2 (C12),
**RMN ¹⁹F (CDCl₃, 282MHz)**
-118,3 (d, 1F, ²J_{F-F}257.5); -119,6 (d, 1F, ²J_{F-F}257.5).

### Synthèse de 25b (Fig. 12)

Sur une suspension du difluoroacide dérivé du galactose **23b** (105 mg ; 0,165 mmol ; 1 éq.), de l'amine dérivée de l'épipodophyllotoxine **16** (75 mg ; 0,181 mmol ; 1,1 éq.), d'HOBT (24 mg ; 0,173 mmol ; 1,05 éq.), d'NMM (35 mg ; 0,338 mmol ; 2,05 éq.) dans le dichlorométhane (10 mL) sous atmosphère d'argon, de l'EDCI (34 mg ; 0,173 mmol ; 1,05 éq.) est additionné. La réaction est agitée à température ambiante pendant cinq jours. L'eau (15 mL) est ajoutée, et la phase aqueuse est extraite avec du dichlorométhane (3 x 20 mL). Les phases organiques sont lavées avec une solution saturée de NaCl (25 mL), séchées sur MgSO₄ et concentrées sous vide pour laisser un solide blanc. Le résidu est purifié par chromatographie sur gel de silice avec comme éluant un mélange dichlorométhane / acétate d'éthyle dans des proportions de huit pour deux.
Le produit désiré pur **25b** est obtenu sous forme de solide blanc avec un rendement pondéral de 27 %.

### Caractérisation de 25b

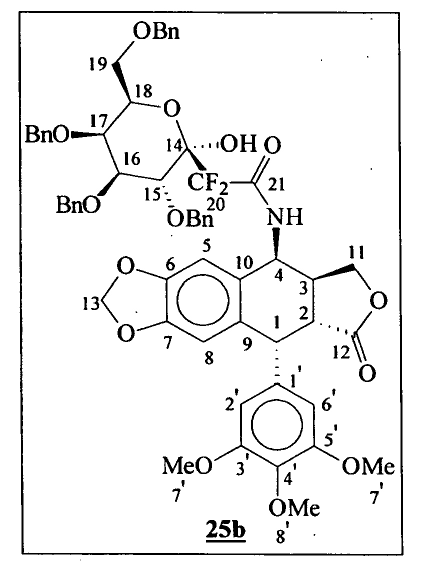

Rf = 0,90 ; éluant : DCM / AcOEt (80 : 20).
**RMN ¹H (CDCl₃, 300MHz)**
2,79-2,92 (m, 2H, H2, H3) ; 3,39 (dd, ³J_{H19-H19} 7.7, ³J_{M19-H18} 1.3, 2H, H19 x 2) ; 3,67 (s, 6H, H7' x 6) ; 3,71 (s, 3H, H8' x 3) ; 3,68-3,87 (m, 3H, H11, H16, H18) ; 4,02-4,10 (m, 1H, H15) ; 4,27-4,89 (m, 11H, H1, H11, H17) ; 5,06 (dd, 1H, ³J_{H4-H3} 4.0, ³J_{H4-NH} 7.3, H4) ; 5,87 (dd, 2H, ³J_{H13-H13} 6.9, J 1.1, H13 x 2) ; 6,18 (s, 2H, H2', H6') ; 6,40 (s, 1H, H8) ; 6,64 (s, 1H, H5) ; 6,74 (d, 1H, ³J_{NH-H4} 7.3, -NH) ; 7,15-7,25 (m, 20H).
**RMN ¹³C (CDCl₃, 75MHz)**
36,9 (C3) ; 41,4 (C2) ; 43,4 (C1) ; 48,2 (C4) ; 55,9 (2C ; C7' x 2) ; 60,5 (C8') ; 67,9 (C19) ; 68,4 (C11) ; 71,1 (C15) ; 72,7 ; 73,0 ; 73,0 (C16) ; 74,0 (C17) ; 74,2 ; 75,2 ; 80,4 (C18) ; 96,1 (t ; ²J_{C-F} 26,8 ; C14) ; 101,3 (C13) ; 107,9 (2C ; C2' ; C6') ; 109,0 (C5); 109,8 (C8); 127,2; 127,2; 127,4; 127,5; 127,6; 127,7; 128,0; 128,1 ; 128,1 ; 128,2 ; 128,2 ; 128,3 ; 132,3 ; 134,3 ; 137,0 ; 137,3 ; 137,4 ; 137,7 ; 138,0 ; 147,3 (C7) ; 148,3 (C6) ; 152,3 (2C ; C3' ; C5') ; 163,0 (t ; ²J_{C-F} 28,0 ; C21) ; 178,8 (C12),
**RMN ¹⁹F (CDCl₃, 282MHz)**
-118,4 (d, 1F, ²J_{F-F} 258.6); - -119,5 (d, 1F, ²J_{F-F} 258.6).

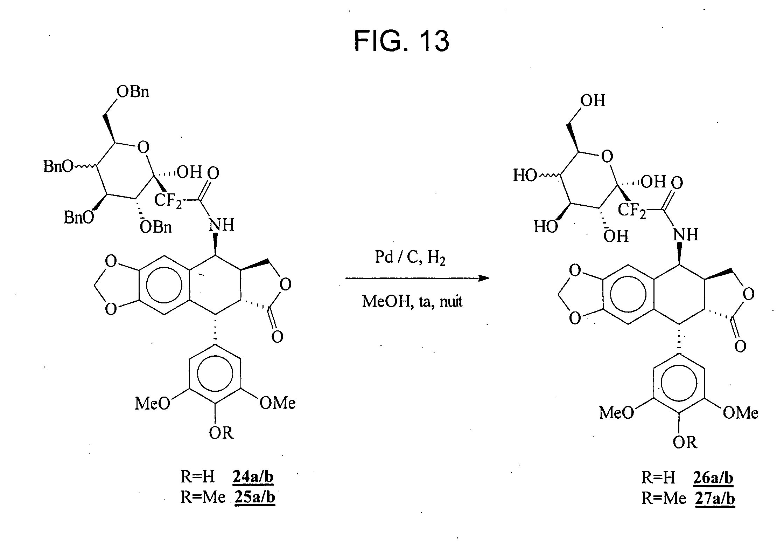

### Synthèse du composé 26a (Fig. 13)

Dans un ballon, le composé **24a** (140 mg ; 0,138 mmol) est dissous dans du méthanol (7 mL) avec du palladium sur charbon et placé sous une atmosphère d'hydrogène. Le mélange est agité pendant une nuit à température ambiante. Le mélange réactionnel est filtré, concentré et purifié par colonne chromatographique sur gel de silice avec comme éluant un mélange dichlorométhane / méthanol dans des proportions de neuf pour un.
Le produit **26a** est isolé sous la forme d'un solide blanc avec un rendement pondéral de 78 %.

### Caractérisation de 26a

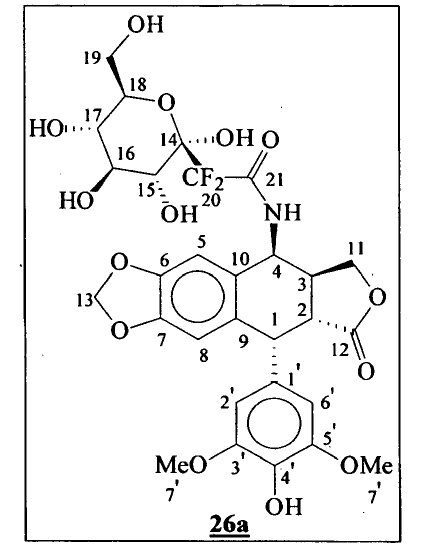

Rf = 0,35 ; éluant : DCM / méthanol (80 : 20).
**RMN ¹H (MeOD, 300MHz)**
2,96-3,11 (m, 1H, H3) ; 3,18-3,33 (m, 2H, H2) ; 3,57-3,71 (m, 5H, H18) ; 3,70 (s, 6H, H7' x 6) ; 3,97 (dd, 1H, ²J_{H11-H11} 9.0, ³J_{H11-H13} 10.9, H11) ; 4,36 (tₐₚₚ, 1H, ²J_{H11-H11} 9.0, H11) ; 4,58 (d, 1H, ³J_{H1-H2} 5.1, H1) ; 5,29 (d, 1H, ³J_{H4-H3} 4.5, H4) ; 5,95 (d; 2H, ³J_{H13-H13} 1.6, H13 x 2) ; 6,32 (s, 2H, H2', H6') ; 6,50 (s, 1H, H8) ; 6,77 (s, 1H, H5).
**RMN ¹³C (MeOD, 75MHz)**
30,7 (C3) ; 38,6 (C2) ; 42,8 (C1) ; 44,9 (C4) ; 56,5 (2C ; C7' x 2) ; 61,7 (C19) ; 70,2 (C11) ; 70,6 ; 71,9 ; 74,7 ; 75,7 ; 97,7 (t ; ²J_{C-F} 25,7 ; C14) ; 102,9 (C13) ; 109,3 (2C ; C2' ; C6') ; 110,2 (C5) ; 110,9 (C8) ; 129,7 ; 131,7; 134,2 ; 135,8 ; 148,6 (2C ; C3' ; C5') ; 148,8 (C7) ; 149,7 (C6) ; 165,6 (t ; ²J_{C-F} 29,1 ; C21) ; 177,1 (C12).
**RMN ¹⁹F (MeOD, 282MHz)**
-119,4 (d, 1F, J_{F-F} 256.4); -120.7 (d, 1F, ²J_{F-F} 256.4).
**Spectrométrie de masse :** ESI+ :678 (M+Na)+.

### Synthèse du composé 27a (Fig. 13)

Dans un ballon, le composé **25a** (100 mg ; 0,097 mmol) est dissous dans du méthanol (5 mL) avec du palladium sur charbon et placé sous une atmosphère d'hydrogène. Le mélange est agité pendant une nuit à température ambiante. Le mélange réactionnel est filtré, concentré pour ainsi donner le produit **27a** désiré sous la forme d'un solide blanc avec un rendement pondéral de 90 %.

### Caractérisation de 27a

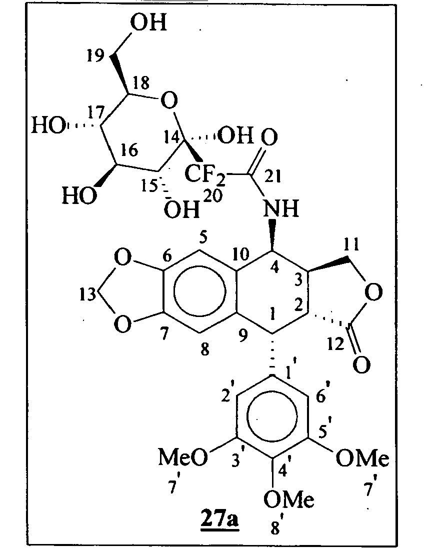

Rf = 0,89 ; éluant : DCM / méthanol (80 : 20).
**RMN ¹H (MeOD, 300MHz)**
2,87-3,07 (m, 1H, H3) ; 3,13-3,28 (m, 2H, H2) ; 3,49-3,60 (m, 5H) 3,60 (s, 9H, H7' x 6, H8' x 3) ; 3,90 (dd, 1H, ²J_{H11-H11} 9.0, ³J_{H11-H3} 10.7, H11) ; 4,28 (tₐₚₚ, 1H, ²J_{H11-H11} 9.0, H11) ; 4,52 (d, 1H, ³J_{H1-H2} 5.2, H1) ; 5,22 (d, 1H, ³J_{H4-H3} 4.6, H4) ; 5,87 (d, 2H, ³J_{H13-H13} 1.0, H13 x 2) ; 6,28 (s, 2H, H2', H6') ; 6,42 (s, 1H, H8) ; 6,70 (s, 1H, H5).
**RMN ¹³C (MeOD, 75MHz)**
39,7 (C3) ; 43,7 (C2) ; 46,1 (C1) ; 49,9 (C4) ; 57,6 (2C ; C7' x 2) ; 62,1 (C8') ; 62,8 (C19) ; 71,3 (C11) ; 71,6; 72,9; 75,8; 76,8; 97,1 (t ; ²J_{C-F} 26,3 ; C14) ; 104,0 (C13) ; 110,5 (2C; C2' ; C6') ; 111,3 (C5); 111,9 (C8); 130,8; 134,9; 138,2; 139,2 ; 150,0 (C7) ; 150,9 (C6) ; 154,9 (2C ; C3' ; C5') ; 165,0 (t ; ²J_{C-F} 28,6 ; C21) ; 178,0 (C12).
**RMN ¹⁹F (MeOD, 282MHz)**
Anomère majo : -119,4 (d, 1F, ²J_{F-F}257.5) ; -120,6 (d, 1F, ²J_{F-F}256.4).
Anomère mino : -119,9 (d, 1F, ²J_{F-F} 257.5) ; -121,1 (d, 1 F, ²J_{F-F} 256.4).
**Spectrométrie de masse :** ESI+
708 (M+K)+, 692 (M+Na)+, 670 (M+H)+.

### Synthèse de 26b (Fig. 13)

Dans un ballon, le composé **24b** (95 mg ; 0,094 mmol) est dissous dans du méthanol (8 mL) avec du palladium sur charbon et placé sous une atmosphère d'hydrogène. Le mélange est agité pendant une nuit à température ambiante. Le mélange réactionnel est filtré, concentré et purifié par colonne chromatographique sur gel de silice avec comme éluant un mélange dichlorométhane / méthanol dans des proportions de huit pour deux.
Le produit **26b** est isolé sous la forme d'un solide blanc avec un rendement pondéral de 89%.

### Caractérisation de 26b

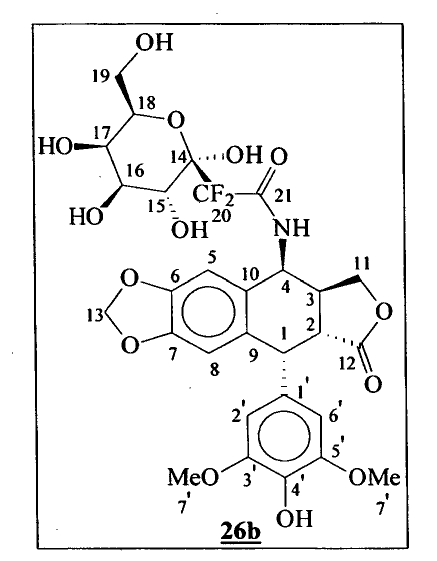

**RMN ¹H (MeOD, 300MHz)**
Anomère 1 : 2,93-3,09 (m ; 1H ; H3) ; 3,16-3,27 (m ; 2H ; H2) ; 3,51-3,61 (m ; 1H ; H19) ; 3,71 (s ; 6H ; H7' x 6) ; 3,70-3,78 (m ; 2H ; H19) ; 3,85-4,16 (m ; 3H ; H11) ; 4,32-4,36 (m ; 2H ; H11) ; 4,57 (d, 1H, ³J_{H1-H2} 5.1, H1) ; 5,32 (d, 1H, ³J_{H4-H3} 4.6, H4) ; 5,94 (s, 2H, H13 x 2) ; 6,33 (s, 2H, H2', H6') ; 6,49 (s, 1H, H8) ; 6,77 (s, 1H, H5).
Anomère 2 : 5,26 (d, 1H, ³J_{H4-H3} 4.5, H4); 6,85 (s, 1H, H5).
**RMN ¹³C (MeOD, 75MHz)**
39,0 (C3) ; 43,2 (C2) ; 45,3 (C1) ; 50,1 (C4) ; 56,9 (2C ; C7' x 2) ; 64,4 (C19) ; 70,6 (C11) ; 72,4 ; 76,2 ; 77,7 ; 82,2 ; 103,3 (C13) ; 109,7 (2C ; C2' ; C6') ; 110,5 (C5) ; 111,3 (C8) ; 130,2 ; 132,2 ; 134,7 ; 136,2 ; 149,0 (2C ; C3' ; C5') ; 149,2 (C7) ; 150,1 (C6) ; 177,6 (C12),
**RMN ¹⁹F (MeOD, 282MHz)**
Anomère 1 : -119,1 (d, 1F, ²J_{F-F} 257.6); -120,3 (d, 1F, ²J_{F-F} 257.6).
Anomère 2 : -121,4 (d, 1F, ²J_{F-F} 256.4); -123,1 (d, 1F, ²J_{F-F} 256.4).
**Spectrométrie de masse :** ESI+ : 694 (M+K)+, 678 (M+Na)+, 656 (M+H)+.

### Synthèse du composé 27b (Fig. 13)

Dans un ballon, le composé **25b** (42 mg ; 0,041 mmol) est dissous dans du méthanol (5 mL) avec du palladium sur charbon et placé sous une atmosphère d'hydrogène. Le mélange est agité pendant une nuit à température ambiante. Le mélange réactionnel est filtré, concentré pour ainsi donner le produit **27b** désiré sous la forme d'un solide blanc avec un rendement pondéral de 96 %.

### Caractérisation de 27b

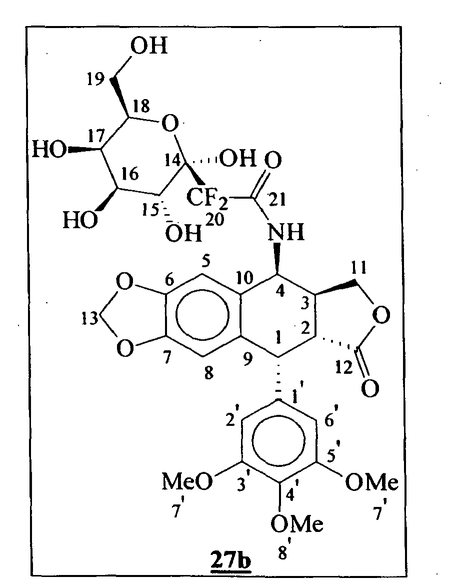

Rf = 0,70 ; éluant : dichlorométhane / méthanol (80 : 20).
**RMN ¹H (MeOD, 300MHz)**
Anomère majoritaire : 2,92-3,09 (m, 1H, H3) ; 3,17-3,23 (m, 1H, H2) ; 3,51 (dd, 1H, ²J_{H19-H19} 7.1, ³J_{H19-H18} 2.7, H19) ; 3,61 (dd, 1H, ²J_{H19-H19} 7.1, ³J_{H19-H18} 3.6, H19) ; 3,68 (s, 9H, H7' x 6, H8' x 3) ; 3,65-3,76 (m, 1H) ; 3,85-4,13 (m, 3H, H11) ; 4,31 (dₐₚₚ, 2H, ²J_{H11-H11} 7.7, H11) ; 4,58 (d, 1H, ³J_{H1-H2} 5.1, H1) ; 5,31 (d, 1H, ³J_{H4-H3} 4.5, H4) ; 5,92 (s, 2H, H13 x 2) ; 6,34 (s, 2H, H2', H6') ; 6,46 (s, 1H, H8) ; 6,75 (s, 1H, H5) ; 8,82 (d, 1H, ³J_{NH-H4} 8.0, NH),.
Anomère minoritaire : 5,25 (d, 1H, ³J_{H4-H3} 4.3, H4) ; 6,84 (s, 1H, H5),
**RMN ¹³C (MeOD, 75MHz)**
Anomère majoritaire : 39,1 (C3) ; 43,0 (C2) ; 45,5 (C1) ; 50,0 (C4) ; 56,9 (2C ; C7' x 2) ; 62,3 (C8') ; 64,4 (C19) ; 68,9 (C11) ; 72,4 ; 76,2 ; 77,7 ; 82,2 ; .103,4 (C13) ; 109,8 (2C ; C2' ; C6') ; 110,6 (C5) ; 111,2 (C8) ; 130,2 ; 134,3 ; 137,6 ; 138,5 ; 149,3 (C7) ; 150,2 (C6) ; 154,2 (2C ; C3' ; C5') ; 176,2 (C12).
Anomère minoritaire : 62,3 (C19) ; 68,8 ; 70,5 ; 72,7 ; 74,0 ; 111,6 (C5).
**RMN ¹⁹F (MeOD, 282MHz)**
Anomère majoritaire : -121,4 (dd, 1F, ²J_{F-F}, 256.4, ²J_{F-F} 12.9) ; -123,0 (dd, 1F, ²J_{F-F} 256.4, ²J_{F-F} 12.6).
Anomère minoritaire : -119,1 (dd, 1F, ²J_{F-F} 257.5, ²J_{F-F} 12.6) ; -120,2 (dd, 1F, ²J_{F-F} 257.5, ²J_{F-F} 12.6).

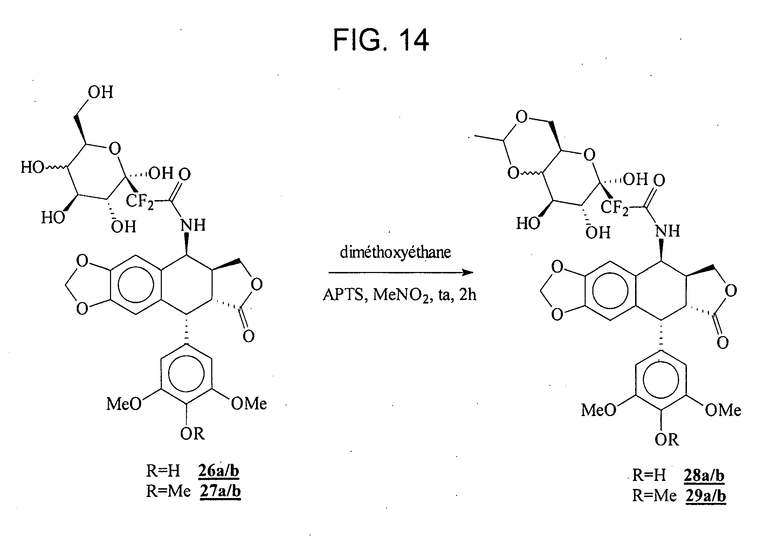

### Synthèse du composé 28a (Fig. 14)

Dans un ballon, le composé **26a** (65 mg ; 0,10 mmol, 1éq.) est dissous dans du nitrométhane MeNO₂ (3 mL) avec de l'acide paratoluène sulphonique APTS (5 mg ; 0,025 mmol ; 0,25 éq.) et du diméthoxyéthane (270 mg ; 3,0 mmol ; 30 éq.) à température ambiante sous atmosphère inerte. Le mélange réactionnel est agité pendant trois heures. De l'eau (10 mL) est ajoutée, et la phase aqueuse est extraite avec du chloroforme CHCl₃ (2 x 20 mL). Les phases organiques sont rassemblées et lavées avec une solution saturée de chlorure de sodium NaCl (20 mL), séchées sur Na₂SO₄ et concentrées sous vide pour donner une huile jaune. Le brut réactionnel est purifié par colonne chromatographique sur gel de silice avec comme éluant un mélange dichlorométhane / méthanol dans des proportions de neuf pour un. Le produit **28a** est isolé sous la forme d'un solide blanc avec un rendement pondéral de 95%.

### Caractérisation de 28a

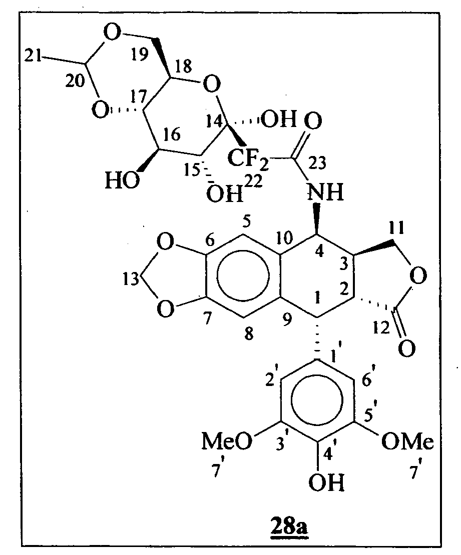

Rf = 0,50 ; éluant : dichlorométhane / méthanol (90 : 10).
**RMN ¹H (MeOD, 300MHz)**
1,27 (d, 3H, ³J_{H21-H20} 5.0, H21 x 3) ; 2,98-3,09 (m, 1H, H3) ; 3,11-3,32 (m, 2H, H2 + H18) ; 3,45-3,48 (m, 1H, H19) ; 3,71 (s, 6H, H7' x 6) ; 3,72-3,87 (m, 5H, H11, H15, H16, H17, H19) ; 4,33 (tₐₚₚ, 1H, ²J_{H11-H11} 7.7, H11) ; 4,57 (d, 1H, ³J_{H1-H2} 4.9, H1) ; 4,74 (q, 1H, ³J_{H20-H21} 5.0, H20) ; 5,31 (d, 1H, ³J_{H4-H3} 4.5, H4) ; 5,94 (d, 2H, ³J_{H13-H13} 1.4, H13 x 2) ; 6,33 (s, 2H, H2', H6') ; 6,49 (s, 1H, H8) ; 6,75 (s, 1H, H5).
**RMN ¹³C (MeOD, 75MHz)**
21,0 (C21) ; 38,8 (C3) ; 43,2 (C2) ; 45,3 (C1) ; 49,8 (C4) ; 57,1 (2C ; C7' x 2) ; 65,3 ; 69,4 (C19) ; 70,4 (C11) ; 72,9 ; 73,1 ; 81,6 (C18) ; 98,6 (t ; ²J_{C-F} 26,3 ; C14) ; 101,7 (C20); 103,4 (C13) ; 109,8 (2C; C2' ; C6'); 110,6 (C5); 111,4 (C8); 130,1 ; 132,1 ; 134,7 ; 136,2 ; 149,0 (2C ; C3' ; C5') ; 149,1 (C7) ; 150,1 (C6) ; 165,5 (t ; ²J_{C-F} 28,0 ; C23) ; 177,4 (C12).
**RMN ¹⁹F (MeOD, 282MHz)**
-119,7 (d, 1F, ²J_{F-F}256.4); -122,3 (d, 1F, ²J_{F-F}257.5).
**Spectrométrie de masse :** ESI- : 680 (M-H).

### Synthèse du composé 29a (Fig. 14)

Dans un ballon, le composé **27a** (45 mg; 0,07 mmol, 1éq.) est dissous dans du nitrométhane (3 mL) avec de l'APTS (3 mg; 0,016 mmol ; 0,25 éq.) et du diméthoxyéthane (170 mg; 1,9 mmol ; 30 éq.) à température ambiante sous atmosphère inerte. Le mélange réactionnel est agité pendant trois heures. De l'eau (10 mL) est ajoutée, et la phase aqueuse est extraite avec du chloroforme CHCl₃ (2 x 20 mL). Les phases organiques sont rassemblées et lavées avec une solution saturée de NaCl (20 mL), séchées sur Na₂SO₄ et concentrées sous vide pour donner une huile jaune. Le brut réactionnel est purifié par colonne chromatographique sur gel de silice avec comme éluant un mélange dichlorométhane / méthanol dans des proportions de neuf pour un. Le produit **29a** est isolé sous la forme d'un solide blanc avec un rendement pondéral de 87%.

### Caractérisation de 29a

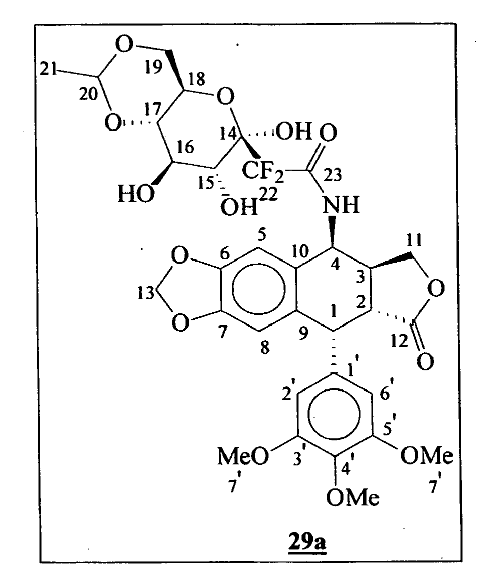

Rf = 0,45 ; éluant : dichlorométhane / méthanol (90 : 10).
**RMN ¹H (MeOD, 300MHz)**
1,28 (d, 3H, ³J_{H21-H20} 5.0, H21 x 3) ; 2,97-3,08 (m, 1H, H3) ; 3,15-3,31 (m, 2H, H2, H18) ; 3,44 (t, 1H, ³J_{H19-H18} 10.1, H19) ; 3,71 (s, 9H, H7' x 6, H8' x 3) ; 3,70-3,96 (m, 4H, H11, H15, H16, H17, H19) ; 4,34 (tₐₚₚ, 1H, ²J_{H11-H11} 8.8, H11) ; 4,61 (d, 1H, ³J_{H1-H2} 5.2, H1) ; 4,73 (q, 1H, ³J_{H20-H21} 5.0, H20) ; 5,30 (d, 1H, ³J_{H4-H3} 4.6, H4) ; 5,94 (dd, 2H, ³J_{H13-H13} 3.2, J 1.0, H13 x 2) ; 6,35 (s, 2H, H2', H6') ; 6,50 (s, 1H, H8) ; 6,75 (s, 1H, H5).
**RMN ¹³C (MeOD, 75MHz)**
21,0 (C21) ; 38,9 (C3) ; 43,0 (C2) ; 45,5 (C1) ; 49,7 (C4) ; 59,9 (2C ; C7' x 2) ; 61,4 (C8') ; 65,2 (C19) ; 69,4 ; 70,4 ; 72,9 ; 73,1 ; 81,6 ; 98,6 (t ; ²J_{C-F} 30,0 ; C14); 101,1 (C20); 103,4 (C13) ; 109,8 (2C; C2' ; C6'); 110,6 (C5); 111,3 (C8) ; 130,2; 134,5 ; 137,5 ; 138,6 ; 149,3 (C7) ; 150,2 (C6) ; 154,2 (2C ; C3' ; C5') ; 165,5 (t ; ²J_{C-F} 33,7 ; C23) ; 177,2 (C12).
**RMN ¹⁹F (MeOD, 282MHz)**

-119,8 (d, 1F, ²J_{F-F} 257.5) ; -121,9 (d, 1F, ²J_{F-F} 257.5).
**Spectrométrie de masse :** ESI+ : 734 (M+K)+, 718 (M+Na)+, 696 (M+H)

### Synthèse du composé 28b (Fig. 14)

Dans un ballon, le composé **26b** (45 mg ; 0,068 mmol ; 1éq.) est dissous dans du nitrométhane (3 mL) avec de l'APTS (4 mg; 0.017 mmol; 0,25 éq.) et du diméthoxyéthane (190 mg; 2,1 mmol; 30 éq.) à température ambiante sous atmosphère inerte. Le mélange réactionnel est agité pendant trois heures. De l'eau (10 mL) est ajoutée, et la phase aqueuse est extraite avec du CHCl₃ (2 x 20 mL). Les phases organiques sont rassemblées et lavées avec une solution saturée de NaCl (20 mL), séchées sur Na₂SO₄ et concentrées sous vide pour laisser une huile jaune. Le brut réactionnel est purifié par colonne chromatographique sur gel de silice avec comme éluant un mélange dichlorométhane / méthanol dans des proportions de neuf pour un. Le produit **28b** est isolé sous la forme d'un solide blanc avec un rendement pondéral de 59%.

### Caractérisation de 28b

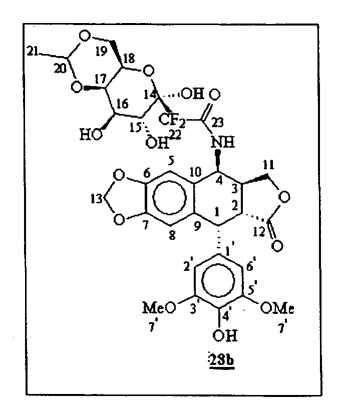

Rf = 0,40 ; éluant : DCM / méthanol (90 : 10).
**RMN ¹H (MeOD, 300MHz)**
Anomère 1 : 1,29 (d, 3H, ³J_{H21-H20} 4.9, H21 x 3) ; 2,94-3,09 (m, 1H, H3) ; 3,18-3,29 (m, 1H, H2) ; 3,71 (s, 6H, H7' x 6) ; 3,61-4,19 (m, 7H, H11, H15, H16, H17, H18, H19 x 2) ; 4,33 (tₐₚₚ, 1H, ²J_{H11-H11} 8.3, H11); 4,57 (t, 1H, ³J_{H1-H2} 5.6, H1) ; 4,76 (q, 1H, ³J_{H20-H21} 4.9, H20) ; 5,33 (d, 1H, ³J_{H4-H3} 4.5, H4) ; 5,94 (s, 2H, H13 x 2) ; 6,33 (s, 2H, H2', H6') ; 6,50 (s, 1H, H8) ; 6,80 (s, 1H, H5).
Anomère 2: 1,27 (d, 3H, ³J_{H21-H20} 4.8, H21 x 3) ; 1,33 (d, 3H, ³J_{H21-H20} 4.8, H21 x 3) ; 4,95 (q, 1H, ³J_{H20-H21} 4.8, H20) ; 5,07 (q, 1H, ³J_{H20-H21} 4.8, H20) ; 5,25 (d, 1H, ³J_{H4-H3} 4.5, H4) ; 6,34 (s, 2H, H2', H6') ; 6,98 (s, 1H, H5).
**RMN ¹³C (MeOD, 75MHz)**
Anomère 1 : 20,4 (C21) ; 39,1 (C3) ; 43,2 (C2) ; 45,3 (C1) ; 49,4 (C4) ; 57,1 (2C ; C7' x 2) ; 65,9 ; 67,0 (C11) ; 70,5 (C 19) ; 71,0 ; 77,4 ; 100,4 (C20) ; 103,3 (C 13) ; 109,7 (2C ; C2' ; C6') ; 110,5 (C5) ; 111,3 (C8) ; 130,2 ; 132,2 ; 134,7 ; 149,0 (2C ; C3' ; C5') ; 149,3 (C7) ; 150,1 (C6) ; 177,1 (C12).
Anomère 2 : 20,6 (C21) ; 21,5 (C21) ; 67,4 (C11) ; 70,7 (C19) ; 76,9 ; 77,1 ; 79,9 ; 82,4 ; 110,8 (C5).
**RMN ¹⁹F (MeOD, 282MHz)**
Anomère 1 : -118,5 (d, 1F, ²J_{F-F254.9}) ; -121,8 (d, 1F, ²J_{F-F}256.4).
Anomère 2 : -121,6 (d, 1F, ²J_{F-F} 255.4) ; -123,5 (d, 1F, ²J_{F-F} 255.4).

### Synthèse du composé 29b (Fig. 14)

Dans un ballon, le composé **27b** (25 mg ; 0,037 mmol ; 1éq.) est dissous dans du nitrométhane (3 mL) avec de l'APTS (2 mg ; 0,01 mmol ; 0,25 éq.) et du diméthoxyéthane (108 mg; 1,2 mmol; 30 éq.) à température ambiante sous atmosphère inerte. Le mélange réactionnel est agité pendant trois heures. De l'eau (10 mL) est ajoutée, et la phase aqueuse est extraite avec du CHCl₃ (2 x 20 mL). Les phases organiques sont rassemblées et lavées avec une solution saturée de NaCl (20 mL), séchées sur Na₂SO₄ et concentrées sous vide pour laisser une huile jaune. Le brut réactionnel est purifié par colonne chromatographique sur gel de silice avec comme éluant un mélange dichlorométhane / méthanol dans des proportions de neuf pour un. Le produit **29b** est isolé sous la forme d'un solide blanc avec un rendement pondéral de 58%.

### Caractérisation de 29b

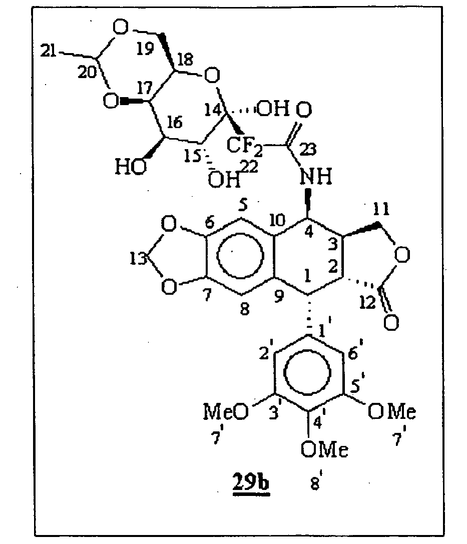

Rf = 0,58 ; éluant : dichlorométhane / méthanol (90 : 10).
**RMN ¹H (MeOD, 300MHz)**
Anomère 1 : 1,29 (d, 3H, ³J_{H21-H20} 5.0, H21 x 3) ; 3,00-3,06 (m, 1H, H3) ; 3,21-3,30 (m, 1H, H2) ; 3,71 (s, 9H, H7' x 6, H8' x 3) ; 3,59-4,20 (m, 7H, H11, H15, H16, H17, H18, H19 x 2) ; 4,29-4,38 (m, 1H, H11) ; 4,61 (tₐₚₚ, 1H, ³J_{H1-H2} 6.7, H1) ; 4,76 (q, 1H, ³J_{H20-H21} 5.0, H20) ; 5,26 (d, 1H, ³J_{H4-H3} 4.5, H4) ; 5,95 (s, 2H, H13 x 2) ; 6,36 (d, 2H, J 2.9, H2', H6') ; 6,49 (s, 1H, H8) ; 6,89 (s, 1H, H5).
Anomère 2 : 1,26 (d, 3H, ³J_{H21-H20} 4.7, H21 x 3) ; 4,96 (q, 1H, ³J_{H20-H21} 4.7, H20) ; 5,34 (d, 1H, ³J_{H4-H3} 4.4, H4) ; 6,78 (d, 1H, J 2.6, H5).
**RMN ¹³C (MeOD, 75MHz)**
Anomère 1 : 21,5 (C21) ; 39,2 (C3) ; 43,0 (C2) ; 45,5 (C1) ; 49,1 (C4) ; 56,9 (2C ; C7' x 2) ; 61,4 (C8') ; 65,9 ; 67,0 ; 68,5 ; 69,9 ; 71,0 ; 77,2 ; 100,4 (C20) ; 103,3 (C13); 109,9 (2C; C2' ; C6'); 110,6 (C5); 111,2 (C8); 130,1 ; 134,3 ; 137,6; 138,6 ; 149,4 (C7) ; 150,2 (C6) ; 154,2 (2C ; C3' ; C5') ; 177,2 (C12),
Anomère 2 : 20,4 (C21) ; 20,6 (C21) ; 70,7 ; 76,6 ; 76,9 ; 77,4 ; 103,9 (C13) ; 109,8 (2C ; C6' ; C2') ; 110,8 (C5),
**RMN ¹⁹F (MeOD, 282MHz)**
Anomère 1 : -119,6 (d, 1F, ²J_{F-F} 256.4) ; - -121,7 (d, 1F, ²J_{F-F}257.5).
Anomère 2 : -121,7 (d, 1F, ²J_{F-F}257.4) ; -124,0 (d, 1F, ²J_{F-F}256.4).
Spectrométrie de masse : ESI- : 694 (M-H).

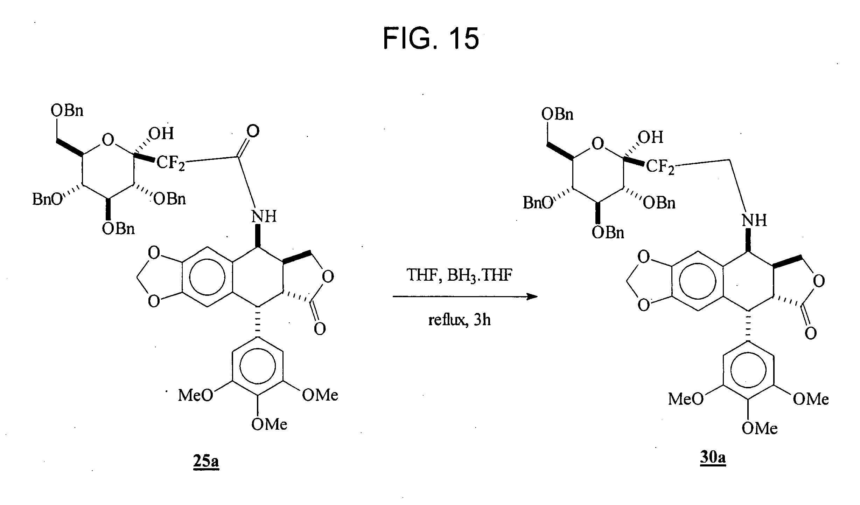

### Synthèse du composé 30a (Fig. 15)

Le produit **25a** (30 mg ; 0.051 mmol ; 1 éq.) est dissous dans 5 mL de THF. Le complexe BH₃.THF 1M (0.10 mL ; 0.102 mmol ; 2 éq.) est additionné sur le mélange à température ambiante, et la réaction est portée à reflux pendant trois heures. On laisse le milieu réactionnel revenir à température ambiante. Une solution d'HCl 1N (10 mL) est ajoutée, et la phase aqueuse est extraite avec du DCM- (3 x 10 mL). Les phases organiques sont lavées avec une solution saturée de NaCl (15 mL), séchées sur MgSO₄ et concentrées sous vide pour laisser un solide blanc. Le résidu est purifié par chromatographie sur gel de silice avec un mélange DCM / AcOEt (90 : 10) comme éluant pour donner le produit désiré pur **30a** sous forme de solide blanc avec un rendement pondéral de 46 %.

### Caractérisation de 30a

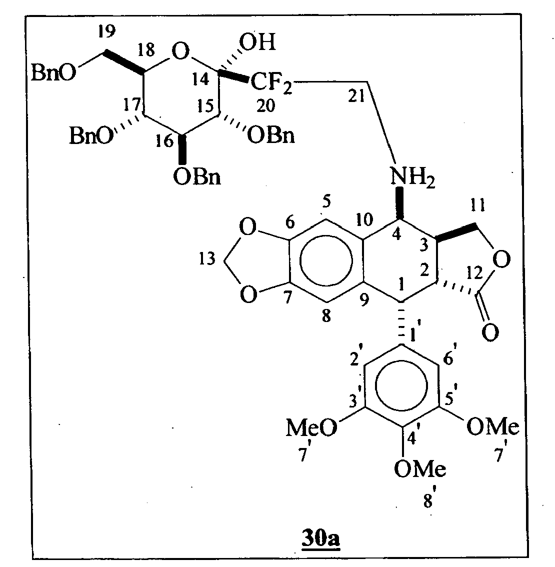

Rf = 0,66 ; éluant : DCM / AcOEt (90 : 10).
**RMN ¹H (CDCl₃, 300MHz)**
2,62-2,78 (m, 1H, H3) ; 3,07 (dd, 1H, ³J 5.2, 14.1, H2) ; 3,52-3,58 (m, 3H, H16, H19 x 2) ; 3,66 (s, 6H, H7' x 6) ; 3,72 (s, 3H, H8' x 3) ; 3,85-3,99 (m, 4H, H4, H15, H17, H18) ; 4,16-4,21 (m, 2H, H11 x 2) ; 4,38-4,53 (m, 4H, H1 ; 4,71-4,82 (m, 7H) ; 5,86 (d, 2H, ²J_{H13-H13} 14.7, H13 x 2) ; 6,17 (s, 2H, H2', H6') ; 6,38 (s, 1H, H8) ; 6,79 (s, 1H, H5) ; 7,12-7,31 (m, 20H).
**RMN ¹³C (CDCl₃, 75MHz)**
39,0 (C3) ; 41,4 (C2) ; 44,1 (C1) ; 56,6 (2C ; C7' x 2) ; 57,0 (C4) ; 61,1 (C8') ; 68,7 (C11) ; 68,9 (C19) ; 72,1 (C15) ; 73,5 ; 75,4 ; 75,7 ; 76,4 ; 78,0 (C16) ; 78,8 (C17) ; 83,8 (C18) ; 96,7 (t ; ²J_{C-F} 9,1 ; C14) ; 101,6 (C13) ; 108,5 (2C ; C2' ; C6') ; 108,9 (C5); 110,3 (C8) ; 127,7 ; 127,8; 127,9; 128,0; 128,1 ; 128,2; 128,5; 128,5; 128,6 ; 128,7 ; 131,7; 135,6; 137,3 ; 137,7 ; 138,0; 138,3 ; 138,5 ; 147,5 (C7); 148,1 (C6) ; 152,6 (2C ; C3' ; C5') ; 175,2 (C12).
**RMN ¹⁹F (CDCl₃, 282MHz)**
-115,5 (dtₐₚₚ, 1F, ²J_{F-F} 252.2, ³J_{F-H} 11.8) ; -117,2 (dtₐₚₚ, 1F, ²J_{F-F} 252.2, ³J_{F-H} 16.1).

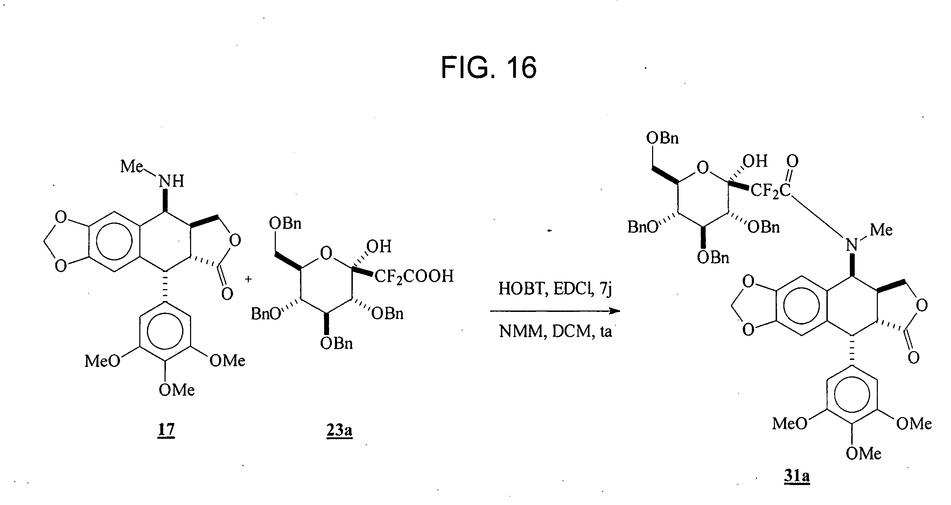

### Synthèse du composé 31a (Fig. 16)

Sur une suspension du difluoroacide dérivé du glucose **23a** (48 mg ; 0.075 mmol ; 1.00 éq.), de l'amine de l'épipodophyllotoxine **17** (31 mg ; 0.082 mmol ; 1.10 éq.), d'HOBT (11 mg ; 0.079 mmol ; 1.05 éq.), de NMM (16 mg ; 0.154 mmol ; 2.05 éq.) dans le dichlorométhane (7 mL) sous atmosphère d'argon, de l'EDCI (16 mg ; 0.079 mmol ; 1.05 éq.) est additionné. La réaction est agitée à température ambiante pendant 7 jours. L'eau (10 mL) est ajoutée, et la phase aqueuse est extraite avec du dichlorométhane (3x10 mL). Les phases organiques sont lavées avec une solution saturée de NaCl (10 mL), séchées sur sulfate de magnésium MgSO₄ et concentrées sous vide pour laisser un solide blanc.

Le résidu est purifié par chromatographie sur gel de silice avec comme éluant un mélange dichlorométhane / acétate d'éthyle dans des proportions de 9 pour 1.

Le produit désiré pur **31a** est obtenu sous forme d'une huile incolore avec un rendement pondéral de 29 %.

### Caractérisation de 31a

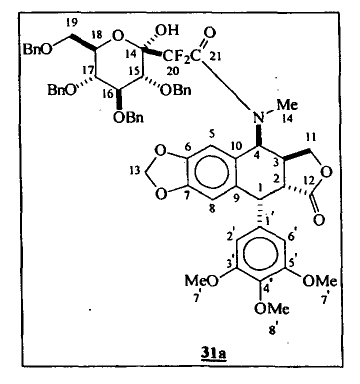

Rf = 0.48, éluant : DCM / AcOEt (80 : 20).
**RMN ¹H (CDCl₃, 300MHz)**
2.52 (s, 1H, H2), 2.65 (s, 3H, H14 x 3), 2.83 (dd, 1H, ³J_{H3-H11} 9.3, 5.0, H3), 3.53-3.80 (m, 3H, H19 x 2, H16), 3.75 (s, 6H, H7' x 6), 3.80 (s, 3H, H8' x 3), 3.88 (s, 1H, H4), 3.93-4.21 (m, 5H, H11 x 2, H15, H17, H18), 4.30 (s, 1H, H1), 4.32-4.88 (m, 8H), 5.93 (dd, 2H, ²J_{H13-H13} 6.3, J 1.2, H13 x 2), 6.29 (s, 2H, H2', H6'), 6.48 (s, 1H, H8), 6.61 (s, 1H, H5), 7.12-7.33 (m, 20H).
**RMN ¹³C (CDCl₃, 75MHz)**
27.6 (C14), 40.3 (C3), 46.5 (C1), 51.3 (C2), 56.7 (2C, C7' x 2), 61.2 (C8'), 62.4 (C4), 66.4 (C11), 68.5 (C 19), 73.0 (C 15), 73.7, 75.4, 75.6, 76.3, 77.7 (C16), 78.4 (C17), 83.1 (C18), 101.0 (C13), 105.8 (2C, C2', C6'), 107.1 (C5), 112.0 (C8), 127.3, 127.4, 127.6, 127.9, 128.1, 128.2, 128.2, 130.9, 136.8, 137.1, 137.6, 137.8, 137.9, 139.3, 145.6 (C7), 147.6 (C6), 153.1 (2C, C3', C5'), 174.3 (C12).
**RMN ¹⁹F (CDCl₃, 282MHz)**
-117.9 (d, 1F, ²J_{F-F} 256.4), -119.7 (d, 1F, ²J_{F-F} 256.4).

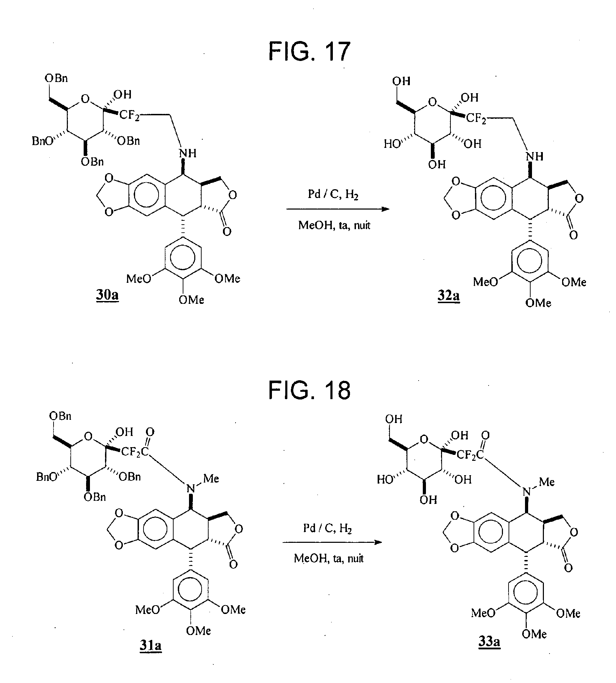

### Synthèse du composé 32a (Fig. 17)

Dans un ballon, le composé **30a** (11 mg ; 0.011 mmol) est dissout dans le méthanol (5 mL) avec le palladium sur charbon et placé sous une atmosphère d'hydrogène. Le mélange est agité pendant la nuit à température ambiante. Le mélange réactionnel est filtré, concentré puis purifié sur colonne de gel de silice avec un mélange DCM / MeOH avec un rapport 80 / 20 pour ainsi laisser le produit désiré **32a** en tant qu'une huile incolore.

### Caractérisation du composé 32a

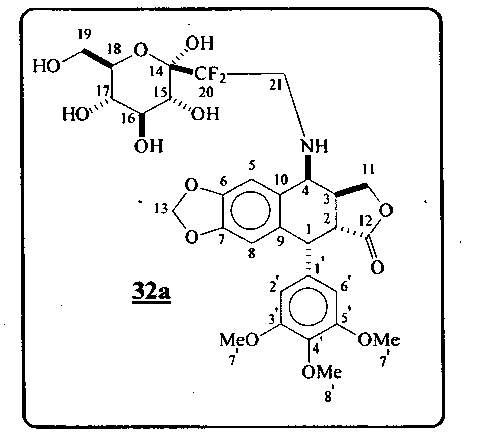

Rf = 0.90, éluant : DCM / MeOH (80 : 20).
**RMN ¹H (CDCl₃, 300MHz)**
2.72-2.99 (m, 1H, H3), 3.22-3.39 (m, 3H, H2, H19 x 2), 3.66 (s, 9H, H7' x 6, H8' x 3), 3.61-3.86 (m, 4H, H15, H16, H17, H18), 4.11 (d, 1H, ³J_{H4-H3} 4.1, H4), 4.33-4.41 (m, 3H, H21, H11 x 2), 4.53 (d, 1H, ³J_{H1-H2} 5.4, H1), 4.70-4.81-(m, 1H, H21), 5.92 (d, 2H, ²J_{H13-H13} 1.3, H13 x 2), 6.29 (s, 2H, H2', H6'), 6.42 (s, 1H, H8), 7.02 (s, 1H, H5).
**RMN ¹³C (CDCl₃, 75MHz)**
40.8 (C3), 42.6 (C2), 45.5 (C1), 56.9 (2C, C7' x 2), 58.2 (C4), 61.4 (C8'), 63.0 (C19), 70.7 (C11), 71.7, 72.6, 75.0, 76.3, 90.0 (C 14), 103.1 (C13), 109.8 (2C, C2', C6'), 110.7 (C5), 111.1 (C8), 133.1, 134.0, 136.9, 138.2, 149.1 (C7), 149.6 (C6), 154.1 (2C, C3', C5'), 178.2 (C12).
**RMN ¹⁹F (CDCl₃, 282MHz)**
-117.4 (dt, 1F, ²J_{F-F} 255.4, ³J_{F-H21} 17.1), -118.6 (ddd, 1F, ²J_{F-F} 255.4, ³J_{F-H21} 15.0, 10.7).
**Spectrométrie de masse** : ESI- : 654 (M)-.

### Synthèse du composé 33a (Fig. 18)

Dans un ballon, le composé **31a** (22 mg ; 0.21 mmol) est dissout dans le méthanol (5 mL) avec le palladium sur charbon et placé sous une atmosphère d'hydrogène. Le mélange est agité pendant la nuit à température ambiante. Le mélange réactionnel est filtré, concentré puis purifié par chromatographie sur colonne de silice avec un mélange DCM / MeOH (80 : 20) comme éluant. Le produit **33a** est isolé sous forme d'un solide blanc avec un rendement de 58%.

### Caractérisation du composé 33a

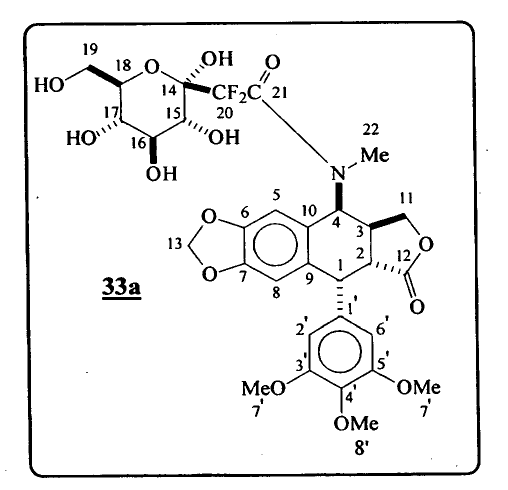

Rf = 0.83, éluant : DCM / méthanol (80 : 20).
**RMN¹H (MeOD, 300MHz)**
2.63 (1s, 1H, H2), 2.71 (s, 3H, H22 x 3), 2.95 (dd, 1H, ³J_{H3-H4} 9.2, ³J_{H3-H11} 5.8, H3), 3.20-3.32 (m, 1H), 3.52-3.73 (m, 5H), 3.74 (s, 9H, H7' x 6, H8' x 3), 4.14 (dd, 1H, ³J_{H11-H3} 5.8, ²J_{H11}-_{H11}11.2, H11), 4.26-4.38 (m, 3H, H1, H4, H11), 5.93 (dd, 2H, J 0.8, ²J_{H13-H13} 9.7, H13 x 2), 6.40 (s, 2H, H2', H6'), 6.46 (s, 1H, H8), 6.86 (s, 1H, H5).
**RMN ¹³C (MeOD, 75MHz)**
28.3 (C22), 41.6 (C3), 47.5 (C1), 52.7 (C2), 56.9 (2C, C7' x 2), 61.3 (C8'), 62.5 (C11), 63.7 (C4), 66.9 (C6), 71.1, 72.2, 75.2, 76.2, 103.0 (C13), 107.5 (2C, C2', C6'), 109.0 (C5), 113.0 (C8), 130.0, 133.2, 138.3, .141.7, 147.7 (C7), 149.7 (C6), 154.8 (2C, C3', C5').
**RMN ¹⁹F (MeOD, 282MHz)**
-120.4 (d, 1F, ²J_{F-F} 254.9); -123.5 (d, 1F, ²J_{F-F} 254.9).

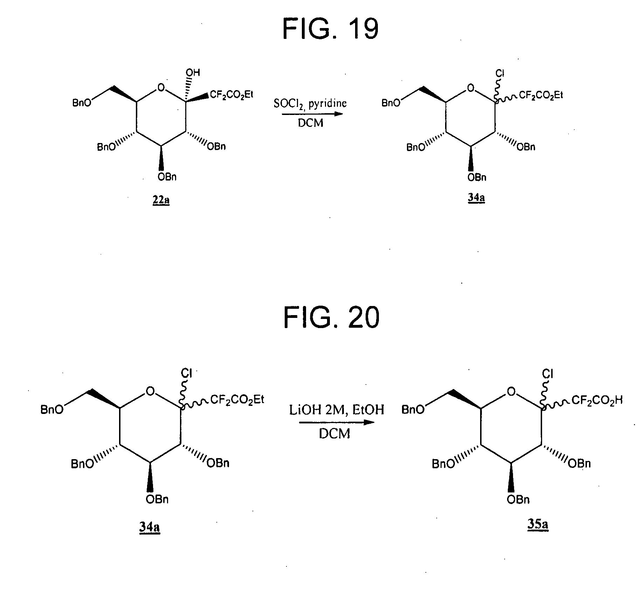

### Synthèse du composé 34a (Fig.19)

Dans un ballon sous atmosphère inerte contenant le difluoroester **22a** (215 mg ; 0,324 mmol ; 1 éq.) en solution dans le dichlorométhane anhydre (5 mL) à -30°C, on additionne goutte à goutte le chlorure de thionyl SOCl₂ (37 µL ; 0,49 mmol ; 1,5 éq.). Après 30 minutes d'agitation à-30°C, on introduit la pyridine (40 µL ; 0,49 mmol ; 1,5 éq.). et on laisse sous agitation 30 minutes de plus. Une solution d'HCl 2M est ajoutée et la phase est extraite trois fois avec du dichlorométhane. Les phases organiques sont rassemblées, lavées avec une solution saturée de NaCl, séchées sur MgSO₄, filtrées et concentrées sous pression réduite. Le brut réactionnel est purifié sur colonne de silice avec un mélange éluant cyclohexane/ acétate d'éthyle 9/1 pour isoler le produit chloré **34a** (mélange des 2 anomères) sous la forme d'une huile incolore avec un rendement de 51%.

### Caractérisation du composé 34a

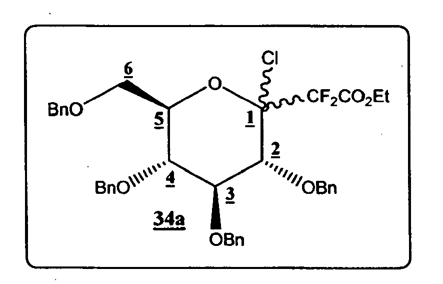

C₃₈H₃₉ClF₂O₇ M= 681,16 g.mol⁻¹
**RMN ¹⁹F (CDCl₃, 282MHz)**
-109,3 (d, 1F, ²J_{F-F}261Hz), -111,2 (d, 1F, ²J_{F-F}261) → 82% majoritaire
-111,5 (d, 1F, ²J_{F-F} 250Hz), -113,6 (d, 1F, ²J_{F-F} 250Hz) → 18% minoritaire
**RMN ¹H (CDCl₃, 300MHz)**
1,07 (t, 7,2Hz, 3H, C**H**₃); 3,74 (dd, 1,9 et 11,6Hz, 1H, H**6**) ; 3,80 (dd, 3,3 et 11,6Hz, 1H, H**6**) ; 3,91-3,97 (m, 3H, C**H**₂ et H**3**); 4,16 (m, 2H, H**5** et H2) ; 4,31 (dd, 7,3 et 10,9Hz, 1H, H**4**) ; 4,48-4,84 (m, 8H,; 4OC**H**₂Ph) ; 7,19-7,40 (m, 20H, **H**ar).
**RMN ¹³C (CDCl₃,75,5MHz)**
Majoritaire
13,8 (**C**H₃) ; 63,1 **C**H₂) ; 68,1 (C**6**) ; 73,1 (O**C**H₂Ph); 73,4 (O**C**H₂Ph) ; 73,7 (O**C**H₂Ph) ; 74,7 (O**C**H₂Ph) ; 76,0 (C**4**) ; 76,7 (C**5** ou C**2**) ; 82,0 (C**5** ou C**2**) ; 82,9 (C**3**) ; 99,8 (dd, 26 et 31Hz, C**1**); 112,1 (dd, 259 et 263Hz, **C**F₂); 127,6 ; 127,8 ; 128,0; 128,1 ; 128,2; 128,3 ; 128,4 ; 128,5 (2C); 128,6; 128,7 (**C**ar.) ; 136,7 ;137,8 ; 138,1 ; 138,4 (**C**ar. quat.) ; 161,5 (t, 33Hz, **C**O₂Et).
Minoritaire
14,0 (**C**H₃); 63,6 (**C**H₂) ; 67,6 (C**6**) ; 73,5 (O**C**H₂Ph) ; 75,0 (O**C**H₂Ph); 75,4 (O**C**H₂Ph) ; 75,7 (C**4**) ;76,2 (O**C**H₂Ph) ; 76,3 (C**5** ou C**2**) ; 79,2 (C**5** ou C**2**) ; 83,4 (C**3**).

### Synthèse du composé 35a (Fig. 20)

Dans un ballon sous atmosphère inerte contenant l'ester chloré **34a** (115 mg ; 0.169 mmol ; 1 éq.) en solution dans l'éthanol (4 mL), une solution aqueuse de lithine (2M ; 2 éq.) est ajoutée et le mélange est agité pendant la nuit à température ambiante. Le milieu est concentré et dissout dans le DCM (5 mL), il est ensuite acidifié avec une solution d'HCl 1M (20 mL). Le mélange est extrait avec du DCM (3 x 20 mL), et les phases organiques sont combinées, lavées avec une solution saturée de NaCl et concentrées directement. L'acide **35a** est isolé ainsi comme une huile blanche qui peut être utilisée directement pour la prochaine étape sans purifications supplémentaires avec un rendement brut de 84%.

### Caractérisation du composé 35a

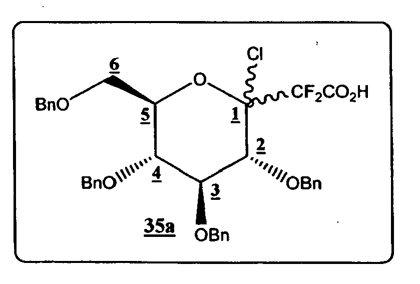

**RMN ¹H (CDCl₃, 300MHz)** 3.40-3.47 (m, 1H), 3.48 (dd, 1H, J 10.6, 6.6), 3.63 (dₐₚₚ, 1H, J 9.2, H6), 3.93-3.95 (m, 2H, H2 + H4), 4.03-4.10 (m, 1H, H5), 4.41-4.60 (m, 3H), 4.73-4.84 (m, 5H), 7.04-7.29 (m, 20H, H_{Ar}).
**RMN ¹³C (CDCl₃, 75MHz)**
68.4 (C6), 71.9 (C5), 73.1, 75.2, 75.6, 76.2, 77.5, 78.2 (C4), 83.3 (C3), 96.1 (t, ²J_{C-F} 26.8, C1), 127.9, 127.9, 128.1, 128.2, 128.3, 128.6, 128.6, 128.7, 136.7, 137.4, 137.6, 138.3, 163.2 (t, ³J_{C-F} 32.0, C8).
**RMN ¹⁹F (CDCl₃, 282MHz)**
-1117.2 (d, ²J_{F-F} 258.6), -118.9 (d, ²J_{F-F} 258.6).

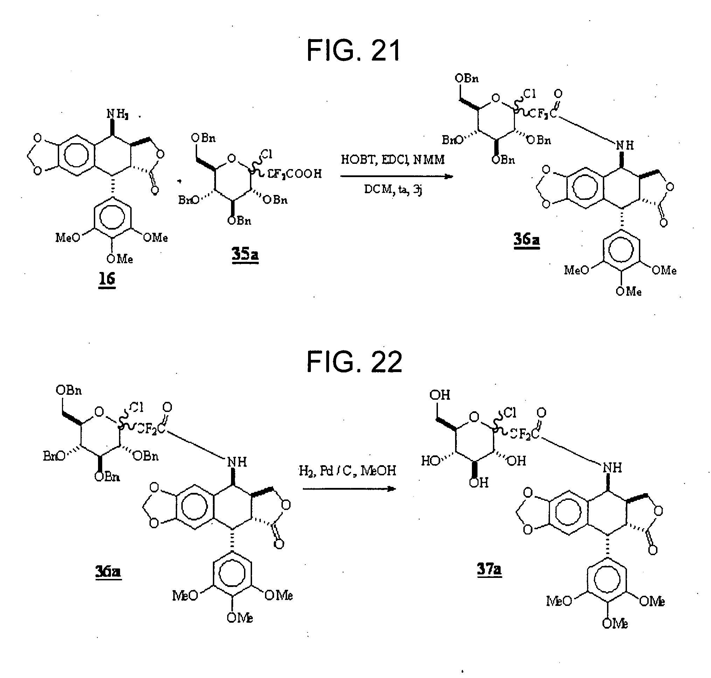

### Synthèse du composé 36a (Fig. 21)

Sur une suspension de l'acide **35a** (90 mg ; 0.138 mmol ; 1 éq.), de l'amine **16** (63 mg ; 0.152 mmol ; 1.1 éq.), d'HOBT (20 mg ; 0.145 mmol ; 1.05 éq.), et de NMM (29 mg ; 0.283 mmol ; 2.05 éq.) dans le DCM (8 mL) sous atmosphère d'argon, l'EDCI (28 mg ; 0.145 mmol ; 1.05 éq.) est additionné. La réaction est agitée à température ambiante pendant 3 jours. L'eau (10 mL) est ajoutée, et la phase aqueuse est extraite avec du DCM (3 x 15 mL). Les phases organiques sont lavées avec une solution saturée de NaCl (15 mL), séchées sur MgSO₄ et concentrées sous vide pour laisser une huile jaune. Le résidu est purifié par chromatographie sur gel de silice avec un mélange DCM / AcOEt (80 : 20) comme éluant pour donner le produit **36a** désiré pur sous forme d'huile jaune avec un rendement de 32 %.

### Caractérisation du composé 36a

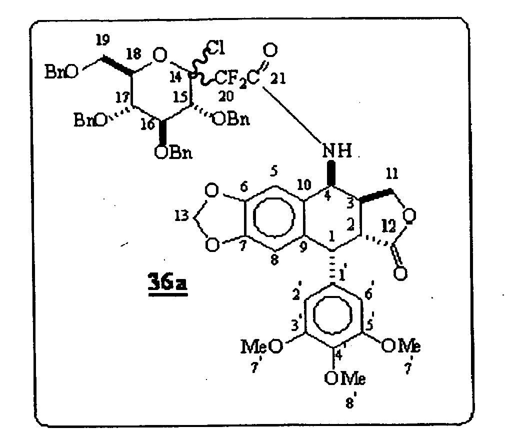

Rf = 0.87, éluant : DCM / AcOEt (80 : 20).
**RMN ¹H (CDCl₃, 300MHz)**
2.81-2.88 (m, 1H, H2), 2.89-2.98 (m, 1H, H3), 3.47-3.63 (m, 3H, H19 x 2), 3.71 (s, 6H, H7' x 6), 3.76 (s, 3H, H8' x 3), 3.91-3.97 (m, 4H, H11), 4.35-4.45 (m, 5H, H11, H1), 4.72-4.84 (m, 5H), 5.19 (dd, 1H, ³J_{H4-H3} 4.7, ³J_{H4-NH} 7.3, H4), 5.91 (dd, 2H, ²J_{H13-H13} 9.1, ³J 0.9, H13 x 2), 6.22 (s, 2H, H2', H6'), 6.43 (s, 1H, H8), 6.72 (s, 1H, H5), 6.96 (d, 1H, ³J_{NH-H4} 7.3, -NH), 7.08-7.32 (m, 20H).
**RMN ¹³C (CDCl₃, 75MHz)**
37.4 (C3), 41.9 (C2), 43.9 (C1), 48.8 (C4), 56.5 (2C, C7' x 2), 61.0 (C8'), 68.1 (C19), 68.8 (C11), 72.4, 73.2, 75.3, 75.8, 76.3, 77.5, 78.1, 83.4, 96.5 (t, ²J_{C-F} 27.4, C14), 101.9 (C13), 108.4 (2C, C2', C6'), 109.3 (C5), 110.4 (C8), 127.6, 127.9, 128.0, 128.1, 128.1, 128.3, 128.6, 128.7, 128.7, 132.8, 134.8, 137.5, 137.6, 138.0, 138.1, 138.4, 148.0 (C7), 148.9 (C6), 152.9 (2C, C3', C5'), 163.8 (t, ²J_{C-F} 30.0, C21),
174.2 (C12).
**RMN ¹⁹F (CDCl₃, 282MHz)**
-116.6 (d, IF, ²J_{F-F} 258.6), -120.4 (d, 1F, ²J_{F-F} 258.6).

### Synthèse du composé 37a (Fig.22)

Dans un ballon, le composé **36a** (44 mg ; 0.042 mmol) est dissout dans le méthanol (10 mL) avec le palladium sur charbon et placé sous une atmosphère d'hydrogène. Le mélange est agité pendant la nuit à température ambiante. Le mélange réactionnel est filtré, concentré pour ainsi laisser le produit **37a** désiré en tant que solide jaune pâle avec un rendement de 68 %.

### Caractérisation du composé 37a

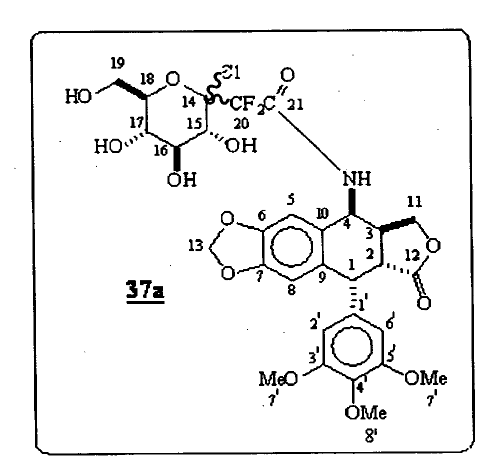

Rf = 0.33, éluant : DCM / méthanol (90 : 10).
**RMN ¹H (MeOD, 300MHz)**
2.99-3.10 (m, 1H, H3), 3.22-3.34 (m, 2H, H2), 3.22-3.72 (m, 5H), 3.71 (s, 9H, H7' x 6, H8' x 3), 3.99 (dd, 1H, ²J_{H11-H11} 8.9, ³J_{H11-H3} 10.9, H11), 4.37 (dd, 1H, ²J_{H11-H11} 8.9, ³J_{H11-H3} 7.6, H11), 4.62 (d, 1H, ³J_{H1-H2} 5.1, H1), 5.31 (d, 1H, ³J_{H4-H3} 4.6, H4), 5.96 (d, 2H, ³J_{H13-H13} 0.6, H13 x 2), 6.37 (s, 2H, H2', H6'), 6.50 (s, 1H, H8), 6.79 (s, 1H, H5).
**RMN ¹³C (MeOD, 75MHz)**
38.8 (C3), 42.8 (C2), 45.3 (C1), 49.9 (C4), 56.7 (2C, C7' x 2), 61.2 (C8'), 61.9 (C 19), 70.4 (C11), 70.8, 72.1, 74.9, 75.9, 97.6 (C 14), 103.2 (C 13), 109.6 (2C, C2', C6'), 110.4 (C5), 111.1 (C8), 130.0, 134.1,137.3,138.4, 149.1 (C7), 150.0 (C6), 154.0 (2C, C3', C5'), 174.8 (C21), 177.1 (C12).
**RMN ¹⁹F (MeOD, 282MHz)**
-119.4 (d, 1F, ²J_{F-F} 257.5), -120.7 (d, 1F, ²J_{F-F}257.5).
**Spectrométrie de masse** : ESI+ : 686 (M-H)-.

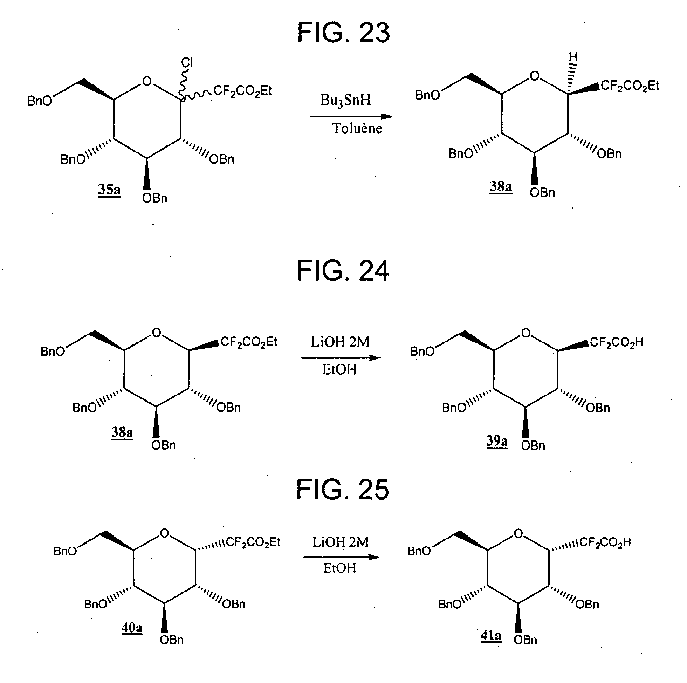

### Synthèse du composé 38a (Fig.23)

Le produit halogéné **35a** est placé avec le tributyl étain (1.5 éq.) dans du toluène sec et la solution est porté à reflux pendant une heure. Après retour à la température ambiante, le milieu est concentré et purifié par colonne chromatographique sur gel de silice avec un éluant cyclohexane / acétate d'éthyle (80 : 20).
Le produit **38a** est isolé avec 23% de rendement

### Caractérisation du composé 38a

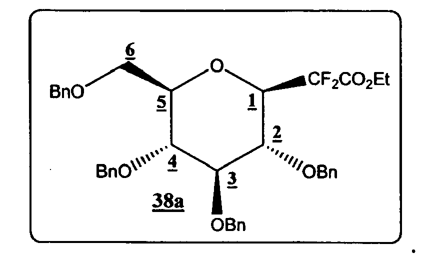

Rf = 0.51, éluant : cyclohexane / acétate d'éthyle (75 : 25).
**RMN ¹H (CDCl₃, 300MHz)**
1.10 (t, 3H, ³J_{H10-H9} 7.2 Hz, H10), 3.56-3.68 (m, 5H), 3.91-4.01 (m, 2H), 4.22-4.86 (m, 10H, 4 x CH₂Ph), 7.18-7.26 (m, 20H, H_{Ar}).
**RMN ¹⁹F (CDCl₃), 282MHz)**
-115.9 (dd, ³J_{F-H} 12.9, ²J_{F-F} 259.7), -118.9 (dd, ³J_{F-H} 10.7, ²J_{F-F} 259.7).

### Synthèse du composé 39a (Fig.24)

Dans un ballon sous atmosphère inerte contenant l'ester **38a** (78 mg ; 0.12 mmol ; 1 éq.) en solution dans l'éthanol (5 mL), une solution aqueuse de lithine (2M ; 2 éq.) est ajoutée et le mélange est agité pendant la nuit à température ambiante. Le milieu est concentré et dissout dans le DCM (5 mL), il est ensuite acidifié avec une solution d'HCl 1M (10 mL). Le mélange est extrait avec du DCM (3 x 10 mL), et les phases organiques sont combinées, lavées avec une solution saturée de NaCl et concentrées directement. L'acide **39a** est isolé ainsi comme une huile jaune qui peut être utilisée directement pour la prochaine étape sans purifications supplémentaires avec un rendement brut de 98%.

### Caractérisation du composé 39a

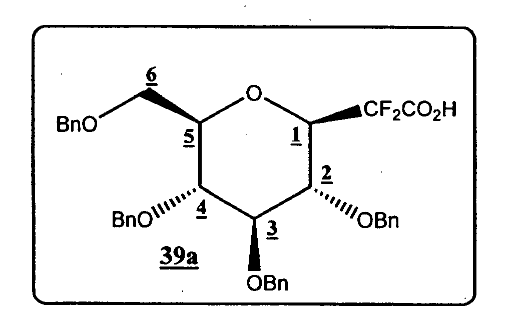

**RMN ¹H (CDCl₃, 300MHz)**
3.53- 4.01 (m, 6H), 4.37-4.87 (m, 9H, 4 x CH₂Ph), 7.08-7.22 (m, 20H, H_{Ar}).
**RMN ¹⁹F (CDCl₃, 282MHz)**
-107.8 (dd, ³J_{F-H} 8.6, ²J_{F-F} 257.0), -110.5 (dd, ³J_{F}-_{H} 12.9, ²J_{F-F} 257.0).
**RMN ¹³C (CDCl₃, 75MHz)**
67.6, 68.8, 70.9, 72.5, 72.8, 73.6, 73.9, 74.2, 75.4, 75.5, 76.3, 77.6, 77.8, 79.7, 86.8, 128.0, 128.2, 128.2, 128.3, 128.3, 128.4, 128.5, 128.7, 128.8, 128.8, 128.9, 128.9, 136.7, 137.7, 137.8, 137.9, 138.0, 138.0, 138.1, 138.5.

### Synthèse du composé 41a (Fig.25) :

Dans un ballon sous atmosphère inerte contenant l'ester **40a** (175 mg ; 0.315 mmol ; 1 éq.) en solution dans l'éthanol (15 mL), une solution aqueuse de lithine (2M ; 2 éq.) est ajoutée et le mélange est agité pendant la nuit à température ambiante. Le milieu est concentré et dissout dans le DCM (5 mL), il est ensuite acidifié avec une solution d'HC11 M (20 mL). Le mélange est extrait avec du DCM (3 x 25 mL), et les phases organiques sont combinées, lavées avec une solution saturée de NaCl et concentrées directement. L'acide **41a** est isolé ainsi comme une huile incolore qui peut être utilisée directement pour la prochaine étape sans purifications supplémentaires avec un rendement brut de 87%.

### Caractérisation du composé 41a

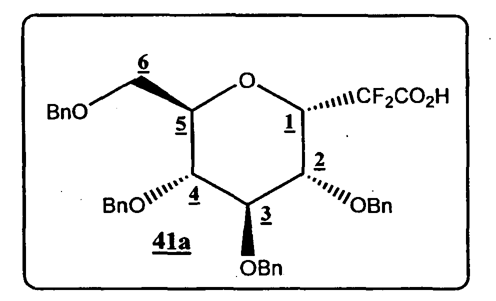

**RMN ¹H (CDCl₃, 300MHz)**
3.51-3.82 (m, 4H), 3.92-4.09 (m, 1H), 4.22-4.75 (m, 9H, 4 x CH₂Ph), 7.08-7.22 (m, 15H,H_{Ar}).
**RMN ¹⁹F (CDCl₃, 282MHz)**
-116.3 (dₐₚₚ, ²J_{F-F} 280.0), -128.1 (d, ²J_{F-F} 284.0).

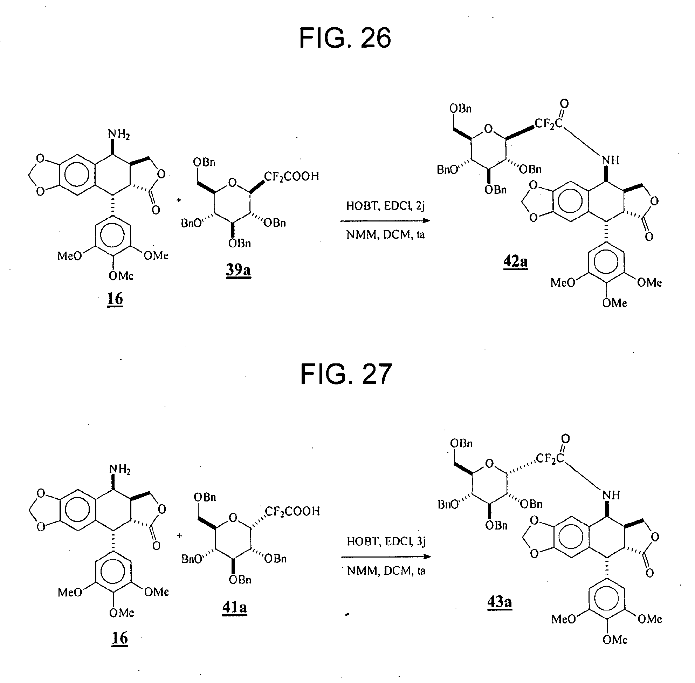

### Synthèse du composé 42a (Fig.26)

Sur une suspension de l'acide **39a** (74 mg ; 0.120 mmol ; 1.00 éq.), de l'amine **16** (50 mg ; 0.135 mmol ; 1.10 éq.), d'HOBT (18 mg ; 0.130 mmol ; 1.05 éq.), et de NMM (31 mg; 0.300 mmol ; 2.05 éq.) dans le DCM (5 mL) sous atmosphère d'argon, l'EDCI (25 mg; 0.130 mmol ; 1.05 éq.) est additionné. La réaction est agitée à température ambiante pendant 2 jours. L'eau (10 mL) est ajoutée, et la phase aqueuse est extraite avec du DCM (3 x 10 mL). Les phases organiques sont lavées avec une solution saturée de NaCl (10 mL), séchées sur MgSO₄ et concentrées sous vide pour laisser une huiole marron clair. Le résidu est purifié par chromatographie sur gel de silice avec un mélange DCM / AcOEt (90 : 10) comme éluant pour donner le produit **42a** pur sous forme d'une huile incolore avec un rendement de 13 %.

### Caractérisation du composé 42a

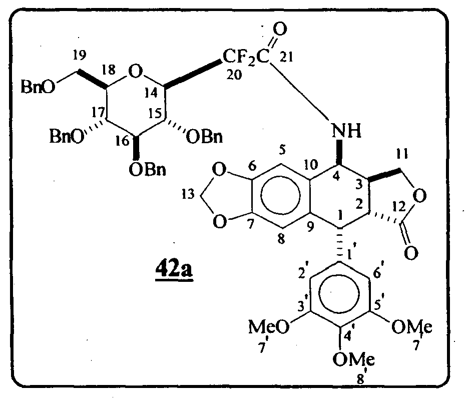

Rf = 0.75, éluant : DCM / AcOEt (80 : 20).
**RMN ¹H (CDCl₃, 300MHz)**
2.62-2.84 (m, 2H, H2, H3), 3.21-3.49 (m, 1H), 3.54 (s, 6H, H7' x 6), 3.60 (s, 3H, H8' x 3), 3.71-3.96 (m, 6H), 4.16-4.69 (m, 12H), 5.00-5.03 (m, 1H ; H4), 5.71-5.79 (m, 2H, H13 x 2), 6.02-6.07 (m, 2H, H2', H6'), 6.28 (d, 1H, J 10.1, H8), 6.54 (s, 1H, J 3.1, H5), 7.00-7.28 (m, 20H).
**RMN ¹³C (CDCl₃, 75MHz)**
37.4 (C3), 41.8 (C2), 43.8 (C1), 48.7 (C4), 56.4 (2C, C7' x 2), 60.9 (C8'), 68.0 (C19), 68.8 (C11), 72.3, 73.2, 75.2, 76.1, 77.4, 79.3, 86.4, 101.9 (C13), 108.3 (2C, C2', C6'), 109.1 (C5), 110.4 (C8), 127.6, 127.9, 127.9, 128.0, 128.2, 128.3, 128.5, 128.6, 128.7, 132.8, 134.7, 136.9, 137.5, 137.8, 137.9, 137.9, 138.4, 147.9 (C7), 148.9 (C6), 152.8 (2C, C3', C5'), 174.1 (C12).
**RMN ¹⁹F (CDCl₃, 282MHz)**
-109.7 (dd, 1F, ³J_{F-H} 3.7 ²J_{F-F} 259.2), -122.0 (dd, 1F, ³J_{F-H} 16.7 ²J_{F-F} 259.2).

### Synthèse du composé 43a (Fig.27)

Sur une suspension de l'acide **41a** (9.0 mg ; 0.0146 mmol ; 1.00 éq.), de l'amine **16** (6.0 mg ; 0.0161 mmol ; 1.10 éq.), d'HOBT (2.1 mg ; 0.0153 mmol ; 1.05 éq.), et de NMM (3.2 mg; 0.0310 mmol ; 2.05 éq.) dans le DCM (2 mL) sous atmosphère d'argon, l'EDCI (3 mg; 0.0153 mmol; 1.05 éq.) est additionné. La réaction est agitée à température ambiante pendant 3 jours. L'eau (5 mL) est ajoutée, et la phase aqueuse est extraite avec du DCM (3 x 10 mL). Les phases organiques sont lavées avec une solution saturée de NaCl (10 mL), séchées sur MgSO₄ et concentrées sous vide pour laisser un solide beige. Le composé **43a** est utilisé dans la suite de la synthèse sans purifications préalables.

### Caractérisation du composé 43a

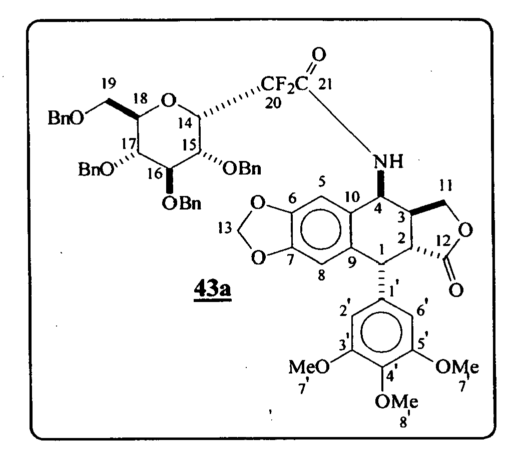

Rf = 0.92, éluant : DCM / AcOEt (80 : 20).
**RMN ¹H (CDCl₃, 300MHz)**
3.22-3.77 (m, 19H), 4.21-4.89 (m, 12H), 5.86-6.41 (m, 6H), 7.00-7.28 (m, 20H, H_{Ar}).
**RMN ¹⁹F (CDCl₃, 282MHz)**
-105.2 (dd, 1F, ³J_{F-H} 13.9, ²J_{F-F} 271.5), -115.5 (dₐₚₚ, 1F, ²J_{F-F} 271.5).

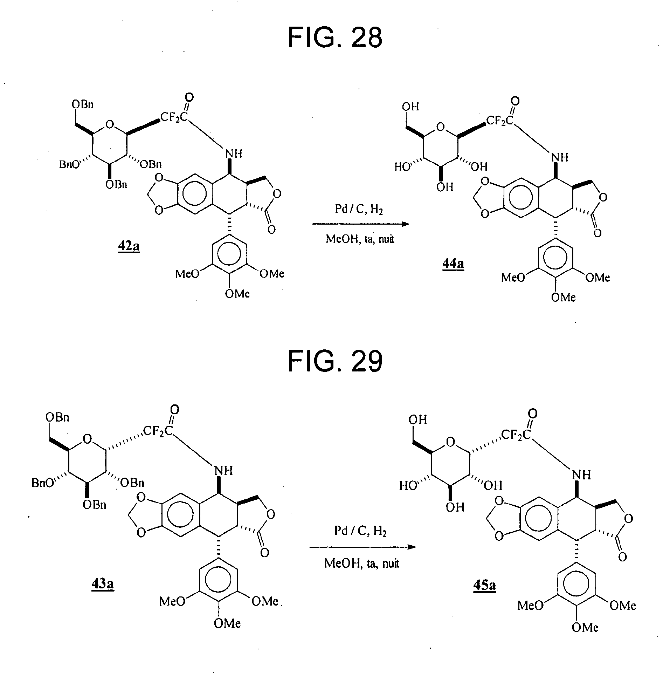

### Synthèse du composé 44a (Fig.28)

Dans un ballon, le composé **42a** (12 mg ; 0.0125 mmol) est dissout dans le méthanol (4 mL) avec le palladium sur charbon et placé sous une atmosphère d'hydrogène. Le mélange est agité pendant la nuit à température ambiante. Le mélange réactionnel est filtré, concentré puis purifié par chromatographie sur colonne de silice avec un mélange DCM / MeOH (90 : 10) comme éluant. Le produit **44a** est isolé sous forme d'un solide beige avec un rendement de 87%.

### Caractérisation du composé 44a

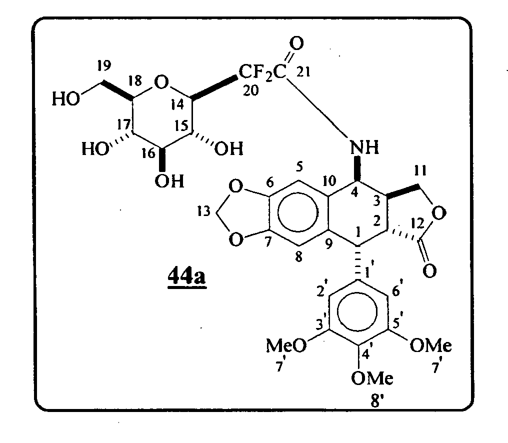

Rf = 0.29, éluant : DCM / méthanol (90 : 10).
**RMN ¹H (MeOD, 300MHz)**
2.98-3.08 (m, 2H, H3, H2), 3.52-3.89 (m, 18H), 3.72 (s, 9H, H7' x 6, H8' x 3), 4.28 (t_{app,} 1H, ²J_{H11-H11} 6.5, H11), 4.60 (s, 1H, H1) 5.22 (dd, 1H, ³J_{H4-H3} 4.5, ³J_{H4-NH} 10.7, H4), 5.94 (s, 2H, H13 x 2), 6.35 (s, 2H, H2', H6'), 6.50 (s, 1H, H8), 6.79 (s, 1H, H5).
**RMN ¹³C (MeOD, 75MHz)**
38.7 (C3), 42.6 (C2), 45.1 (C1), 49.9 (C4), 56.5 (2C, C7' x 2), 61.0 (C8'), 61.9 (C19), 70.2 (C11), 70.6, 79.3, 82.1, 102.9 (C13), 109.5 (2C, C2', C6'), 110.3 (C5), 110.8 (C8), 129.8, 134.0, 137.2, 138.1, 148.9 (C7), 149.8 (C6), 153.8 (2C, C3', C5'), 176.9 (C12).
**RMN ¹⁹F (MeOD, 282MHz)**
-114.4 (dd, 1F, ²J_{F-F} 259.1, ²J_{F-H} 6.8).
-121.9 (dd, 1F, ²J_{F-F}259.1, ²J_{F-H} 16.1).
**Spectrométrie de masse** : ESI- : 652 (M-H)-.

### Synthèse du composé 45a (Fig.29)

Dans un ballon, le composé **43a** (39 mg ; 0.04 mmol) est dissout dans le méthanol (5 mL) avec le palladium sur charbon et placé sous une atmosphère d'hydrogène. Le mélange est agité pendant la nuit à température ambiante. Le mélange réactionnel est filtré, concentré puis purifié par chromatographie sur colonne de silice avec un mélange DCM / MeOH (80 : 20) comme éluant. Le produit **45a** est isolé sous forme d'un solide blanc avec un rendement de 70%.

### Caractérisation du composé 45a

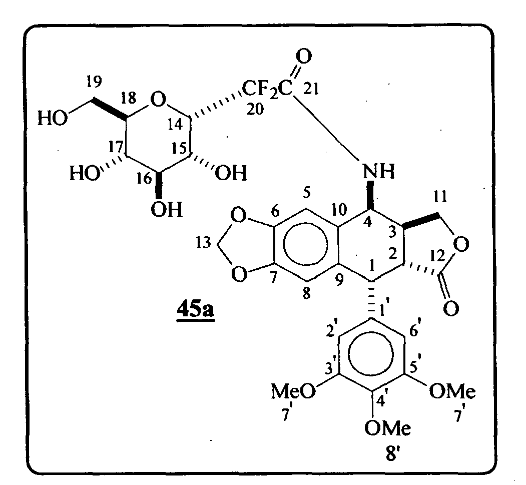

Rf = 0.83, éluant : DCM / méthanol (80 : 20).
**RMN ¹H (MeOD, 300MHz)**
2.92-3.09 (m, 1H, H3), 3.22-3.38 (m, 2H, H2), 3.65-3.81 (m, 15H, H7' x 6, H8' x 3), 4.13 (dd, 1H, ²J_{H11-H11} 9.0, ³J_{H11-H3} 10.7, H11), 4.30 (tₐₚₚ, 1H, ²J_{H11-H11} 9.0, H11), 4.60 (d, 1H, ³J_{H1-H2} 5.1, H1), 5.27 (d, 1H, ³J_{H4-H3} 4.6, H4), 5.94 (d, 2H, ²J_{H13-H13} 1.0, H 13 x 2), 6.36 (s, 2H, H2', H6'), 6.48 (s, 1H, H8), 6.75 (s, 1H, H5).
**RMN ¹³C (MeOD, 75MHz)**
38.8 (C3), 42.6 (C2), 45.1 (Cl), 49.8 (C4), 57.5 (2C, C7' x 2), 61.0 (C8'), 62.3 (C19), 70.1 (C11), 70.8, 71.4, 75.1, 78.6, 102.9 (C13), 109.4 (2C, C2', C6'), 110.0 (C5), 110.8 (C8), 130.0, 133.9, 137.2, 138.1, 148.9 (C7), 149.7 (C6), 153.8 (2C, C3', C5'), 177.1 (C12).
**RMN ¹⁹F (MeOD, 282MHz)**
(-110.9) - (-111.0) (m, 2F).
**Spectrométrie de masse** : ESI- : 652 (M-H)-.

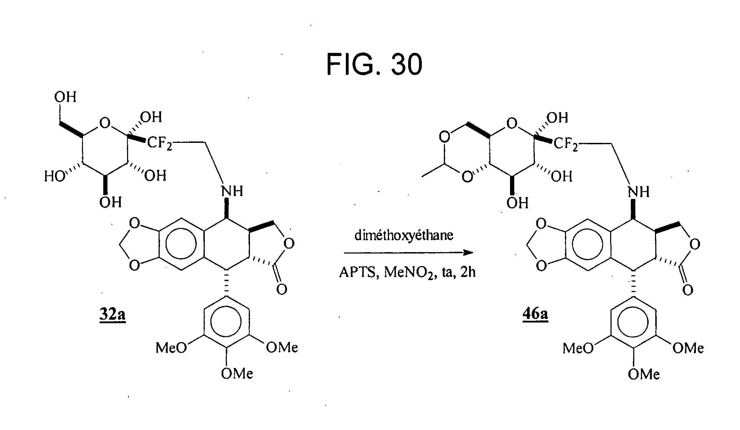

### Synthèse du composé 46a (Fig.30)

Dans un ballon, le composé **32a** (40 mg ; 0.061 mmol, 1.00 éq.) est dissout dans le nitrométhane (10 mL) avec l'APTS (3 mg; 0.015 mmol; 0.25 éq.) et le diméthoxyéthane (166 mg ; 1.840 mmol ; 30.00 éq.) à température ambiante sous atmosphère inerte. Le mélange réactionnel est agité pendant 4h. De l'eau (20 mL) est ajoutée, et la phase aqueuse est extraite avec du CHCl₃ (2 x 30 mL). Les phases organiques sont rassemblées et lavées avec une solution saturée de NaCl (30 mL), séchées sur Na₂SO₄ et concentrées sous vide pour laisser un solide beige. Le brut réactionnel est purifié par colonne chromatographique sur gel de silice avec un mélange DCM / MeOH (90 : 10) comme éluant. Le produit **46a** est isolé comme un solide beige avec 81 % de rendement.

### Caractérisation du composé 46a

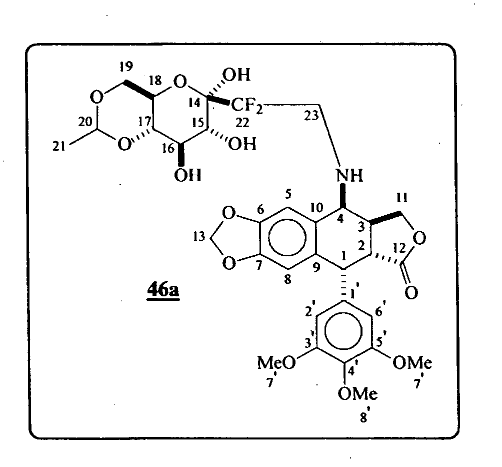

Rf = 0.44, éluant : DCM / méthanol (90 : 10).
**RMN ¹H (MeOD, 300MHz)**
1.30 (d, 3H, ³J_{H21}-_{H20} 5.0, H21 x 3), 2.82-2.85 (m, 1H, H3), 3.21-3.38 (m, 4H, H2, H23 x 2), 3.52 (t, 1H, ²J_{H19-H19} 10.1, H19), 3.69 (s, 9H, H7' x 6, H8' x 3), 3.66-3.77 (m, 1H), 4.03 (dd, 1H, ²J_{H19-H19} 10.1, ³J_{H19-H18} 5.0, H19), 4.0.8 (d, 1H, J 4.1, H4), 4.31 (dd, 1H, 2JH11-H11 8.2, ³J_{H11-H3} 10.9, H11), 4.39 (t, 1H, ²J_{H11-H11} 8.2, H11), 4.53 (d, 1H, ³J_{H1-H2} 5.4, H1), 4.75 (q, 1H, ³J_{H20-H21} 5.0, H20), 5.93 (s, 2H, H13 x 2), 6.31 (s, 2H, H2', H6'), 6.43 (s, 1H, H8), 6.95 (s, 1H, H5).
**RMN ¹³C (MeOD, 75MHz)**
20.6 (C21), 40.3 (C3), 42.2 (C2), 45.0 (C1), 51.3 (t, ²J_{C23-F} 24.6, C23), 56.5 (2C, C7' x 2), 57.6 (C4), 61.0 (C8'), 64.5, 69.3 (C19), 70.2 (C11), 72.8, 72.9, 81.5, 99.1 (t, ²J_{C14-F}26.3, C14), 100.7 (C20), 102.8 (C13), 109.4 (2C, C2', C6'), 110.1 (C5), 110.9 (C8), 132.8, 133.6, 137.7, 138.0, 148.7 (C7), 149.2 (C6), 153.7 (2C, C3'; C5'), 177.8 (C12).
**RMN ¹⁹F (CDCl₃, 282MHz)**
-117.9 (ddd, 1F, ²J_{F-F} 255.4, ³J_{F-H21} 17.2, 14.0), -119.4 (ddd, 1F, ²J_{F-F} 255.4, ³J_{F-H21} 17.2, 11.8).
Spectrométrie de masse : ESI+ : 682 (M+H)+.

### Résultats de cytotoxicité

Les premiers tests de cytotoxicité ont été réalisés sur différentes lignées cellulaires telles que les cellules KB, PC3, MCF7 et MCF7R, SF268, HL60, HT29 ,A549 à des concentrations de 10⁻⁵M en triplicate. La podophyllotoxine **1** et l'étoposide **2** ont également été testés dans les mêmes conditions comme références.
Les résultats sont exprimés en pourcentage d'inhibition de la croissance cellulaire.

| | **1** | **2** | **26a** | **28a** | **27a** | **29a** | **26b** | **28b** | **27b** | **29b** | **32a** | **31a** | **37a** | **44a** | **46a** |
|---|---|---|---|---|---|---|---|---|---|---|---|---|---|---|---|
| KB | **90** | **84** | 43 | 54 | **91** | 66 | 12 | 15 | 12 | 0 | **94** | 22 | **91** | **90** | **86** |
| PC3 | **65** | **47** | 10 | 31 | **63** | 8 | 8 | 6 | 8 | 3 | - | - | | | |
| MCF7 | **55** | **57** | 37 | 32 | **53** | 40 | 20 | 33 | 20 | 7 | - | - | | | |
| MCF7R | **55** | **22** | 35 | 49 | **70** | 44 | 16 | 20 | 10 | 7 | - | - | | | |
| SF268 | **74** | **82** | 7 | 28 | **65** | 11 | 7 | 17 | 7 | 6 | - | - | | | |
| HL60 | **74** | **75** | 17 | 43 | **79** | 17 | 19 | 12 | 19 | 0 | - | - | | | |
| HT29 | **84** | **79** | 10 | 14 | **87** | 17 | 13 | 17 | 8 | 6 | - | - | | | |
| A549 | **83** | **65** | 23 | 21 | **78** | 6 | 7 | 10 | 6 | 1 | - | - | | | |

On observe que sur ce type de cellules le composé **27a** possède une activité comparable à celle de l'étoposide et de la podophyllotoxine, et même meilleure sur certaines lignées cellulaires. De même, le composé **32a** testé sur cellules KB présente une cytotoxicité voisine de l'étoposide et de la podophyllotoxine, ainsi que **37a ,44a, 46a**

Les valeurs d'IC₅₀ sont exprimées en µM sur cellules KB en duplicate et ont été réalisé pour les 4 composés leader à savoir **27a, 32a, 44a, 37a** et **46a** en parallèle avec la podophyllotoxine et l'étoposide.

| | **IC₅₀ (µM)** |
|---|---|
| **Podophyllotoxine 1** | 0,015 / 0,022 |
| **27a** | 3,45 / 2,55 |
| **32a** | 0,135 / 0,175 |
| **44a** | 0.323/ 0.341 |
| **37a** | 0.273/ 0.306 |
| **46a** | 4.707/ 6.026 |
| **Etoposide** | 3.509/1.644 |

On observe donc pour ces deux composés des valeurs intéressantes en terme de cytotoxicité et d'IC50 pour le composé **32a** , **37a** et **44a** ce qui fait de ces composés de bons agents de chimiothérapie pour le traitement du cancer.

Ces composés sont actuellement sujets à des tests *in vivo.*

## Revendications

1. Composé glycoconjugué gem-difluoré de formule générale I : 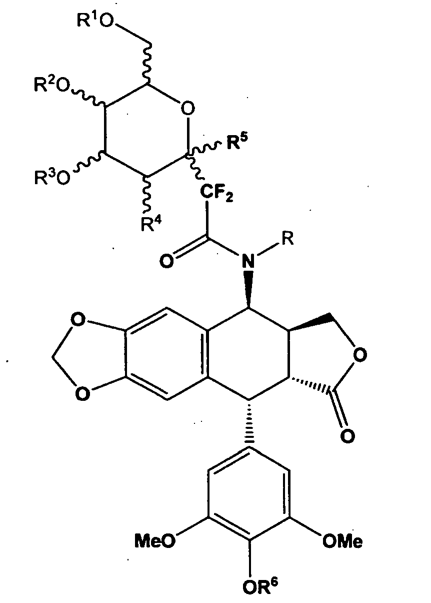 où R représente un atome d'hydrogène ou un groupe alkyle linéaire ou ramifié,
benzyle, acétyle, benzoyle,
R¹ et R², identiques ou différents,
représentent un atome d'hydrogène
ou un groupe protecteur alkyle linéaire ou ramifié, benzyle, benzoyle, acétyle, pivaloyle, trialkylsilyle, tertiobutyldiphénylsilyle
ou un groupe acétal, du type CR'R",
avec R'et R", identiques ou différents, représentent un atome d'hydrogène ou un groupe alkyle linéaire ou ramifié, aryle, benzyle, thiophène,
R³ représente un atome d'hydrogène
ou un groupe protecteur alkyle linéaire ou ramifié, benzyle, benzoyle, acétyle, pivaloyle, trialkylsilyle , tertiobutyldiphénylsilyle,
R⁴ représente OR''', NGR'GR", N₃, ou un phtalimide
avec R''' représente un atome d'hydrogène ou un groupe protecteur alkyle linéaire ou ramifié, benzyle, benzoyle, acétyle, pivaloyle, trialkylsilyle, tertiobutyldiphénylsilyle, GR'et GR", identiques ou différents, représentent un atome d'hydrogène ou un groupe alkyle linéaire ou ramifié, benzyle, benzoyle, acétyle, alkyloxycarbonyle, allyloxycarbonyle, benzyloxycarbonyle,
R⁵ représente un groupe hydroxyle libre ou protégé ou un halogène,
R⁶ représente un atome d'hydrogène ou un groupe alkyle linéaire ou ramifié, acétyle, benzyle, PO₃H, PO₃Na,
ainsi que ses dérivés à l'état de base, de sel d'addition à un acide minéral ou organique, d'hydrate ou de solvat éventuellement pharmaceutiquement acceptable.

2. Composé selon la revendication 1 de formule générale II : 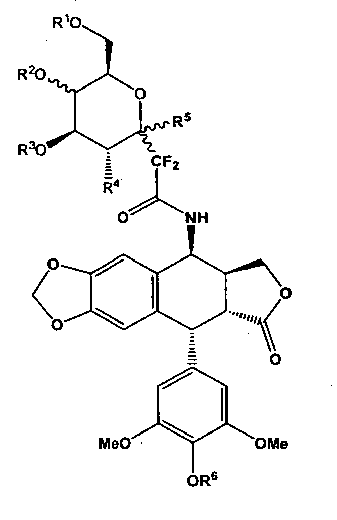 dans laquelle R, R¹, R², R³, R⁴, R⁵ et R⁶ sont tels que définis dans la formule I,
ainsi que ses dérivés à l'état de base, de sel d'addition à un acide minéral ou organique, d'hydrate ou de solvat éventuellement pharmaceutiquement acceptable.

3. Composé de formule générale III obtenu par réaction d'un composé selon la revendication 1 ou 2 : 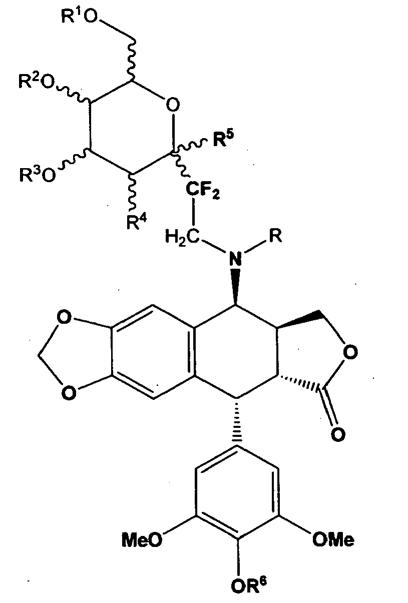 ou R représente un atome d'hydrogène ou un groupe alkyle linéaire ou ramifié,
benzyle, acétyle, benzoyle,
R¹ et R², identiques ou différents,
représentent un atome d'hydrogène
ou un groupe protecteur alkyle linéaire ou ramifié, benzyle, benzoyle, acétyle, pivaloyle, trialkylsilyle, tertiobutyldiphénylsilyle
ou un groupe acétal, du type CR'R",
avec R'et R", identiques ou différents, représentent un atome d'hydrogène ou un groupe alkyle linéaire ou ramifié, aryle, benzyle, thiophène,
R³ représente un atome d'hydrogène
ou un groupe protecteur alkyle linéaire ou ramifié, benzyle, benzoyle, acétyle, pivaloyle, trialkylsilyle , tertiobutyldiphénylsilyle,
R⁴ représente OR''', NGR' GR'', N₃, ou un phtalimide
avec R''' représente un atome d'hydrogène ou un groupe protecteur alkyle linéaire ou ramifié, benzyle, benzoyle, acétyle, pivaloyle, trialkylsilyle, tertiobutyldiphénylsilyle, GR'et GR", identiques ou différents, représentent un atome d'hydrogène ou un groupe alkyle linéaire ou ramifié, benzyle, benzoyle, acétyle, alkyloxycarbonyle, allyloxycarbonyle, benzyloxycarbonyle,
R⁵ représente un groupe hydroxyle libre ou protégé ou un halogène,
R⁶ représente un atome d'hydrogène ou un groupe alkyle linéaire ou ramifié, acétyle, benzyle, PO₃H, PO₃Na,
ainsi que ses dérivés à l'état de base, de sel d'addition à un acide minéral ou organique, d'hydrate ou de solvat éventuellement pharmaceutiquement acceptable.

4. Utilisation d'un composé selon la revendication 1 ou 2 pour la synthèse d'un composé de formule générale III : 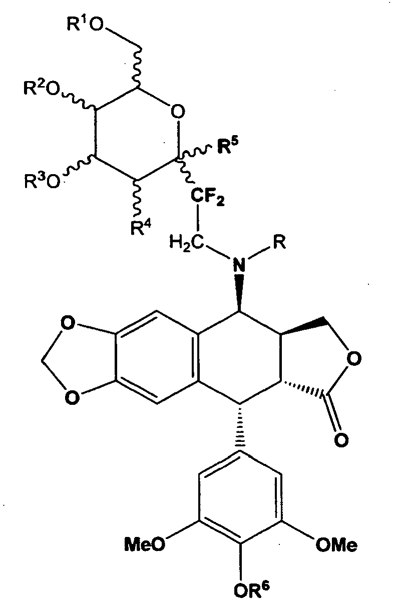 où R représente un atome d'hydrogène ou un groupe alkyle linéaire ou ramifié, benzyle, acétyle, benzoyle,
R¹ et R², identiques ou différents,
représentent un atome d'hydrogène
ou un groupe protecteur alkyle linéaire ou ramifié, benzyle, benzoyle, acétyle, pivaloyle, trialkylsilyle, tertiobutyldiphénylsilyle
ou un groupe acétal, du type CR'R",
avec R'et R", identiques ou différents, représentent un atome d'hydrogène ou un groupe alkyle linéaire ou ramifié, aryle, benzyle, thiophène,
R³ représente un atome d'hydrogène
ou un groupe protecteur alkyle linéaire ou ramifié, benzyle, benzoyle, acétyle, pivaloyle, trialkylsilyle , tertiobutyldiphénylsilyle,
R⁴ représente OR"', NGR'GR", N₃, ou un phtalimide
avec R"' représente un atome d'hydrogène ou un groupe protecteur alkyle linéaire ou ramifié, benzyle, benzoyle, acétyle, pivaloyle, trialkylsilyle, tertiobutyldiphénylsilyle, GR'et GR", identiques ou différents, représentent un atome d'hydrogène ou un groupe alkyle linéaire ou ramifié, benzyle, benzoyle, acétyle, alkyloxycarbonyle, allyloxycarbonyle, benzyloxycarbonyle,
R⁵ représente un groupe hydroxyle libre ou protégé ou un halogène,
R⁶ représente un atome d'hydrogène ou un groupe alkyle linéaire ou ramifié, acétyle, benzyle, PO₃H, PO₃Na,
ainsi que ses dérivés à l'état de base, de sel d'addition à un acide minéral ou organique, d'hydrate ou de solvat éventuellement pharmaceutiquement acceptable.

5. Procédé de préparation d'un composé selon la revendication 1,
**caractérisé en ce qu'**il comprend une étape de couplage entre un composé de formule IV: 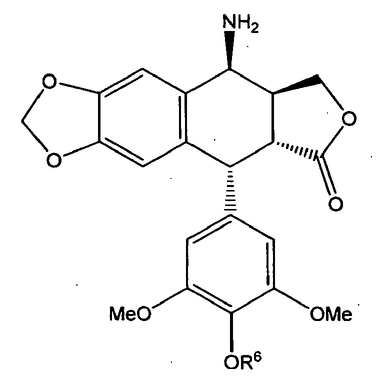 dans laquelle R⁶ est tel que défini dans la formule I
et un composé de formule V : 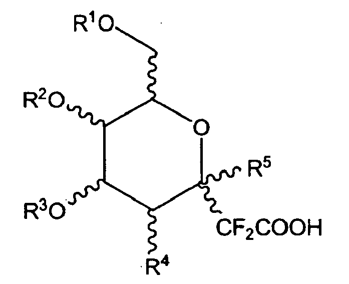 dans laquelle R¹, R², R³, R⁴, R⁵ sont tels que définis dans la formule I.

6. Procédé selon la revendication 5,
**caractérisé en ce que** ledit composé de formule IV est obtenu par épimérisation puis substitution de la fonction alcool en position 4 par un groupe azido réduit ultérieurement en amine.

7. Procédé selon la revendication 5,
**caractérisé en ce que** ledit composé de formule V est obtenu par un composé intermédiaire de formule VI : 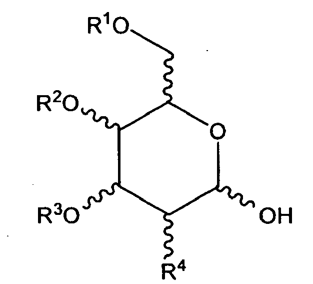 dans laquelle R¹, R², R³, R⁴ sont tels que définis dans la formule I.

8. Procédé selon la revendication 5,
**caractérisé en ce que**, lorsque dans le composé de formule V, R⁵ représente un groupe hydroxyle, il comprend, en outre, une oxydation en lactone du composé de formule VI suivi d'un réaction de Reformatsky.

9. Composition,
**caractérisée en ce qu'**elle comporte au moins un composé l'une des revendications 1 à 3.

10. Médicament contenant en tant que principe actif au moins un composé selon l'une des revendications 1 à 3.

11. Utilisation d'au moins un composé selon l'une des revendications 1 à 3 pour la préparation de médicaments pour le traitement de cancers.

## Claims

1. A gem-difluorinated glycoconjugated compound of general formula I: 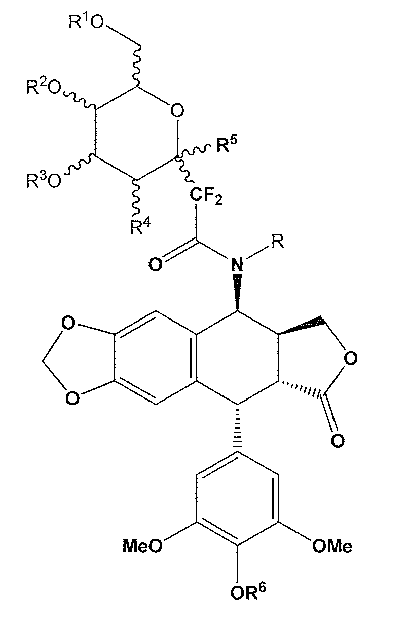 wherein R represents a hydrogen atom or a linear or branched alkyl,
benzyl, acetyl, benzoyl group,
R¹ and R², either identical or different,
represent a hydrogen atom
or a protective linear or branched alkyl, benzyl, benzoyl, acetyl, pivaloyl, trialkylsilyl, tertiobutyldiphenylsilyl group or an acetal group, of the CR'R" type,
with R'and R", either identical or different, representing a hydrogen atom or a linear or branched alkyl, aryl, benzyl, thiophene group,
R³ represents a hydrogen atom
or a protective linear or branched alkyl, benzyl, benzoyl, acetyl, pivaloyl, trialkylsilyl, tertiobutyldiphenylsilyl group,
R⁴ represents OR''', NGR'GR", N₃, or a phthalimide with R''' representing a hydrogen atom or a linear or branched alkyl, benzyl, benzoyl, acetyl, pivaloyl, trialkylsilyl, tertiobutyldiphenylsilyl protective group, GR' and GR", either identical or different representing a hydrogen atom or a linear or branched alkyl, benzyl, benzoyl, acetyl, alkyloxycarbonyl, allyloxycarbonyl, benzyloxycarbonyl group,
R⁵ represents a free or protective hydroxyl group or a halogen, R⁶ represents a hydrogen atom or a linear or branched alkyl, acetyl, benzyl, PO₃H, PO₃Na group,
as well as its derivatives in the state of a base, of a mineral or organic acid addition salt, of a hydrate or a possibly pharmaceutically acceptable solvate.

2. The compound according to claim 1 of general formula II: 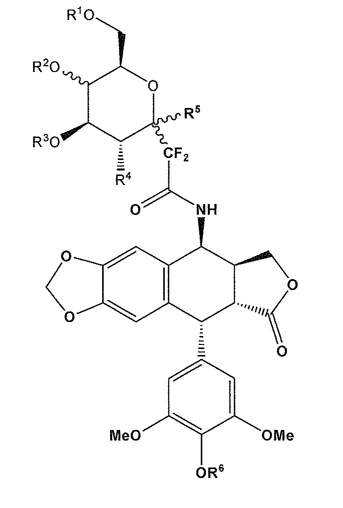 wherein R, R¹, R², R³, R⁴, R⁵ and R⁶ are as defined in formula I,
as well as its derivatives in the state of a base, of a mineral or organic acid addition salt, of a hydrate or a possibly pharmaceutically acceptable solvate.

3. The compound of general formula III obtained by reaction of a compound according to claim 1 or 2: 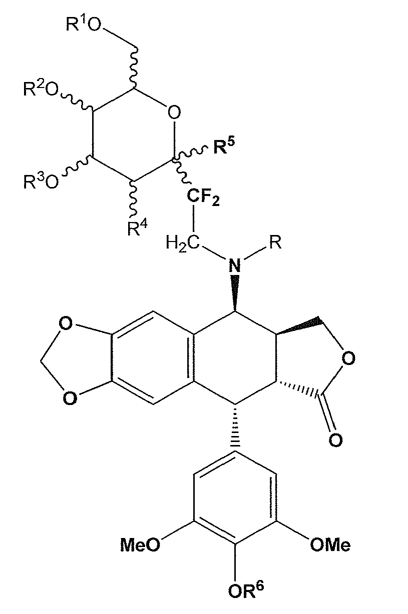 wherein R represents a hydrogen atom or a linear or branched alkyl,
benzyl, acetyl, benzoyl group,
R¹ and R², either identical or different,
represent a hydrogen atom
or a protective linear or branched alkyl, benzyl, benzoyl, acetyl, pivaloyl, trialkylsilyl, tertiobutyldiphenylsilyl group or an acetal group of the CR'R" type,
with R'and R", either identical or different, representing a hydrogen atom or a linear or branched alkyl, aryl, benzyl, thiophene group,
R³ represents a hydrogen atom
or a protective linear or branched alkyl, benzyl, benzoyl, acetyl, pivaloyl, trialkylsilyl, tertiobutyldiphenylsilyl group,
R⁴ represents OR''', NGR'GR'', N₃, or a phthalimide with R''' representing a hydrogen atom or a protective linear or branched alkyl, benzyl, benzoyl, acetyl pivaloyl, trialkylsilyl, tertiobutyldiphenylsilyl group, GR'and GR'', either identical or different, representing a hydrogen atom or a linear or branched alkyl, benzyl, benzoyl, acetyl, alkyloxycarbonyl, allyloxycarbonyl, benzyloxycarbonyl group,
R⁵ represents a free or protective hydroxyl group or a halogen,
R⁶ represents a hydrogen atom or a linear or branched alkyl, acetyl, benzyl, PO₃H, PO₃Na group,
as well as its derivatives in the state of a base, of a mineral or organic acid addition salt, or of a hydrate or a possibly pharmaceutically acceptable solvate.

4. The use of a compound according to claim 1 or 2 for the synthesis of a compound of general formula III: 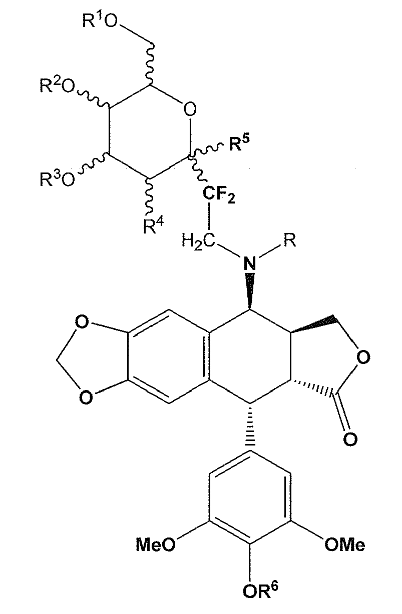 wherein R represents a hydrogen atom or a linear or branched alkyl, benzyl, acetyl, benzoyl group,
R¹ and R², either identical or different,
represent a hydrogen atom
or a protective linear or branched alkyl, benzyl, benzoyl, acetyl, pivaloyl, trialkylsilyl, tertiobutyldiphenylsilyl group or an acetal group of the CR'R" type,
with R'and R", either identical or different, representing a hydrogen atom or a linear or branched alkyl, aryl, benzyl, thiophene group,
R³ represents a hydrogen atom
or a linear or branched alkyl, benzyl, benzoyl, acetyl, pivaloyl, trialkylsilyl , tertiobutyldiphenylsilyl protective group,
R⁴ represents OR''', NGR'GR", N₃, or a phthalimide with R''' representing a hydrogen atom or a linear or branched alkyl, benzyl, benzoyl, acetyl, pivaloyl, trialkylsilyl, tertiobutyldiphenylsilyl protective group, GR'and GR", either identical or different, represent a hydrogen atom or a linear or branched alkyl, benzyl, benzoyl, acetyl, alkyloxycarbonyl, allyloxycarbonyl, benzyloxycarbonyl group,
R⁵ represents a free or protective hydroxyl group or a halogen, R⁶ represents a hydrogen atom or a linear or branched alkyl, acetyl, benzyl, PO₃H, PO₃Na group,
as well as its derivatives in the state of a base of a mineral organic acid addition salt, or of a hydrate or a possibly pharmaceutically acceptable solvate.

5. A method for preparing a compound according to claim 1, **characterized in that** it comprises a coupling step between a compound of formula IV : 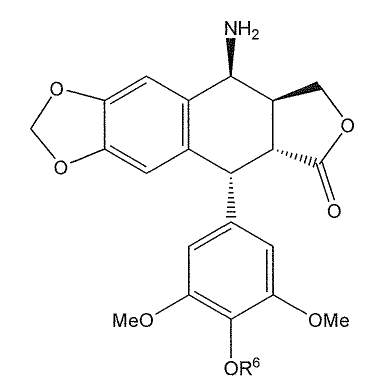 wherein R⁶ is as defined in formula I
and a compound of formula V: 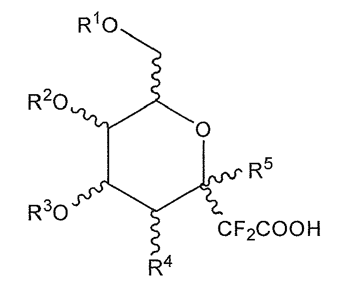 wherein R¹, R², R³, R⁴, R⁵ are as defined in formula I.

6. The method according to claim 5,
**characterized in that** said compound of formula IV is obtained by epimerization and then substitution of the alcohol function in position 4 with an azido group subsequently reduced to an amine group.

7. The method according to claim 5,
**characterized in that** said compound of formula V is obtained by an intermediate compound of formula VI : 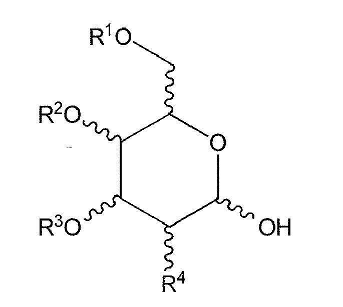 wherein R¹, R², R³, R⁴ are as defined in formula I.

8. The method according to claim 5,
**characterized in that**, when in the compound of formula V R⁵ represents a hydroxyl group, it further comprises an oxidation into a lactone of the compound of formula VI followed by a Reformatsky reaction.

9. A composition,
**characterized in that** it comprises at least one compound according to any of claims 1 to 3.

10. A drug containing as active ingredient at least one compound according to any of claims 1 to 3.

11. The use of at least one compound according to any of claims 1 to 3 for preparing drugs for treating cancers.

## Patentansprüche

1. Glycokonjugierte gem-difluorierte Verbindung der allgemeinen Formel I: 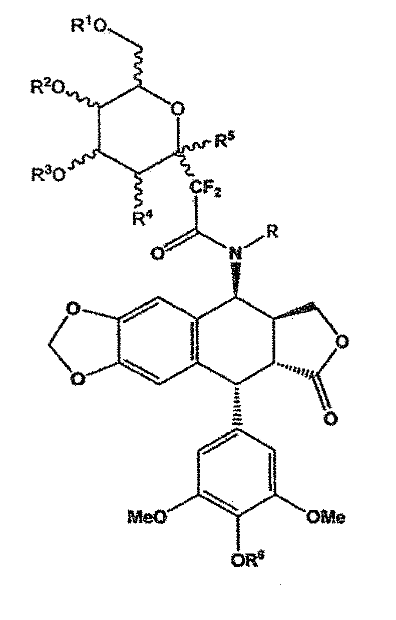 wobei
R ein Wasserstoffatom oder eine lineare oder verzweigte Alkyl-, Benzyl-, Acetyl-, Benzoylgruppe darstellt,
R¹ und R², gleich oder verschieden, ein Wasserstoffatom
oder eine lineare oder verzweigte Alkyl-, Benzyl-, Benzoyl-, Acetyl-, Pivaloyl-, Trialkylsilyl-, tert.-Butyldiphenylsilyl-Schutzgruppe
oder eine Acetalgruppe des Typs CR'R" darstellen,
wobei R' und R", gleich oder verschieden, ein Wasserstoffatom oder eine lineare oder verzweigte Alkyl-, Aryl-, Benzyl-, Thiophengruppe darstellen,
R³ ein Wasserstoffatom
oder eine lineare oder verzweigte Alkyl-, Benzyl-, Benzoyl-, Acetyl-, Pivaloyl-, Trialkylsilyl-, tert.-Butyldiphenylsilyl-Schutzgruppe darstellt,
R⁴ OR''', NGR'GR", N₃ oder ein Phtalimid darstellt,
wobei R''' ein Wasserstoffatom oder eine lineare oder verzweigte Alkyl-, Benzyl-, Benzoyl-, Acetyl-, Pivaloyl-, Trialkylsilyl-, tert.-Butyl- diphenylsilyl-Schutzgruppe darstellt,
GR' und GR", gleich oder verschieden, ein Wasserstoffatom oder eine lineare oder verzweigte Alkylgruppe, Benzyl-, Benzoyl-, Acetyl-, Alkyloxycarbonyl-, Allyloxycarbonyl-, Benzyloxy- carbonylgruppe darstellen,
R⁵ eine freie oder geschützte Hydroxylgruppe oder ein Halogen darstellt,
R⁶ ein Wasserstoffatom oder eine lineare oder verzweigte Alkyl-, Acetyl-, Benzylgruppe, PO₃H, PO₃Na darstellt,
sowie ihre Derivate in Form von Base, Säure-Additionssalz mit einer Mineralsäure oder organischen Säure, von Hydrat oder Solvat, gegebenenfalls pharmazeutisch verträglich.

2. Verbindung nach Anspruch 1 der allgemeinen Formel II, 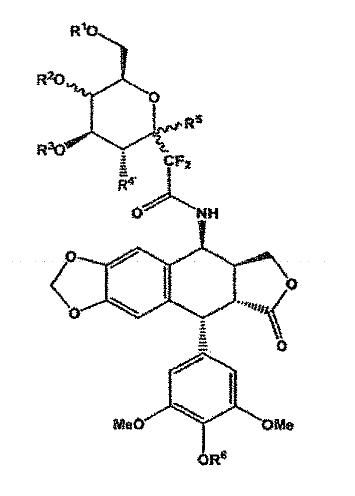 wobei R, R¹, R², R³, R⁴, R⁵ und R⁶ wie in Formel I definiert sind, sowie ihre Derivate in Form von Base, Säure-Additionssalz mit einer Mineralsäure oder organischen Säure, von Hydrat oder Solvat, gegebenenfalls pharmazeutisch verträglich.

3. Verbindung der allgemeinen Formel III, erhalten durch Umsetzung einer Verbindung nach Anspruch 1 oder 2: 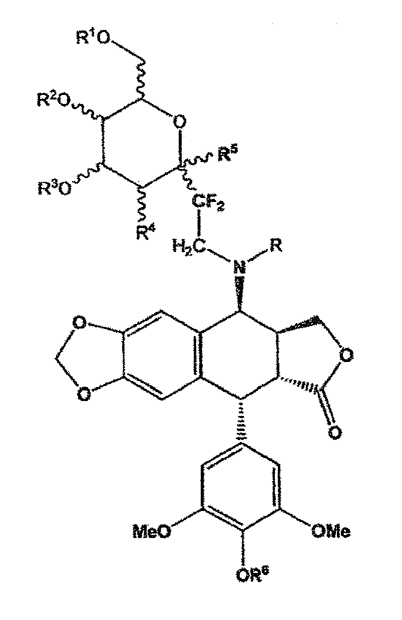 wobei
R ein Wasserstoffatom oder eine lineare oder verzweigte Alkyl-, Benzyl-, Acetyl-, Benzoylgruppe darstellt,
R¹ und R², gleich oder verschieden,
ein Wasserstoffatom
oder eine lineare oder verzweigte Alkyl-, Benzyl-, Benzoyl-, Acetyl-, Pivaloyl-, Trialkylsilyl-, tert.-Butyldiphenylsilyl-Schutzgruppe oder eine Acetalgruppe des Typs CR'R" darstellen,
wobei R' und R", gleich oder verschieden, ein Wasserstoffatom oder eine lineare oder verzweigte Alkyl-, Aryl-, Benzyl-, Thiophengruppe darstellen,
R³ ein Wasserstoffatom
oder eine lineare oder verzweigte Alkyl-, Benzyl-, Benzoyl-, Acetyl-, Pivaloyl-, Trialkylsilyl-, tert.-Butyldiphenylsilyl-Schutzgruppe darstellt,
R⁴ OR''', NGR'GR", N₃ oder ein Phtalimid darstellt,
wobei R''' ein Wasserstoffatom oder eine lineare oder verzweigte Alkyl-, Benzyl-, Benzoyl-, Acetyl-, Pivaloyl-, Trialkylsilyl-, tert.-Butyl- diphenylsilyl-Schutzgruppe darstellt,
GR' und GR", gleich oder verschieden, ein Wasserstoffatom oder eine lineare oder verzweigte Alkylgruppe, Benzyl-, Benzoyl-, Acetyl-, Alkyloxycarbonyl-, Allyloxycarbonyl-, Benzyloxy- carbonylgruppe darstellen,
R⁵ eine freie oder geschützte Hydroxylgruppe oder ein Halogen darstellt,
R⁶ ein Wasserstoffatom oder eine lineare oder verzweigte Alkyl-, Acetyl-, Benzylgruppe, PO₃H, PO₃Na darstellt,
sowie ihre Derivate Form von Base, Säure-Additionssalz mit einer Mineralsäure oder organischen Säure, von Hydrat oder Solvat, gegebenenfalls pharmazeutisch verträglich.

4. Verwendung einer Verbindung nach Anspruch 1 oder 2 zur Synthese einer Verbindung der allgemeinen Formel III, 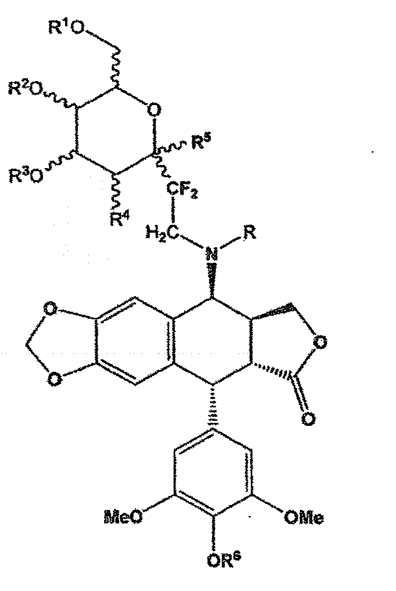 wobei
R ein Wasserstoffatom oder eine lineare oder verzweigte Alkyl-, Benzyl-, Acetyl-, Benzoylgruppe darstellt,
R¹ und R², gleich oder verschieden,
ein Wasserstoffatom
oder eine lineare oder verzweigte Alkyl-, Benzyl-, Benzoyl-, Acetyl-, Pivaloyl-, Trialkylsilyl-, tert.-Butyldiphenylsilyl-Schutzgruppe oder eine Acetalgruppe des Typs CR'R" darstellen,
wobei R' und R", gleich oder verschieden, ein Wasserstoffatom oder eine lineare oder verzweigte Alkyl-, Aryl-, Benzyl-, Thiophengruppe darstellen,
R³ ein Wasserstoffatom
oder eine lineare oder verzweigte Alkyl-, Benzyl-, Benzoyl-, Acetyl-, Pivaloyl-, Trialkylsilyl-, tert.-Butyldiphenylsilyl-Schutzgruppe darstellt,
R⁴ OR''', NGR'GR", N₃ oder ein Phtalimid darstellt,
wobei R''' ein Wasserstoffatom oder eine lineare oder verzweigte Alkyl-, Benzyl-, Benzoyl-, Acetyl-, Pivaloyl-, Trialkylsilyl-, tert.-Butyl- diphenylsilyl-Schutzgruppe darstellt,
GR' und GR", gleich oder verschieden, ein Wasserstoffatom oder eine lineare oder verzweigte Alkylgruppe, Benzyl-, Benzoyl-, Acetyl-, Alkyloxycarbonyl-, Allyloxycarbonyl-, Benzyloxy- carbonylgruppe darstellen,
R⁵ eine freie oder geschützte Hydroxylgruppe oder ein Halogen darstellt,
R⁶ ein Wasserstoffatom oder eine lineare oder verzweigte Alkyl-, Acetyl-, Benzylgruppe, PO₃H, PO₃Na darstellt,
sowie ihre Derivate Form von Base, Säure-Additionssalz mit einer Mineralsäure oder organischen Säure, von Hydrat oder Solvat, gegebenenfalls pharmazeutisch verträglich.

5. Verfahren zur Herstellung einer Verbindung nach Anspruch 1, **dadurch gekennzeichnet, dass** es einen Schritt der Kupplung zwischen einer Verbindung der Formel IV: 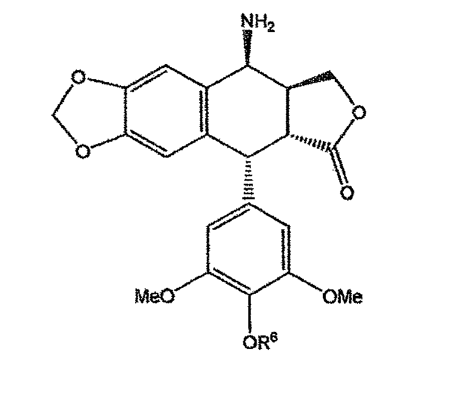 wobei R⁶ wie in Formel I definiert ist und einer Verbindung der Formel V: 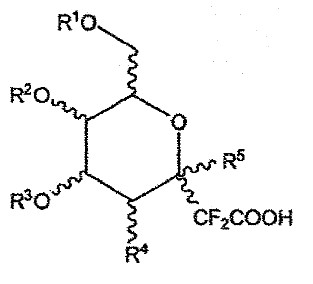 wobei R¹, R², R³, R⁴, R⁵ wie in Formel I definiert sind, umfasst.

6. Verfahren nach Anspruch 5, **dadurch gekennzeichnet, dass** die Verbindung der Formel IV durch Epimerisierung und anschließende Substitution der Alkoholfunktion in Position 4 durch eine Azidogruppe, die dann zu Amin reduziert wird, erhalten wird.

7. Verfahren nach Anspruch 5, **dadurch gekennzeichnet, dass** die Verbindung der Formel V durch eine intermediäre Verbindung der Formel VI erhalten wird: 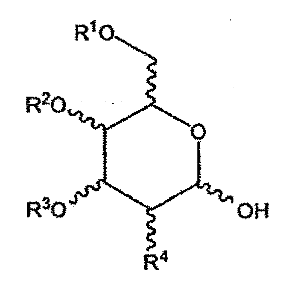 wobei R¹, R², R³, R⁴ wie in der Formel I definiert sind.

8. Verfahren nach Anspruch 5,
**dadurch gekennzeichnet, dass**, wenn in der Verbindung der Formel V R⁵ eine Hydroxylgruppe darstellt, es weiterhin eine Oxidation zum Lacton der Verbindung der Formel VI und anschließende Reformatsky-Reaktion umfasst.

9. Zusammensetzung,
**dadurch gekennzeichnet, dass** sie mindestens eine Verbindung nach einem der Ansprüche 1 bis 3 umfasst.

10. Medikament, das als Wirkstoff mindestens eine Verbindung nach einem der Ansprüche 1 bis 3 umfasst.

11. Verwendung von mindestens einer Verbindung nach einem der Ansprüche 1 bis 3 für die Herstellung von Medikamenten zur Behandlung von Krebsen.
